# EUROPÄISCHE PATENTSCHRIFT

(11) **EP 2 051 714 B1**
(45) Veröffentlichungstag und Bekanntmachung des Hinweises auf die Patenterteilung: **13.04.2011**
(21) Anmeldenummer: 07788188.6
(22) Anmeldetag: 03.08.2007
(51) Int. Cl.: A61K 31/538, A61K 31/423, A61P 9/06, A61P 11/08, A61P 15/06, A61P 17/06

(54) **ARZNEIMITTELKOMBINATIONEN ZUR BEHANDLUNG VON ATEMWEGSERKRANKUNGEN**
DRUG COMBINATIONS FOR THE TREATMENT OF RESPIRATORY TRACT DISEASES
COMBINAISONS DE MÉDICAMENTS POUR LE TRAITEMENT DES INFECTIONS DES VOIES RESPIRATOIRES

(30) Priorität: 07.08.2006 EP 06118527
(43) Veröffentlichungstag der Anmeldung: 29.04.2009
(73) Patentinhaber: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: KONETZKI,Ingo, 88447 Warthausen (DE); BOUYSSOU, Thierry, 88447 Warthausen (DE); PESTEL, Sabine, 88448 Attenweiler (DE); SCHNAPP, Andreas, 88400 Biberach (DE)
(74) Vertreter: Hammann, Heinz
(86) Internationale Anmeldenummer: PCT/EP2007/058050
(87) Internationale Veröffentlichungsnummer: WO 2008/017637

(56) Entgegenhaltungen:
- WO-A-01/83462
- WO-A-03/000241
- WO-A-2005/102349
- WO-A-2006/087315

## Beschreibung

Die vorliegende Erfindung betrifft neue Arzneimittelkombinationen, die neben einer oder mehrerer, bevorzugt einer Verbindung der allgemeinen Formel **1** 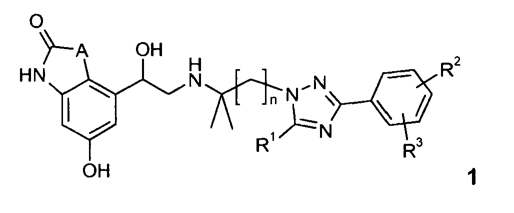 worin die Reste n, A, R¹, R² und R³ die in den Ansprüchen und in der Beschreibung genannten Bedeutungen haben können, wenigstens einen weiteren Wirkstoff **2** enthalten, Verfahren zu deren Herstellung sowie deren Verwendung als Arzneimittel.

### STAND DER TECHNIK

- WO 2001/83462 offenbart Betamimetika (β-adrenerge Substanzen) ähnlicher Struktur
- WO 2003/000241 offenbart Kombinationen auf der Basis von Anticholinergika, Corticosteroiden und Betamimetika, sowie deren Verwendung bei der Therapie von atemwegserkrankungen.
- WO 2005/102349 offenbart Arzneimittelkombinationen von Betamimetika mit weiteren Atemwegsmedikamenten aus den Klassen der Anticholinergika, PDE-IV-Inhibitoren, Steroide, LTD4-Antagonisten und EGFR-Hemmer.
- WO 2006/087315 ist eine nachveröffentlichte Schutzrechtsanmeldung die Verbindungen der allgemeinen Formel 1 offenbart.

### DETAILLIERTE BESCHREIBUNG DER ERFINDUNG

Die vorliegende Erfindung betrifft Arzneimittelkombinationen, die neben einer oder mehrerer, bevorzugt einer Verbindung der allgemeinen Formel **1** 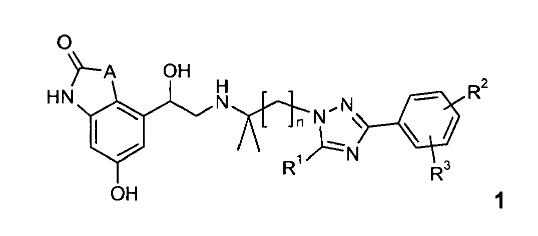 worin die Verbindung der Formel 1 ausgewählt ist aus der Gruppe bestehend aus:
**1.1:** 8-(2-{3-[3-(4-Fluor-phenyl)-5-methyl-[1,2,4]triazol-1-yl]-1,1-dimethyl-propylamino}-1-hydroxy-ethyl)-6-hydroxy-4H-benzo[1,4]oxazin-3-on;
**1.2:** 8-{2-[1,1-Dimethyl-3-(5-methyl-3-p-tolyl-[1,2,4]triazol-1yl)-propylamino]-1-hydroxyethyl}-6-hydroxy-4H-benzo[1,4]oxazin-3-on;
**1.3:** 8-(2-{1,1-Dimethyl-3-[5-methyl-3-(4-trifluormethyl-phenyl)-[1,2,4]triazol-1-yl]-propylamino}-1-hydroxy-ethyl)-6-hydroxy-4H-benzo[1,4]oxazin-3-on;
**1.4:** 8-(2-{3-[3-(3,5-Difluor-phenyl)-5-methyl-[1,2,4]triazol-1-yl]-1,1-dimethyl-propylamino}-1-hydroxy-ethyl)-6-hydroxy-4H-benzo[1,4]oxazin-3-on;
**1.6:** 8-(2-{3-[3-(4-Chlor-phenyl)-5-methyl-[1,2,4]triazol-1-yl]-1,1-dimethyl-propylamino}-1-hydroxy-ethyl)-6-hydroxy-4H-benzo[1,4]oxazin-3-on;
gegebenenfalls in Form der einzelnen optischen Isomeren, Mischungen der einzelnen Enantiomeren oder Racemate oder in Form der Säureadditionssalze mit pharmakologisch unbedenklichen Säuren sowie gegebenenfalls in Form der Solvate und/oder Hydrate enthalten;
wenigstens einen weiteren Wirkstoff **2** enthalten.

Bevorzugt betrifft die vorliegende Erfindung Arzneimittelkombinationen, die neben einer oder mehrere, bevorzugt einer Verbindung der Formel 1 als weiteren Wirkstoff **2** einen oder mehrere Verbindungen enthalten, die ausgewählt sind aus den Klassen der Anticholinergika **(2a),** PDE-IV-Inhibitoren **(2b),** Steroide **(2c),** LTD4-Antagonisten **(2d)** und EGFR-Hemmer **(2e).**

Ein weiterer Aspekt der vorliegenden Erfindung betrifft die vorstehend genannten neuen Verbindungen der Formel **1** in Form der einzelnen optischen Isomeren, Mischungen der einzelnen Enantiomeren oder Racemate. Besonders bevorzugt sind dabei Verbindungen der Formel **1** in Form der enantiomerenreinen Verbindungen, wobei die R-Enantiomere der Verbindungen der Formel **1** erfindungsgemäß von herausragender Bedeutung sind. Die R-Enantiomere der Verbindungen der Formel **1** sind darstellbar durch die allgemeine Formel ***R*-1** 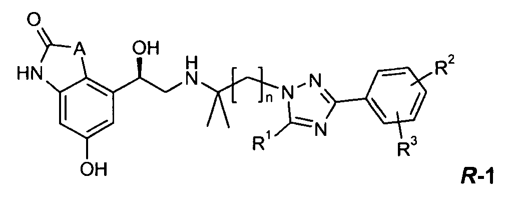 worin die Reste R¹, R² und R³ die vorstehend genannten Bedeutungen haben können. Verfahren zur Auftrennung von Racematen in die jeweiligen Enantiomere sind im Stand der Technik bekannt und können zur Darstellung der enantiomerenreinen R- bzw. S-Enantiomere der Verbindungen der Formel **1** in analoger Art und Weise zur Anwendung gelangen.

Wie vorstehend genannt, können die Verbindungen der Formel **1** in ihre Salze, insbesondere für die pharmazeutische Anwendung, in ihre physiologisch und pharmakologisch verträglichen Salze überführt werden. Diese Salze können einerseits als physiologisch und pharmakologisch verträgliche Säureadditionssalze der Verbindungen der Formel **1** mit anorganischen oder organischen Säuren vorliegen. Zur Darstellung der Säureadditionssalze kommen beispielsweise Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure in Betracht. Ferner können Mischungen der vorgenannten Säuren eingesetzt werden. Zur Darstellung der Alkali und Erdalkalimetallsalze der Verbindung der Formel **1** kommen vorzugsweise die Alkali und Erdalkalihydroxide und -hydride in Betracht, wobei die Hydroxide und Hydride der Alkalimetalle, besonders des Natriums und Kaliums bevorzugt, Natrium und Kaliumhydroxid besonders bevorzugt sind.

Gegebenenfalls können die Verbindungen der allgemeinen Formel **1** in ihre Salze, insbesondere für die pharmazeutische Anwendung, in ihre pharmakologisch unbedenklichen Säureadditionssalze mit einer anorganischen oder organischen Säure, überführt werden. Als Säuren kommen hierfür beispielsweise Bernsteinsäure, Bromwasserstoffsäure, Essigsäure, Fumarsäure, Maleinsäure, Methansulfonsäure, Milchsäure, Phosphorsäure, Salzsäure, Schwefelsäure, Weinsäure oder Zitronensäure in Betracht. Ferner können Mischungen der vorgenannten Säuren eingesetzt werden.

Gegenstand der Erfindung sind die jeweiligen Verbindungen gegebenenfalls in Form der einzelnen optischen Isomeren, Mischungen der einzelnen Enantiomeren oder Racemate, in Form der Tautomere sowie in Form der freien Basen oder der entsprechenden Säureadditionssalze mit pharmakologisch unbedenklichen Säuren wie beispielsweise Säureadditionssalze mit Halogenwasserstoffsäuren beispielsweise Chlor- oder Bromwasserstoffsäure oder organische Säuren wie beispielsweise Oxal-, Fumar-, Diglycol- oder Methansulfonsäure.

Bevorzugte Arzneimittelkombinationen enthalten neben ein oder mehrerer, bevorzugt einer Verbindung der Formel **1** als weiteren Wirkstoff ein oder mehrere, bevorzugt ein Anticholinergikum **2a,** gegebenenfalls in Kombination mit pharmazeutisch verträglichen Hilfsstoffen.

In den erfindungsgemäßen Arzneimittelkombinationen ist das Anticholinergikum **2a** bevorzugt ausgewählt aus der Gruppe bestehend aus Tiotropiumsalzen **(2a.1),** Oxitropiumsalzen **(2a.2),** Flutropiumsalzen **(2a.3),** Ipratropiumsalzen **(2a.4),** Glycopyrroniumsalzen **(2a.5),** Trospiumsalzen **(2a.6)** und den Verbindungen der Formeln **2a.7** bis **2a.13.**

In den vorstehend genannten Salzen **2a.1** bis **2a.6** stellen die Kationen Tiotropium, Oxitropium, Flutropium, Ipratropium, Glycopyrronium und Trospium die pharmakologisch aktiven Bestandteile dar. Eine explizite Bezugnahme auf die vorstehend genannten Kationen erfolgt durch die Bezeichnungen **2a.1'** bis **2a.6'.** Jede Bezugnahme auf die vorstehend genannten Salze **2a.1** bis **2a.6** schließt naturgemäß eine Bezugnahme auf die entsprechenden Kationen Tiotropium **(2a.1'),** Oxitropium **(2a.2'),** Flutropium **(2a.3'),** Ipratropium **(2a.4'),** Glycopyrronium **(2a.5'),** Trospium **(2a.6')** mit ein.

Unter den Salzen **2a.1** bis **2a.6** werden erfindungsgemäß diejenigen Verbindungen verstanden, die neben den Kationen Tiotropium **(2a.1'),** Oxitropium **(2a.2'),** Flutropium **(2a.3'),** Ipratropium **(2a.4'),** Glycopyrronium (2a.5') und Trospium **(2a.6')** als Gegenion (Anion) Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat enthalten, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt. Im Fall der Trospiumsalze **(2a.6)** ist das Chlorid besonders bevorzugt. Bei den anderen Salzen **2a.1** bis **2a.5** sind die Methansulfonate und Bromide von besonderer Bedeutung. Von besonderer Bedeutung sind Arzneimittelkombinationen, die Tiotropiumsalze **(2a.1),** Oxitropiumsalze **(2a.2)** oder Ipratropiumsalze **(2a.4)** enthalten, wobei die jeweiligen Bromide erfindungsgemäß besonders bedeutsam sind. Von besonderer Bedeutung ist das Tiotropiumbromid **(2a.1).** Die vorstehend genannten Salze können in den erfindungsgemäßen Arzneimittelkombinationen gegebenenfalls in Form ihrer Solvate oder Hydrate, bevorzugt in Form ihrer Hydrate vorliegen. Im Falle des Tiotropiumbromids enthalten die erfindungsgemäßen Arzneimittelkombinationen dieses bevorzugt in Form des kristallinen Tiotropiumbromid Monohydrats, welches aus der WO 02/30928 bekannt ist. Wird das Tiotropiumbromid in den erfindungsgemäßen Arzneimittelkombinationen in wasserfreier Form eingesetzt, so gelangt bevorzugt das wasserfreie kristalline Tiotropiumbromid zur Anwendung, welches aus der WO 03/000265 bekannt ist.

Beispiele für erfindungsgemäße bevorzugte Arzneimittelkombinationen bevorzugter Verbindungen der Formel **1** mit den vorstehend genannten Anticholinergikan **2a.1** bis **2a.6** sind Kombinationen enthaltend die Verbindungen **1.2** und **2a.1; 1.2** und **2a.2; 1.2** und **2a.3; 1.2** und **2a.4; 1.2** und **2a.5; 1.2** und **2a.6; 1.3** und **2a.1; 1.3** und **2a.2; 1.3** und **2a.3; 1.3** und **2a.4; 1.3** und **2a.5; 1.3** und **2a.6; 1.6** und **2a.1; 1.6** und **2a.2; 1.6** und **2a.3; 1.6** und **2a.4; 1.6** und **2a.5; 1.6** und **2a.6; 1.7** und **2a.1; 1.7** und **2a.2; 1.7** und **2a.3; 1.7** und **2a.4; 1.7** und **2a.5; 1.7** und **2a.6; 1.9** und **2a.1; 1.9** und **2a.2; 1.9** und **2a.3; 1.9** und **2a.4; 1.9** und **2a.5; 1.9** und **2a.6;** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate. Von den vorstehend genannten Kombinationen sind erfindungsgemäß ferner diejenigen bevorzugt, die als Verbindung **2a** eine der Verbindungen **2a.1, 2a.2** oder **2a.4** enthalten, wobei jene Kombinationen, die die Verbindung **2a.1** enthalten, erfindungsgemäß besonders bedeutsam sind.

Gegebenenfalls weisen die vorstehend genannten Anticholinergika chirale Kohlenstoffzentren auf. In diesem Fall können die erfindungsgemäßen Arzneimittelkombinationen die Anticholinergika in Form ihrer Enantiomere, Mischungen der Enantiomere oder Racemate enthalten, wobei vorzugsweise enantiomerenreine Anticholinergika zum Einsatz gelangen.

In einer ebenfalls bevorzugten Ausführungsform der vorliegenden Erfindung sind die in den erfindungsgemäßen Arzneimittelkombinationen enthaltenen Anticholinergika **2a** ausgewählt aus den Salzen der Formel **2a.7** 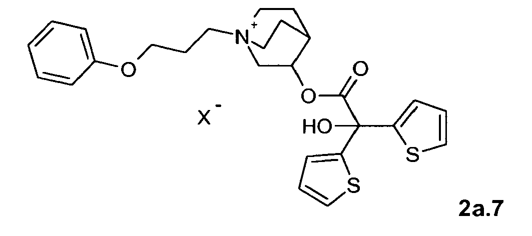 worin
- X⁻: ein einfach negativ geladenes Anion, bevorzugt ein Anion ausgewählt aus der Gruppe bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat bedeutet
gegebenenfalls in Form ihrer Racemate, Enantiomere oder Hydrate. Bevorzugte Arzneimittelkombinationen enthalten Salze der Formel **2a.7,** worin
- X⁻: ein einfach negativ geladenes Anion, bevorzugt ein Anion ausgewählt aus der Gruppe bestehend aus Fluorid, Chlorid, Bromid, Methansulfonat und p-Toluolsulfonat, bevorzugt Bromid, bedeutet,

gegebenenfalls in Form ihrer Racemate, Enantiomere oder Hydrate. Bevorzugte Arzneimittelkombinationen enthalten Salze der Formel **2a.7,** worin
- X⁻: ein einfach negativ geladenes Anion, bevorzugt ein Anion ausgewählt aus der Gruppe bestehend aus Chlorid, Bromid und Methansulfonat, bevorzugt Bromid, bedeutet,
gegebenenfalls in Form ihrer Racemate, Enantiomere oder Hydrate. Besonders bevorzugte Arzneimittelkombinationen enthalten die Verbindung der Formel **2a.7** in Form des Bromids. Von besonderer Bedeutung sind solche Arzneimittelkombinationen, die die Enantiomere der Formel **2a.7-en** 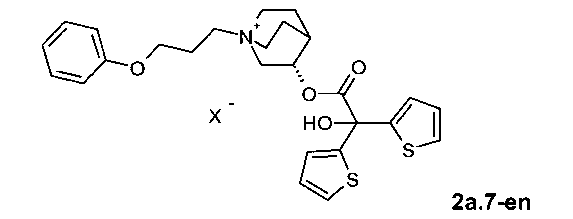 enthalten, worin X⁻ die vorstehend genannten Bedeutungen aufweisen kann.

Beispiele für erfindungsgemäße Arzneimittelkombinationen bevorzugter Verbindungen der Formel 1 mit den vorstehend genannten Anticholinergikan **2a.7** sind Kombinationen enthaltend die Verbindungen **1.2** und **2a.7; 1.2** und **2a.7-en; 1.3** und **2a.7; 1.3** und **2a.7-en; 1.6** und **2a.7; 1.6** und **2a.7-en; 1.7** und 2a.7; **1.7** und **2a.7-en; 1.9** und **2a.7; 1.9** und **2a.7-en;** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate.

In einer weiteren bevorzugten Ausführungsform der vorliegenden Erfindung sind die in den erfindungsgemäßen Arzneimittelkombinationen enthaltenen Anticholinergika **2a** ausgewählt aus den Salzen der Formel **2a.8** 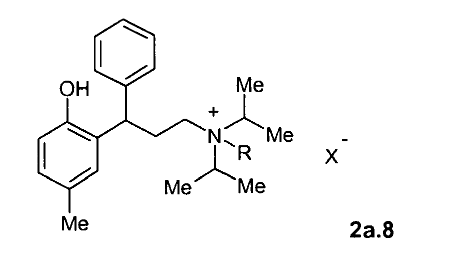 worin R entweder Methyl **(2a.8.1)** oder Ethyl **(2a.8.2)** bedeuten und worin X⁻ die vorstehend genannten Bedeutungen aufweisen kann. In einer alternativen Ausführungsform ist die Verbindung der Formel **2a.8** in Form der freien Base **2a.8-base** 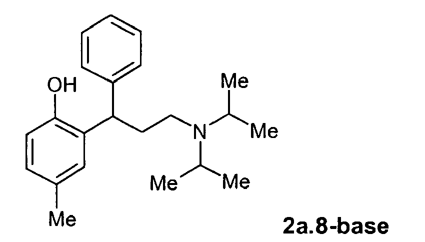 enthalten.

Die erfindungsgemäßen Arzneimittelkombinationen können das Anticholinergikum der Formel **2a.8** (oder **2a.8-base**) in Form ihrer Enantiomere, Mischungen der Enantiomere oder Racemate enthalten. Vorzugsweise sind die Anticholinergika der Formel **2a.8** (oder **2a.8-base**) in Form ihrer R-Enantiomere enthalten.

Beispiele für erfindungsgemäße Arzneimittelkombinationen bevorzugter Verbindungen der Formel 1 mit den vorstehend genannten Anticholinergikan **2a.8** sind Kombinationen enthaltend die Verbindungen **1.2** und **2a.8.1; 1.2** und **2a.8.2;** 1.3 und **2a.8.1;** 1.3 und **2a.8.2; 1.6** und **2a.8.1; 1.6** und **2a.8.2;** 1.7 und **2a.8.1; 1.7** und **2a.8.2; 1.9** und **2a.8.1; 1.9** und **2a.8.2;** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate.

In einer weiteren bevorzugten Ausführungsform der vorliegenden Erfindung sind die in den erfindungsgemäßen Arzneimittelkombinationen enthaltenen Anticholinergika **2a** ausgewählt aus den Verbindungen der Formel **2a.9** 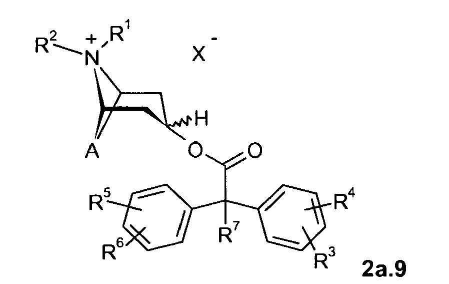 worin
- A: eine zweibindige Gruppe ausgewählt aus den Gruppen 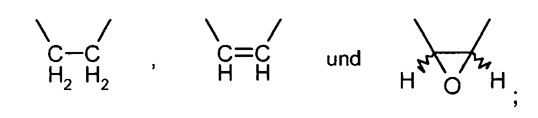
- X⁻: eine der vorstehend genannten einfach negativ geladenen Anionen, bevorzugt Chlorid, Bromid oder Methansulfonat,
- R¹ und R²: gleich oder verschieden eine Gruppe ausgewählt aus Methyl, Ethyl, n-Propyl und iso-Propyl, die gegebenenfalls substituiert sein kann durch Hydroxy oder Fluor, bevorzugt unsubstituiertes Methyl;
- R³, R⁴, R⁵ und R⁶,: gleich oder verschieden Wasserstoff, Methyl, Ethyl, Methyloxy, Ethyloxy, Hydroxy, Fluor, Chlor, Brom, CN, CF₃ oder NO₂;
- R⁷: Wasserstoff, Methyl, Ethyl, Methyloxy, Ethyloxy, -CH₂-F, -CH₂-CH₂-F, -O-CH₂F, -O-CH₂CH₂F, -CH₂OH, -CH₂CH₂OH, CF₃, -CH₂-OMe, -CH₂-CH₂-OMe, -CH₂-OEt, -CH₂-CH₂-OEt, -O-COMe, -O-COEt, -O-COCF₃, -O-COCF₃, Fluor, Chlor oder Brom bedeuten.

Die Verbindungen der Formel 2a.9 sind im Stand der Technik bekannt (WO 02/32899).

Im Rahmen der erfindungsgemäßen Arzneimittelkombinationen bevorzugte Verbindungen der Formel 2a.9 sind solche, in denen
- X⁻: Bromid;
- R¹ und R²: gleich oder verschieden, Methyl oder Ethyl, bevorzugt Methyl;
- R³, R⁴, R⁵ und R⁶,: gleich oder verschieden, Wasserstoff, Methyl, Methyloxy, Chlor oder Fluor;
- R⁷: Wasserstoff, Methyl oder Fluor bedeuten.

Von besonderer Bedeutung sind Arzneimittelkombinationen, die solche Verbindungen der Formel 2a.9 enthalten, in denen
- A: eine zweibindige Gruppe ausgewählt aus

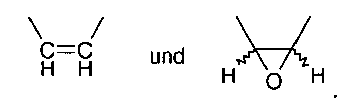

Von besonderer Bedeutung sind diejenigen Arzneimittelkombinationen, die neben einer Verbindung der Formel **1** eine der nachfolgenden Verbindungen der Formel **2a.9** enthalten:
- 2,2-Diphenylpropionsäuretropenolester-methobromid **(2a.9.1),**
- 2,2-Diphenylpropionsäurescopinester-methobromid **(2a.9.2),**
- 2-Fluor-2,2-Diphenylessigsäurescopinester-methobromid **(2a.9.3),**
- 2-Fluor-2,2-Diphenylessigsäuretropenolester-methobromid **(2a.9.4).**

Die Verbindungen der Formel **2a.9** können gegebenenfalls in Form ihrer Enantiomere, Mischungen ihrer Enantiomere oder Racemate, sowie gegebenenfalls in Form ihrer Hydrate und/oder Solvate enthalten sein.

Beispiele für erfindungsgemäße Arzneimittelkombinationen bevorzugter Verbindungen der Formel 1 mit den vorstehend genannten Anticholinergikan **2a.9** sind Kombinationen enthaltend die Verbindungen **1.2** und **2a.9.1; 1.2** und **2a.9.2; 1.2** und **2a.9.3; 1.2** und **2a.9.4; 1.3** und **2a.9.1; 1.3** und **2a.9.2; 1.3** und **2a.9.3; 1.3** und **2a.9.4; 1.6** und **2a.9.1; 1.6** und **2a.9.2; 1.6** und **2a.9.3; 1.6** und **2a.9.4; 1.7** und **2a.9.1; 1.7** und **2a.9.2; 1.7** und **2a.9.3; 1.7** und **2a.9.4; 1.9** und **2a.9.1; 1.9** und **2a.9.2; 1.9** und **2a.9.3; 1.9** und **2a.9.4;** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate. Von den vorstehend genannten Kombinationen sind erfindungsgemäß ferner diejenigen bevorzugt, die als Verbindung **2a.9** eine der Verbindungen **2a.9.1** oder **2a.9.2** enthalten, wobei jene Kombinationen, die die Verbindung **2a.9.2** enthalten, erfindungsgemäß besonders bedeutsam sind.

In einer weiteren bevorzugten Ausführungsform der vorliegenden Erfindung sind die in den erfindungsgemäßen Arzneimittelkombinationen enthaltenen Anticholinergika **2a** ausgewählt aus den Verbindungen der Formel **2a.10** 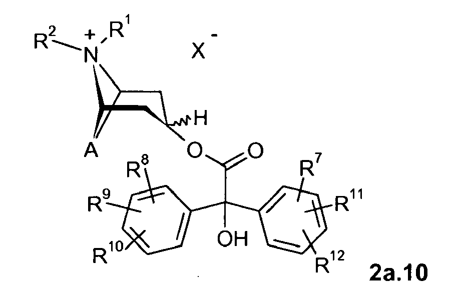 worin
A, X R¹ und R² die vorstehend genannten Bedeutungen haben können und worin R⁷, R⁸, R⁹, R¹⁰, R¹¹ und R¹², gleich oder verschieden, Wasserstoff, Methyl, Ethyl, Methyloxy, Ethyloxy, Hydroxy, Fluor, Chlor, Brom, CN, CF₃ oder NO₂, bedeuten, wobei zumindest eine der Gruppen R⁷, R⁸, R⁹, R¹⁰, R¹¹ und R¹² nicht Wasserstoff sein kann.

Die Verbindungen der Formel **2a.1 0** sind im Stand der Technik bekannt (WO 02/32898).

Im Rahmen der erfindungsgemäßen Arzneimittelkombinationen bevorzugte Verbindungen der Formel **2a.10** sind solche, in denen
- A: eine zweibindige Gruppe ausgewählt aus 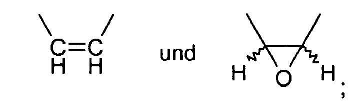
- X⁻: Bromid;
- R¹ und R²: gleich oder verschieden, Methyl oder Ethyl, bevorzugt Methyl;
- R⁷, R⁸, R⁹, R¹⁰, R¹¹ und R¹²,: gleich oder verschieden Wasserstoff, Fluor, Chlor oder Brom, bevorzugt Fluor, bedeuten, wobei zumindest eine der Gruppen R⁷, R⁸, R⁹, R¹⁰, R¹¹ und R¹² nicht Wasserstoff sein kann.

Von besonderer Bedeutung sind diejenigen Arzneimittelkombinationen, die neben einer Verbindung der Formel 1 eine der nachfolgenden Verbindungen der Formel **2a.10** enthalten:
- 3,3',4,4'-Tetrafluorbenzilsäuretropenolester-Methobromid **(2a.10.1),**
- 3,3',4,4'-Tetrafluorbenzilsäurescopinester-Methobromid **(2a.10.2),**
- 4,4'-Difluorbenzilsäuretropenolester-Methobromid **(2a.10.3),**
- 4,4'-Difluorbenzilsäurescopinester-Methobromid **(2a.10.4),**
- 3,3'-Difluorbenzilsäuretropenolester-Methobromid **(2a.10.5),**
- 3,3'-Difluorbenzilsäurescopinester-Methobromid **(2a.10.6).**

Die Verbindungen der Formel **2a.10** können gegebenenfalls in Form ihrer Enantiomere, Mischungen ihrer Enantiomere oder Racemate, sowie gegebenenfalls in Form ihrer Hydrate und/oder Solvate enthalten sein.

Beispiele für erfindungsgemäße Arzneimittelkombinationen bevorzugter Verbindungen der Formel 1 mit den vorstehend genannten Anticholinergikan **2a.10** sind Kombinationen enthaltend die Verbindungen **1.2** und **2a.10.1; 1.2** und **2a.10.2; 1.2** und **2a.10.3; 1.2** und **2a.10.4; 1.2** und **2a.10.5; 1.2** und **2a.10.6;1.3** und **2a.10.1; 1.3** und **2a.10.2; 1.3** und **2a.10.3; 1.3** und **2a.10.4; 1.3** und **2a.10.5; 1.3** und **2a.10.6; 1.6** und **2a.10.1; 1.6** und **2a.10.2; 1.6** und **2a.10.3; 1.6** und **2a.10.4; 1.6** und **2a.10.5; 1.6** und **2a.10.6; 1.7** und **2a.10.1; 1.7** und **2a.10.2; 1.7** und **2a.10.3; 1.7** und **2a.10.4; 1.7** und **2a.10.5; 1.7** und **2a.10.6; 1.9** und **2a.10.1; 1.9** und **2a.10.2; 1.9** und **2a.10.3; 1.9** und **2a.10.4; 1.9** und **2a.10.5; 1.9** und **2a.10.6;** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate. Von den vorstehend genannten Kombinationen sind erfindungsgemäß ferner diejenigen bevorzugt, die als Verbindung **2a.10** eine der Verbindungen **2a.10.1, 2a.10.2 , 2a.10.3** oder **2a.10.4** enthalten, wobei jene Kombinationen, die die Verbindungen **2a.10.1** oder **2a.1 0.2** enthalten, erfindungsgemäß besonders bedeutsam sind.

In einer weiteren bevorzugten Ausführungsform der vorliegenden Erfindung sind die in den erfindungsgemäßen Arzneimittelkombinationen enthaltenen Anticholinergika **2a** ausgewählt aus den Verbindungen der Formel **2a.11** 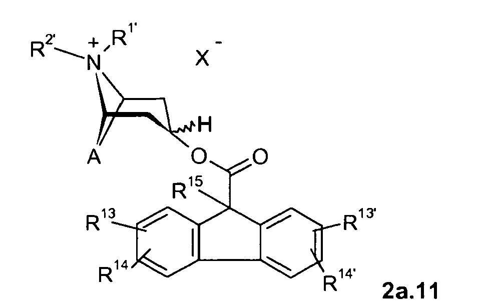 worin
- A und X⁻: die vorstehend genannten Bedeutungen haben können und worin
- R¹⁵: Wasserstoff, Hydroxy, Methyl, Ethyl, -CF₃, CHF₂ oder Fluor;
- R^{1'} und R^{2'}: gleich oder verschieden, C₁-C₅-Alkyl, welches gegebenenfalls substituiert sein kann durch C₃-C₆-Cycloalkyl, Hydroxy oder Halogen, oder R^{1'} und R^{2'} gemeinsam eine -C₃-C₅-Alkylenbrücke;
- R¹³, R¹⁴, R^{13'} und R^{14'}: gleich oder verschieden, Wasserstoff, -C₁-C₄-Alkyl, -C₁-C₄-Alkyloxy, Hydroxy, -CF₃, -CHF₂, CN, NO₂ oder Halogen bedeuten.

Die Verbindungen der Formel **2a.11** sind im Stand der Technik bekannt (WO 03/064419).

Im Rahmen der erfindungsgemäßen Arzneimittelkombinationen bevorzugte Verbindungen der Formel **2a.11** sind solche, in denen
- A: eine zweibindige Gruppe ausgewählt aus 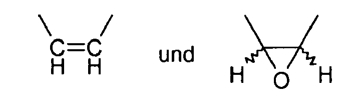
- X⁻: ein Anion ausgewählt aus Chlorid, Bromid und Methansulfonat, bevorzugt Bromid;
- R¹⁵: Hydroxy, Methyl oder Fluor, bevorzugt Methyl oder Hydroxy;
- R^{1'} und R^{2'}: gleich oder verschieden, Methyl oder Ethyl, bevorzugt Methyl;
- R¹³, R¹⁴, R^{13'} und R^{14'}: gleich oder verschieden, Wasserstoff, -CF₃, -CHF₂ oder Fluor, bevorzugt Wasserstoff oder Fluor bedeuten.

Im Rahmen der erfindungsgemäßen Arzneimittelkombinationen besonders bevorzugte Verbindungen der Formel **2a.11** sind solche, in denen
- A: eine zweibindige Gruppe ausgewählt aus 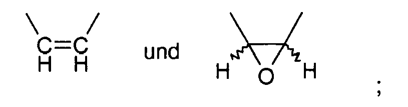
- X⁻: Bromid;
- R¹⁵: Hydroxy oder Methyl, bevorzugt Methyl;
- R^{1'} und R^{2'}: gleich oder verschieden, Methyl oder Ethyl, bevorzugt Methyl;
- R¹³, R¹⁴, R^{13'} und R^{14'}: gleich oder verschieden, Wasserstoff oder Fluor bedeuten.

Von besonderer Bedeutung sind diejenigen Arzneimittelkombinationen, die neben einer Verbindung der Formel **1** eine der nachfolgenden Verbindungen der Formel **2a.11** enthalten:
- 9-Hydroxy-fluoren-9-carbonsäuretropenolester -Methobromid **(2a.11.1);**
- 9-Fluor-fluoren-9-carbonsäuretropenolester -Methobromid **(2a.11.2);**
- 9-Hydroxy-fluoren-9-carbonsäurescopinester -Methobromid **(2a.11.3);**
- 9-Fluor-fluoren-9-carbonsäurescopinester Methobromid **(2a.11.4);**
- 9-Methyl-fluoren-9-carbonsäuretropenolester Methobromid **(2a.11.5);**
- 9-Methyl-fluoren-9-carbonsäurescopinester Methobromid **(2a.11.6);**

Die Verbindungen der Formel **2a.11** können gegebenenfalls in Form ihrer Enantiomere, Mischungen ihrer Enantiomere oder Racemate, sowie gegebenenfalls in Form ihrer Hydrate und/oder Solvate enthalten sein.

Beispiele für erfindungsgemäße Arzneimittelkombinationen bevorzugter Verbindungen der Formel 1 mit den vorstehend genannten Anticholinergikan **2a.11** sind Kombinationen enthaltend die Verbindungen **1.2** und **2a.11.1; 1.2** und **2a.11.2; 1.2** und **2a.11.3; 1.2** und **2a.11.4; 1.2** und **2a.11.5; 1.2** und **2a.11.6;1.3** und **2a.11.1; 1.3** und **2a.11.2; 1.3** und **2a.11.3; 1.3** und **2a.11.4; 1.3** und **2a.11.5; 1.3** und **2a.11.6; 1.6** und **2a.11.1; 1.6** und **2a.11.2; 1.6** und **2a.11.3; 1.6** und **2a.11.4; 1.6** und **2a.11.5; 1.6** und **2a.11.6; 1.7** und **2a.11.1; 1.7** und **2a.11.2; 1.7** und **2a.11.3; 1.7** und **2a.11.4; 1.7** und **2a.11.5; 1.7** und **2a.11.6; 1.9** und **2a.11.1; 1.9** und **2a.11.2; 1.9** und **2a.11.3; 1.9** und **2a.11.4; 1.9** und **2a.11.5; 1.9** und **2a.11.6;** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate. Von den vorstehend genannten Kombinationen sind erfindungsgemäß ferner diejenigen bevorzugt, die als Verbindung **2a.11** eine der Verbindungen **2a.11.2, 2a.11.4 , 2a.11.5** oder **2a.11.6** enthalten, wobei jene Kombinationen, die die Verbindungen 2a.11.5 oder **2a.11.6** enthalten, erfindungsgemäß besonders bedeutsam sind.

In einer weiteren bevorzugten Ausführungsform der vorliegenden Erfindung sind die in den erfindungsgemäßen Arzneimittelkombinationen enthaltenen Anticholinergika **2a** ausgewählt aus den Verbindungen der Formel **2a.12** 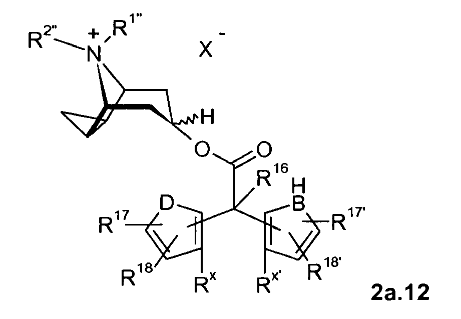 worin X⁻ die vorstehend genannten Bedeutungen haben kann und worin
- D und B: gleich oder verschieden, bevorzugt gleich O, S, NH, CH₂, CH=CH oder N(C₁-C₄-Alkyl);
- R¹⁶: Wasserstoff, Hydroxy, -C₁-C₄-Alkyl, -C₁-C₄-Alkyloxy, -C₁-C₄-Alkylen-Halogen, -O-C₁-C₄-Alkylen-Halogen, -C₁-C₄-Alkylen-OH, -CF₃, CHF₂, -C₁-C₄-Alkylen-C₁-C₄-alkyloxy, -O-COC₁-C₄-Alkyl, -O-COC₁-C₄-Alkylen-halogen, -C₁-C₄-Alkylen-C₃-C₆-cycloalkyl, -O-COCF₃ oder Halogen;
- R^{1"} und R^{2"}: gleich oder verschieden, -C₁-C₅-Alkyl, das gegebenenfalls durch -C₃-C₆-Cycloalkyl, Hydroxy oder Halogen substituiert sein kann, oder R^{1"} und R^{2"} gemeinsam eine -C₃-C₅-Alkylenbrücke;
- R¹⁷, R¹⁸, R^{17'} und R^{18'},: gleich oder verschieden, Wasserstoff, -C₁C₄-Alkyl, -C₁-C₄-Alkyloxy, Hydroxy, -CF₃, -CHF₂, CN, NO₂ oder Halogen;
- R^{x} und R^{x'}: gleich oder verschieden, Wasserstoff, -C₁-C₄-Alkyl, -C₁-C₄-Alkyloxy, Hydroxy, -CF₃, -CHF₂, CN, NO₂ oder Halogen, oder R^{x} und R^{x'} gemeinsam eine Einfachbindung oder eine der zweibindigen Gruppen O, S, NH, CH₂, CH₂-CH₂, N(C₁-C₄-alkyl), CH(C₁-C₄-alkyl) und -C(C₁-C₄-alkyl)₂ bedeuten.

Die Verbindungen der Formel **2a.12** sind im Stand der Technik bekannt (WO 03/064418).

Im Rahmen der erfindungsgemäßen Arzneimittelkombinationen bevorzugte Verbindungen der Formel **2a.12** sind solche, in denen
- X⁻: Chlorid, Bromid oder Methansulfonat, bevorzugt Bromid;
- D und B: gleich oder verschieden, bevorzugt gleich O, S, NH oder CH=CH;
- R¹⁶: Wasserstoff, Hydroxy, -C₁-C₄-Alkyl, -C₁-C₄-Alkyloxy, -CF₃, -CHF₂, Fluor, Chlor oder Brom;
- R^{1"} und R^{2"}: gleich oder verschieden, C₁-C₄-Alkyl, welches gegebenenfalls substituiert sein kann durch Hydroxy, Fluor, Chlor oder Brom, oder R^{1"} und R^{2"} gemeinsam eine -C₃-C₄-Alkylenbrücke;
- R¹⁷, R¹⁸, R^{17'} und R^{18'},: gleich oder verschieden, Wasserstoff, C₁-C₄-Alkyl, C₁-C₄-Alkyloxy, Hydroxy, -CF₃, -CHF₂, CN, NO₂, Fluor, Chlor oder Brom;
- R^{x} und R^{x'}: gleich oder verschieden, Wasserstoff, C₁-C₄-Alkyl, C₁-C₄-Alkyloxy, Hydroxy, -CF₃, -CHF₂, CN, NO₂, Fluor, Chlor oder Brom, oder R^{x} und R^{x'} gemeinsam eine Einfachbindung oder eine zweibindige Gruppe ausgewählt aus O, S, NH- und CH₂ bedeuten.

Im Rahmen der erfindungsgemäßen Arzneimittelkombinationen besonders bevorzugte Verbindungen der Formel **2a.12** sind solche, in denen
- X⁻: Chlorid, Bromid, oder Methansulfonat, bevorzugt Bromid;
- D und B: gleich oder verschieden, bevorzugt gleich, S oder CH=CH;
- R¹⁶: Wasserstoff, Hydroxy oder Methyl;
- R^{1"} und R^{2"}: gleich oder verschieden, Methyl oder Ethyl;
- R¹⁷, R¹⁸, R^{17'} und R^{18'},: gleich oder verschieden, Wasserstoff, -CF₃ oder Fluor, bevorzugt Wasserstoff;
- R^{x} und R^{x'}: gleich oder verschieden, Wasserstoff, -CF₃ oder Fluor, bevorzugt Wasserstoff, oder R^{x} und R^{x'} gemeinsam eine Einfachbindung oder -O bedeuten.

Im Rahmen der erfindungsgemäßen Arzneimittelkombinationen besonders bevorzugte Verbindungen der Formel **2a.12** sind ferner solche, in denen
- X⁻: Bromid;
- D und B: -CH=CH-;
- R¹⁶: Wasserstoff, Hydroxy oder Methyl;
- R^{1"} und R^{2"}: Methyl;
- R¹⁷, R¹⁸, R^{17'} und R^{18'},: gleich oder verschieden, Wasserstoff oder Fluor, bevorzugt Wasserstoff;
- R^{x} und R^{x'}: gleich oder verschieden, Wasserstoff oder Fluor, bevorzugt Wasserstoff, oder R^{x} und R^{x'} gemeinsam eine Einfachbindung oder die Gruppe -O bedeuten.

Von besonderer Bedeutung sind diejenigen Arzneimittelkombinationen, die neben einer Verbindung der Formel **1** eine der nachfolgenden Verbindungen der Formel **2a.12** enthalten:
- Benzilsäurecyclopropyltropinester-Methobromid **(2a.12.1);**
- 2,2-Diphenylpropionsäurecyclopropyltropinester -Methobromid **(2a.12.2);**
- 9-Hydroxy-xanthen-9-carbonsäurecyclopropyltropinester-Methobromid **(2a.12.3);**
- 9-Methyl-fluoren-9-carbonsäurecyclopropyltropinester-Methobromid **(2a.12.4);**
- 9-Methyl-xanthen-9-carbonsäurecyclopropyltropinester -Methobromid **(2a.12.5);**
- 9-Hydroxy-fluoren-9-carbonsäurecyclopropyltropinester -Methobromid **(2a.12.6);**
- 4,4'-Difluorbenzilsäuremethylestercyclopropyltropinester -Methobromid **(2a.12.7).**

Die Verbindungen der Formel **2a.12** können gegebenenfalls in Form ihrer Enantiomere, Mischungen ihrer Enantiomere oder Racemate, sowie gegebenenfalls in Form ihrer Hydrate und/oder Solvate enthalten sein.

Beispiele für erfindungsgemäße Arzneimittelkombinationen bevorzugter Verbindungen der Formel **1** mit den vorstehend genannten Anticholinergikan **2a.12** sind Kombinationen enthaltend die Verbindungen **1.2** und **2a.12.1; 1.2** und **2a.12.2; 1.2** und **2a.12.3; 1.2** und **2a.12.4; 1.2** und **2a.12.5; 1.2** und **2a.12.6; 1.2** und **2a.12.7; 1.3** und **2a.12.1; 1.3** und **2a.12.2; 1.3** und **2a.12.3; 1.3** und **2a.12.4; 1.3** und **2a.12.5; 1.3** und **2a.12.6; 1.3** und **2a.12.7; 1.6** und **2a.12.1; 1.6** und **2a.12.2; 1.6** und **2a.12.3; 1.6** und **2a.12.4; 1.6** und **2a.12.5; 1.6** und **2a.12.6; 1.6** und **2a.12.7; 1.7** und **2a.12.1; 1.7** und **2a.12.2; 1.7** und **2a.12.3; 1.7** und **2a.12.4; 1.7** und **2a.12.5; 1.7** und **2a.12.6; 1.7** und **2a.12.7; 1.9** und **2a.12.1; 1.9** und **2a.12.2; 1.9** und **2a.12.3; 1.9** und **2a.12.4; 1.9** und **2a.12.5; 1.9** und **2a.12.6; 1.9** und **2a.12.7;** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate. Von den vorstehend genannten Kombinationen sind erfindungsgemäß ferner diejenigen bevorzugt, die als Verbindung **2a.12** eine der Verbindungen **2a.12.1, 2a.12.2, 2a.12.5** oder **2a.12.7** enthalten, wobei jene Kombinationen, die die Verbindungen **2a.12.1** oder **2a.12.2** enthalten, erfindungsgemäß besonders bedeutsam sind.

In einer weiteren bevorzugten Ausführungsform der vorliegenden Erfindung sind die in den erfindungsgemäßen Arzneimittelkombinationen enthaltenen Anticholinergika **2a** ausgewählt aus den Verbindungen der Formel **2a.13** 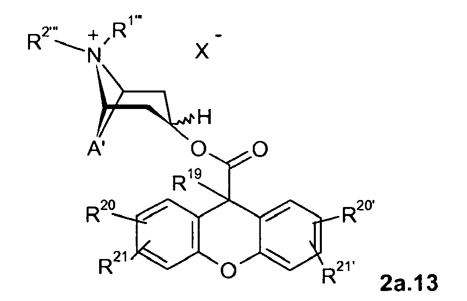 worin X -die vorstehend genannten Bedeutungen haben kann und worin
- A': eine zweibindige Gruppe ausgewählt aus 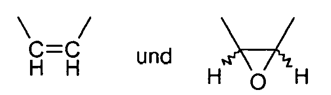
- R¹⁹: Hydroxy, Methyl, Hydroxymethyl, Ethyl, -CF₃, CHF₂ oder Fluor;
- R^{1'"} und R^{2'"}: gleich oder verschieden, C₁-C₅-Alkyl, welches gegebenenfalls substituiert sein kann durch C₃-C₆-Cycloalkyl, Hydroxy oder Halogen, oder R^{1'"} und R^{2'"} gemeinsam eine -C₃-C₅-Alkylenbrücke;
- R²⁰, R²¹, R^{20'} und R^{21'}: gleich oder verschieden, Wasserstoff, -C₁-C₄-Alkyl, -C₁-C₄-Alkyloxy, Hydroxy, -CF₃, -CHF₂, CN, NO₂ oder Halogen bedeuten.

Die Verbindungen der Formel **2a.13** sind im Stand der Technik bekannt (WO 03/064417).

Im Rahmen der erfindungsgemäßen Arzneimittelkombinationen bevorzugte Verbindungen der Formel **2a.13** sind solche, in denen
- A': eine zweibindige Gruppe ausgewählt aus

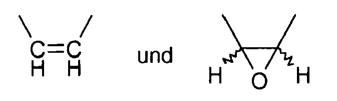

- X⁻: Chlorid, Bromid oder Methansulfnat, bevorzugt Bromid;
- R¹⁹: Hydroxy oder Methyl;
- R^{1'"} und R^{2'"}: gleich oder verschieden, Methyl oder Ethyl, bevorzugt Methyl;
- R²⁰, R²¹, R^{20'} und R^{21'}: gleich oder verschieden, Wasserstoff, -CF₃, -CHF₂ oder Fluor, bevorzugt Wasserstoff oder Fluor bedeuten.

Im Rahmen der erfindungsgemäßen Arzneimittelkombinationen besonders bevorzugte Verbindungen der Formel **2a.13** sind solche, in denen
- A': eine zweibindige Gruppe ausgewählt aus 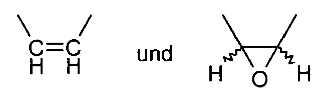
- X⁻: Bromid;
- R¹⁹: Hydroxy oder Methyl, bevorzugt Methyl;
- R^{1'"} und R^{2'"}: gleich oder verschieden, Methyl oder Ethyl, bevorzugt Methyl;
- R²⁰, R²¹, R^{20'} und R^{21'}: gleich oder verschieden, Wasserstoff oder Fluor bedeuten.

Von besonderer Bedeutung sind diejenigen Arzneimittelkombinationen, die neben einer Verbindung der Formel 1 eine der nachfolgenden Verbindungen der Formel **2a.13** enthalten:
- 9-Hydroxy-xanthen-9-carbonsäuretropenolester -Methobromid **(2a.13.1);**
- 9-Hydroxy-xanthen-9-carbonsäurescopinester Methobromid **(2a.13.2);**
- 9-Methyl-xanthen-9-carbonsäuretropenolester -Methobromid **(2a.13.3);**
- 9-Methyl-xanthen-9-carbonsäurescopinester -Methobromid **(2a.13.4);**
- 9-Ethyl-xanthen-9-carbonsäuretropenolester Methobromid **(2a.13.5);**
- 9-Difluormethyl-xanthen-9-carbonsäuretropenolester -Methobromid **(2a.13.6);**
- 9-Hydroxymethyl-xanthen-9-carbonsäurescopinester -Methobromid **(2a.13.7).**

Die Verbindungen der Formel **2a.13** können gegebenenfalls in Form ihrer Enantiomere, Mischungen ihrer Enantiomere oder Racemate, sowie gegebenenfalls in Form ihrer Hydrate und/oder Solvate enthalten sein.

Beispiele für erfindungsgemäße Arzneimittelkombinationen bevorzugter Verbindungen der Formel **1** mit den vorstehend genannten Anticholinergikan **2a.13** sind Kombinationen enthaltend die Verbindungen **1.2** und **2a.13.1; 1.2** und **2a.13.2; 1.2** und **2a.13.3; 1.2** und **2a.13.4; 1.2** und **2a.13.5; 1.2** und **2a.13.6; 1.2** und **2a.13.7; 1.3** und **2a.13.1; 1.3** und **2a.13.2; 1.3** und **2a.13.3; 1.3** und **2a.13.4; 1.3** und **2a.13.5; 1.3** und **2a.13.6; 1.3** und **2a.13.7; 1.6** und **2a.13.1; 1.6** und **2a.13.2; 1.6** und **2a.13.3; 1.6** und **2a.13.4; 1.6** und **2a.13.5; 1.6** und **2a.13.6; 1.6** und **2a.13.7; 1.7** und **2a.13.1; 1.7** und **2a.13.2; 1.7** und **2a.13.3; 1.7** und **2a.13.4; 1.7** und **2a.13.5; 1.7** und **2a.13.6; 1.7** und **2a.13.7; 1.9** und **2a.13.1; 1.9** und **2a.13.2; 1.9** und **2a.13.3; 1.9** und **2a.13.4; 1.9** und **2a.13.5; 1.9** und **2a.13.6; 1.9** und **2a.13.7;** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate. Von den vorstehend genannten Kombinationen sind erfindungsgemäß ferner diejenigen bevorzugt, die als Verbindung **2a.11** eine der Verbindungen **2a.13.2, 2a.13.3, 2a.13.4** oder **2a.13.5** enthalten, wobei jene Kombinationen, die die Verbindungen **2a.13.3** oder **2a.13.4** enthalten, erfindungsgemäß besonders bedeutsam sind.

Im Rahmen der vorliegenden Erfindung ist eine Bezugnahme auf Anticholinergika 1' als Bezugnahme auf die pharmakologisch aktiven Kationen der jeweiligen Salze zu verstehen. Diese Kationen sind Tiotropium **(2a.1'),** Oxitropium **(2a.2'),** Flutropium **(2a.3'),** Ipratropium **(2a.4'),** Glycopyrronium **(2a.5'),** Trospium **(2a.6')** sowie die nachstehenden Kationen 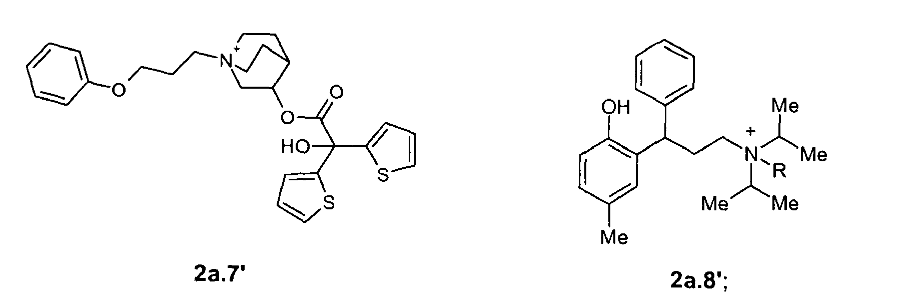 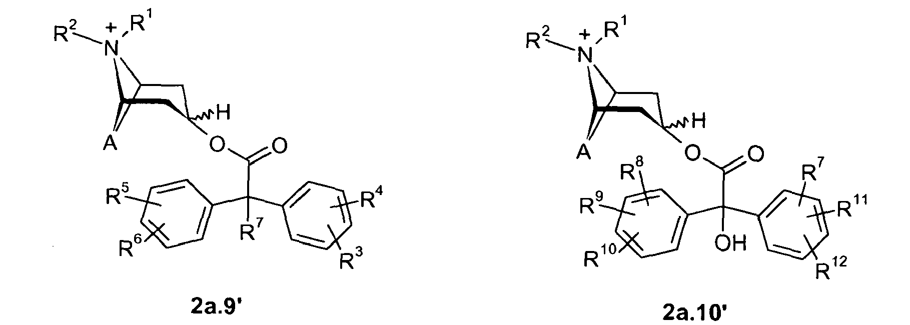 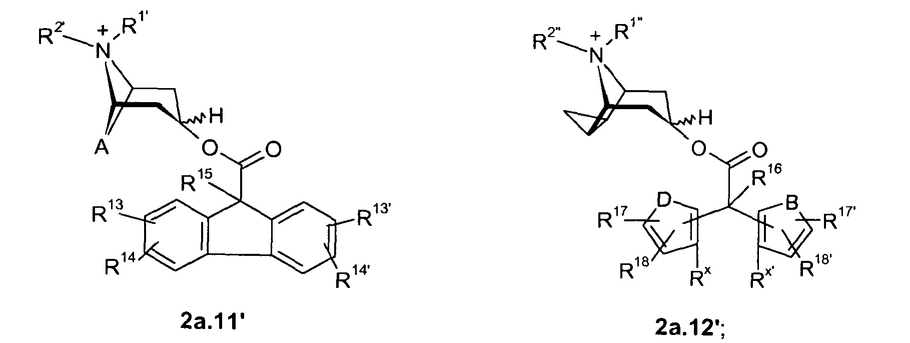 oder

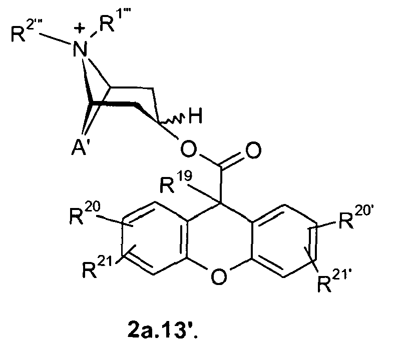

Erfindungsgemäß ferner bevorzugte Arzneimittelkombinationen enthalten neben ein oder mehrerer, bevorzugt einer Verbindung der Formel **1** als weiteren Wirkstoff ein oder mehrere, bevorzugt einen PDE IV-Inhibitor **2b,** gegebenenfalls in Kombination mit pharmazeutisch verträglichen Hilfsstoffen.

In solchen Arzneimittelkombinationen ist der PDE IV-Inhibitor **2b** bevorzugt ausgewählt aus der Gruppe bestehend aus Enprofyllin, Theophyllin, Roflumilast, Ariflo (Cilomilast), CP-325,366, BY343, D-4396 (Sch-351591), AWD-12-281 (GW-842470), N-(3,5-Dichloro-1-oxo-pyridin-4-yl)-4-difluoromethoxy-3-cyclopropylmethoxybenzamid, NCS-613, Pumafentine, (-)p-[(4*a*R*,10*b*S*)-9-Ethoxy-1,2,3,4,4a,10b-hexahydro-8-methoxy-2-methylbenzo[s][1,6]naphthyridin-6-yl]-N,N-diisopropylbenzamid, (R)-(+)-1-(4-Bromobenzyl)-4-[(3-cyclopentyloxy)-4-methoxyphenyl]-2-pyrrolidon, 3-(Cyclopentyloxy-4-methoxyphenyl)-1-(4-N'-[N-2-cyano-S-methyl-isothioureido]benzyl)-2-pyrrolidon, cis[4-Cyano-4-(3-cyclopentyloxy-4-methoxyphenyl)cyclohexan-1-carbonsäure], 2-carbomethoxy-4-cyano-4-(3-cyclopropylmethoxy-4-difluoromethoxyphenyl)cyclohexan-1-on, cis[4-Cyano-4-(3-cyclopropylmethoxy-4-difluoromethoxyphenyl)cyclohexan-1-ol], (R)-(+)-Ethyl[4-(3-cyclopentyloxy-4-methoxyphenyl)pyrrolidin-2-yliden]acetat, (S)-(-)-Ethyl[4-(3-cyclopentyloxy-4-methoxyphenyl)pyrrolidin-2-yliden]acetat, CDP840, Bay-198004, D-4418, PD-168787, T-440, T-2585, Arofyllin, Atizoram, V-11294A, CI-1018, CDC-801, CDC-3052, D-22888, YM-58997, Z-15370, 9-Cyclopentyl-5,6-dihydro-7-ethyl-3-(2-thienyl)-9*H*-pyrazolo[3,4-c]-1,2,4-triazolo[4,3-a]pyridin und 9-Cyclopentyl-5,6-dihydro-7-ethyl-3-(*tert*-butyl)-9*H*-pyrazolo[3,4-c]-1,2,4-triazolo[4,3-a]pyridin, gegebenenfalls in Form ihrer Racemate, Enantiomere oder Diastereomere und gegebenenfalls in Form ihrer pharmakologisch verträglichen Säureadditionssalze, Solvate und/oder Hydrate.

In besonders bevorzugten Arzneimittelkombinationen ist der PDE IV-Inhibitor **2b** ausgewählt aus der Gruppe bestehend aus Enprofyllin **(2b.1)**, Roflumilast **(2b.2),** Ariflo (Cilomilast) **(2b.3),** AWD-12-281 (GW-842470) **(2b.4),** N-(3,5-Dichloro-1-oxo-pyridin-4-yl)-4-difluoromethoxy-3-cyclopropylmethoxybenzamid **(2b.5),** T-440 **(2b.6),** T-2585 **(2b.7),** Arofyllin **(2b.8),** Cis[4-Cyano-4-(3-cyclopentyloxy-4-methoxyphenyl)cyclohexan-1-carbonsäure] **(2b.9),** 2-Carbomethoxy-4-cyano-4-(3-cyclopropylmethoxy-4-difluoromethoxyphenyl)cyclohexan-1-on **(2b.10),** Cis[4-cyano-4-(3-cyclopropylmethoxy-4-difluoromethoxyphenyl)cyclohexan-1-ol] (**2b.11**), PD-168787 **(2b.12),** Atizoram **(2b.13),** V-11294A **(2b.14),** CI-1018 **(2b.15),** CDC-801 **(2b.16),** D-22888 **(2b.17),** YM-58997 **(2b.18),** Z-15370 **(2b.19),** 9-Cyclopentyl-5,6-dihydro-7-ethyl-3-(2-thienyl)-9*H*-pyrazolo[3,4-c]-1,2,4-triazolo[4,3-a]pyridin **(2b.20)** und 9-Cyclopentyl-5,6-dihydro-7-ethyl-3-(*tert*-butyl)-9*H-*pyrazolo[3,4-c]-1,2,4-triazolo[4,3-a]pyridin **(2b.21),** gegebenenfalls in Form ihrer Racemate, Enantiomere oder Diastereomere und gegebenenfalls in Form ihrer pharmakologisch verträglichen Säureadditionssalze, Solvate und/oder Hydrate.

In besonders bevorzugten Arzneimittelkombinationen ist der PDE IV-Inhibitor **2b** ausgewählt aus der Gruppe bestehend aus Roflumilast **(2b.2),** Ariflo (Cilomilast) **(2b.3),** AWD-12-281 (GW-842470) **(2b.4),** Arofyllin **(2b.8),** 2-Carbomethoxy-4-cyano-4-(3-cyclopropylmethoxy4-difluoromethoxyphenyl)cyclohexan-1-on **(2b.10),** Cis[4-cyano-4-(3-cyclopropylmethoxy-4-difluoromethoxyphenyl)cyclohexan-1-ol] **(2b.11),** Atizoram **(2b.13),** Z-15370 **(2b.19),** 9-Cyclopentyl-5,6-dihydro-7-ethyl-3-(2-thienyl)-9*H*-pyrazolo[3,4-c]-1,2,4-triazolo[4,3-a]pyridin **(2b.20)** und 9-Cyclopentyl-5,6-dihydro-7-ethyl-3-(*tert*-butyl)-9*H-*pyrazolo[3,4-c]-1,2,4-triazolo[4,3-a]pyridin **(2b.21),** wobei Roflumilast **(2b.2),** Z-15370 **(2b.19)** und AWD-12-281 **(2b.4)** besondere Bedeutung zukommt, gegebenenfalls in Form ihrer Racemate, Enantiomere oder Diastereomere und gegebenenfalls in Form ihrer pharmakologisch verträglichen Säureadditionssalze, Solvate und/oder Hydrate.

Unter Säureadditionssalzen mit pharmakologisch verträglichen Säuren zu deren Bildung die Verbindungen 2b gegebenenfalls in der Lage sind werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Beispiele für erfindungsgemäß bevorzugte Arzneimittelkombinationen bevorzugter Verbindungen der Formel **1** mit den vorstehend genannten PDE IV-Inhibitoren **2b** sind Kombinationen enthaltend die Verbindungen **1.2** und **2b.1; 1.2** und **2b.2; 1.2** und **2b.3; 1.2** und **2b.4; 1.2** und **2b.5; 1.2** und **2b.6; 1.2** und **2b.7; 1.2** und **2b.8; 1.2** und **2b.9; 1.2** und **2b.10; 1.2** und **2b.11; 1.2** und **2b.12; 1.2** und **2b.13; 1.2** und **2b.14; 1.2** und **2b.15; 1.2** und **2b.16; 1.2** und **2b.17; 1.2** und **2b.18; 1.2** und **2b.19; 1.2** und **2b.20; 1.2** und **2b.21; 1.3** und **2b.1; 1.3** und **2b.2; 1.3** und **2b.3; 1.3** und **2b.4; 1.3** und **2b.5; 1.3** und **2b.6; 1.3** und **2b.7; 1.3** und **2b.8; 1.3** und **2b.9; 1.3** und **2b.10; 1.3** und **2b.11; 1.3** und **2b.12; 1.3** und **2b.13; 1.3** und **2b.14; 1.3** und **2b.15; 1.3** und **2b.16; 1.3** und **2b.17; 1.3** und **2b.18; 1.3** und **2b.19; 1.3** und **2b.20; 1.3** und **2b.21; 1.6** und **2b.1; 1.6** und **2b.2; 1.6** und **2b.3; 1.6** und **2b.4; 1.6** und **2b.5; 1.6** und **2b.6; 1.6** und **2b.7; 1.6** und **2b.8; 1.6** und **2b.9; 1.6** und **2b.10; 1.6** und **2b.11; 1.6** und **2b.12; 1.6** und **2b.13; 1.6** und **2b.14; 1.6** und 2b.15; **1.6** und **2b.16; 1.6** und **2b.17; 1.6** und **2b.18; 1.6** und **2b.19; 1.6** und **2b.20; 1.6** und **2b.21; 1.7** und **2b.1; 1.7** und **2b.2; 1.7** und **2b.3; 1.7** und **2b.4; 1.7** und **2b.5; 1.7** und **2b.6; 1.7** und **2b.7; 1.7** und **2b.8; 1.7** und **2b.9; 1.7** und **2b.10; 1.7** und **2b.11; 1.7** und **2b.12; 1.7** und **2b.13; 1.7** und **2b.14; 1.7** und **2b.15; 1.7** und **2b.16; 1.7** und **2b.17; 1.7** und **2b.18; 1.7** und **2b.19; 1.7** und **2b.20; 1.7** und **2b.21; 1.9** und **2b.1; 1.9** und **2b.2; 1.9** und **2b.3; 1.9** und **2b.4; 1.9** und **2b.5; 1.9** und **2b.6; 1.9** und **2b.7; 1.9** und **2b.8; 1.9** und **2b.9; 1.9** und **2b.10; 1.9** und **2b.11; 1.9** und **2b.12; 1.9** und **2b.13; 1.9** und **2b.14; 1.9** und **2b.15;** 1.9 und **2b.16**; **1.9** und **2b.17; 1.9** und **2b.18; 1.9** und **2b.19; 1.9** und **2b.20; 1.9** und **2b.21;** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate. Von den vorstehend genannten Kombinationen sind erfindungsgemäß ferner diejenigen bevorzugt, die als Verbindung **2b** eine der Verbindungen **2b.2, 2b.3, 2b.4, 2b.8, 2b.10, 2b.11, 2b.13, 2b.19, 2b.20** oder **2b.21,** enthalten, wobei jene Kombinationen, die eine der Verbindungen **2b.2, 2b.4** oder **2b.19** enthalten, erfindungsgemäß besonders bedeutsam sind.

Erfindungsgemäß ferner bevorzugte Arzneimittelkombinationen enthalten neben ein oder mehrerer, bevorzugt einer Verbindung der Formel **1** als weiteren Wirkstoff ein oder mehrere, bevorzugt ein Steroid **2c,** gegebenenfalls in Kombination mit pharmazeutisch verträglichen Hilfsstoffen.

In solchen Arzneimittelkombinationen ist das Steroid **2c** bevorzugt ausgewählt aus der Gruppe bestehend aus Prednisolon **(2c.1),** Prednison **(2c.2),** Butixocortpropionat **(2c.3),** RPR-106541 **(2c.4),** Flunisolid **(2c.5),** Beclomethason **(2c.6),** Triamcinolon **(2c.7),** Budesonid **(2c.8),** Fluticason **(2c.9),** Mometason **(2c.10),** Ciclesonid **(2c.11),** Rofleponid **(2c.12),** ST-126 **(2c.13),** Dexamethason **(2c.14),** 6α,9α-Difluoro-17α-[(2-furanylcarbonyl)oxy]-11β-hydroxy-16α-methyl-3-oxo-androsta-1,4-dien-17β-carbothionsäure (S)-fluoromethylester **(2c.15),** 6α,9α-Difluoro-11β-hydroxy-16α-methyl-3-oxo-17α-propionyloxy-androsta-1,4-dien-17β-carbothionsäure (S)-(2-oxo-tetrahydro-furan-3S-yl)ester **(2c.16)** und Etiprednol-dichloroacetat (BNP-166, **2c.17),** gegebenenfalls in Form ihrer Racemate, Enantiomere oder Diastereomere und gegebenenfalls in Form ihrer Salze und Derivate, ihrer Solvate und/oder Hydrate.

In besonders bevorzugten Arzneimittelkombinationen ist das Steroid **2c** ausgewählt aus der Gruppe bestehend aus Flunisolid **(2c.5),** Beclomethason **(2c.6),** Triamcinolon **(2c.7),** Budesonid **(2c.8),** Fluticason **(2c.9),** Mometason **(2c.10),** Ciclesonid **(2c.11),** Rofleponid **(2c.12),** ST-126 **(2c.13),** Dexamethason **(2c.14),** 6α,9α-Difluoro-17α-[(2-furanylcarbonyl)oxy]-11β-hydroxy-16α-methyl-3-oxo-androsta-1,4-dien-17β-carbothionsäure (S)-fluoromethylester **(2c.15),** 6α,9α-Difluoro-11β-hydroxy-16α-methyl-3-oxo-17α-propionyloxy-androsta-1,4-dien-17β-carbothionsäure (S)-(2-oxo-tetrahydro-furan-3S-yl)ester **(2c.16)** und Etiprednol-dichloroacetat **(2c.17),** gegebenenfalls in Form ihrer Racemate, Enantiomere oder Diastereomere und gegebenenfalls in Form ihrer Salze und Derivate, ihrer Solvate und/oder Hydrate.

In besonders bevorzugten Arzneimittelkombinationen ist das Steroid **2c** ausgewählt aus der Gruppe bestehend aus Budesonid **(2c.8),** Fluticason **(2c.9),** Mometason **(2c.10),** Ciclesonid **(2c.11),** 6α,9α-Difluoro-17α-[(2-furanylcarbonyl)oxy]-11β-hydroxy-16α-methyl-3-oxo-androsta-1,4-dien-17β-carbothionsäure (S)-fluoromethylester **(2c.15)** und Etiprednol-dichloroacetat **(2c.17),** gegebenenfalls in Form ihrer Racemate, Enantiomere oder Diastereomere und gegebenenfalls in Form ihrer Salze und Derivate, ihrer Solvate und/oder Hydrate.

Jede Bezugnahme auf Steroide **2c** schließt eine Bezugnahme auf deren gegebenenfalls existierende Salze oder Derivate, Hydrate oder Solvate mit ein. Beispiele möglicher Salze und Derivate der Steroide **2c** können sein: Alkalisalze, wie beispielsweise Natrium- oder Kaliumsalze, Sulfobenzoate, Phosphate, Isonicotinate, Acetate, Propionate, Dihydrogenphosphate, Palmitate, Pivalate oder auch Furoate.

Beispiele für erfindungsgemäß bevorzugte Arzneimittelkombinationen bevorzugter Verbindungen der Formel **1** mit den vorstehend genannten Steroiden **2c** sind Kombinationen enthaltend die Verbindungen **1.2** und **2c.1; 1.2** und **2c.2; 1.2** und **2c.3; 1.2** und **2c.4; 1.2** und **2c.5; 1.2** und **2c.6; 1.2** und **2c.7; 1.2** und **2c.8; 1.2** und **2c.9; 1.2** und **2c.10; 1.2** und **2c.11; 1.2** und **2c.12; 1.2** und **2c.13; 1.2** und **2c.14; 1.2** und **2c.15; 1.2** und **2c.16; 1.2** und **2c.17; 1.3** und **2c.1; 1.3** und **2c.2; 1.3** und **2c.3; 1.3** und **2c.4; 1.3** und **2c.5; 1.3** und **2c.6; 1.3** und **2c.7; 1.3** und **2c.8; 1.3** und **2c.9; 1.3** und **2c.10; 1.3** und **2c.11; 1.3** und **2c.12; 1.3** und **2c.13; 1.3** und **2c.14; 1.3** und **2c.15; 1.3** und **2c.16; 1.3** und **2c.17; 1.6** und **2c.1; 1.6** und **2c.2; 1.6** und **2c.3; 1.6** und **2c.4; 1.6** und **2c.5; 1.6** und **2c.6; 1.6** und **2c.7; 1.6** und **2c.8; 1.6** und **2c.9; 1.6** und **2c.10; 1.6** und **2c.11; 1.6** und **2c.12; 1.6** und **2c.13; 1.6** und **2c.14; 1.6** und **2c.15; 1.6** und **2c.16; 1.6** und **2c.17; 1.7** und **2c.1; 1.7** und **2c.2; 1.7** und **2c.3; 1.7** und **2c.4; 1.7** und **2c.5; 1.7** und **2c.6; 1.7** und **2c.7; 1.7** und **2c.8; 1.7** und **2c.9; 1.7** und **2c.10; 1.7** und **2c.11; 1.7** und **2c.12; 1.7** und **2c.13; 1.7** und **2c.14; 1.7** und **2c.15; 1.7** und **2c.16; 1.7** und **2c.17; 1.9** und **2c.1; 1.9** und **2c.2; 1.9** und **2c.3; 1.9** und **2c.4; 1.9** und **2c.5; 1.9** und **2c.6; 1.9** und **2c.7; 1.9** und **2c.8; 1.9** und **2c.9; 1.9** und **2c.10; 1.9** und **2c.11; 1.9** und **2c.12; 1.9** und **2c.13; 1.9** und **2c.14; 1.9** und **2c.15; 1.9** und **2c.16; 1.9** und **2c.17;** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate. Von den vorstehend genannten Kombinationen sind erfindungsgemäß ferner diejenigen bevorzugt, die als Verbindung **2c** eine der Verbindungen **2c.5, 2c.6, 2c.7, 2c.8, 2c.9, 2c.10, 2c.11, 2c.12, 2c.13, 2c.14, 2c.15, 2c.16** oder **2c.17** enthalten, wobei jene Kombinationen, die eine der Verbindungen **2c.8, 2c.9, 2c.10, 2c.11, 2c.15** oder **2c.17** enthalten, erfindungsgemäß besonders bedeutsam sind.

Erfindungsgemäß ferner bevorzugte Arzneimittelkombinationen enthalten neben ein oder mehrerer, bevorzugt einer Verbindung der Formel 1 als weiteren Wirkstoff ein oder mehrere, bevorzugt einen LTD4-Antagonist 2d, gegebenenfalls in Kombination mit pharmazeutisch verträglichen Hilfsstoffen.

In solchen Arzneimittelkombinationen ist der LTD4-Antagonist 2d bevorzugt ausgewählt aus der Gruppe bestehend aus Montelukast **(2d.1),** 1-(((R)-(3-(2-(6,7-Difluoro-2-quinolinyl)ethenyl)phenyl)-3-(2-(2- hydroxy-2-propyl)phenyl)thio)methylcyclopropanessigsäure **(2d.2),** 1-(((1(R)-3(3-(2-(2,3-Dichlorothieno[3,2-b]pyridin-5-yl)-(E)-ethenyl)phenyl)-3-(2-(1-hydroxy-1-methylethyl)phenyl)propyl)thio)methyl)cyclopropanessigsäure **(2d.3),** Pranlukast **(2d.4),** Zafirlukast **(2d.5),** [2-[[2-(4-tert-Butyl-2-thiazolyl)-5-benzofuranyl]oxymethyl]phenyl]essigsäure **(2d.6),** MCC-847 (ZD-3523) **(2d.7),** MN-001 **(2d.8),** MEN-91507 (LM-1507) **(2d.9),** VUF-5078 **(2d.10)**, VUF-K-8707 **(2d.11)** und L-733321 **(2d.12),** gegebenenfalls in Form ihrer Racemate, Enantiomere oder Diastereomere, gegebenenfalls in Form ihrer pharmakologisch verträglichen Säureadditionssalze sowie gegebenenfalls in Form ihrer Salze und Derivate, ihrer Solvate und/oder Hydrate.

In bevorzugten Arzneimittelkombinationen ist der LTD4-Antagonist **2d** ausgewählt aus der Gruppe bestehend aus Montelukast **(2d.1),** Pranlukast **(2d.4),** Zafirlukast **(2d.5),** MCC-847 (ZD-3523) **(2d.7),** MN-001 **(2d.8),** MEN-91507 (LM-1507) **(2d.9),** VUF-5078 **(2d.10),** VUF-K-8707 **(2d.11)** und L-733321 **(2d.12),** gegebenenfalls in Form ihrer Racemate, Enantiomere oder Diastereomere, gegebenenfalls in Form ihrer pharmakologisch verträglichen Säureadditionssalze sowie gegebenenfalls in Form ihrer Salze und Derivate, ihrer Solvate und/oder Hydrate.

In besonders bevorzugten Arzneimittelkombinationen ist der LTD4-Antagonist **2d** ausgewählt aus der Gruppe bestehend aus Montelukast **(2d.1),** Pranlukast **(2d.4),** Zafirlukast **(2d.5),** MCC-847 (ZD-3523) **(2d.7),** MN-001 **(2d.8)** und MEN-91507 (LM-1507) **(2d.9),** wobei Montelukast **(2d.1),** Pranlukast **(2d.4)** und Zafirlukast **(2d.5)** besonders bevorzugt sind, gegebenenfalls in Form ihrer Racemate, Enantiomere oder Diastereomere, gegebenenfalls in Form ihrer pharmakologisch verträglichen Säureadditionssalze sowie gegebenenfalls in Form ihrer Salze und Derivate, ihrer Solvate und/oder Hydrate.

Unter Säureadditionssalzen mit pharmakologisch verträglichen Säuren zu deren Bildung die Verbindungen **2d** gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Unter Salzen oder Derivaten zu deren Bildung die Verbindungen **2d** gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Beispiele für erfindungsgemäße bevorzugte Arzneimittelkombinationen bevorzugter Verbindungen der Formel **1** mit den vorstehend genannten LTD4-Antagonisten **2d** sind Kombinationen enthaltend die Verbindungen **1.2** und **2d.1; 1.2** und **2d.2; 1.2** und **2d.3; 1.2** und **2d.4; 1.2** und **2d.5; 1.2** und **2d.6; 1.2** und **2d.7; 1.2** und **2d.8; 1.2** und **2d.9; 1.2** und **2d.10; 1.2** und **2d.11; 1.2** und **2d.12; 1.3** und **2d.1; 1.3** und **2d.2; 1.3** und **2d.3; 1.3** und **2d.4; 1.3** und **2d.5; 1.3** und **2d.6; 1.3** und **2d.7; 1.3** und **2d.8; 1.3** und **2d.9; 1.3** und **2d.10; 1.3** und **2d.11; 1.3** und **2d.12; 1.6** und **2d.1; 1.6** und **2d.2; 1.6** und **2d.3; 1.6** und **2d.4; 1.6** und **2d.5; 1.6** und **2d.6; 1.6** und **2d.7; 1.6** und **2d.8; 1.6** und **2d.9; 1.6** und **2d.10; 1.6** und **2d.11; 1.6** und **2d.12; 1.7** und **2d.1; 1.7** und **2d.2; 1.7** und **2d.3; 1.7** und **2d.4; 1.7** und **2d.5; 1.7** und **2d.6; 1.7** und **2d.7; 1.7** und **2d.8; 1.7** und **2d.9; 1.7** und **2d.10; 1.7** und **2d.11; 1.7** und **2d.12; 1.9** und **2d.1; 1.9** und **2d.2; 1.9** und **2d.3; 1.9** und **2d.4; 1.9** und **2d.5; 1.9** und **2d.6; 1.9** und **2d.7; 1.9** und **2d.8; 1.9** und **2d.9; 1.9** und **2d.10; 1.9** und **2d.11; 1.9** und **2d.12;** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate. Von den vorstehend genannten Kombinationen sind erfindungsgemäß ferner diejenigen bevorzugt, die als Verbindung **2d** eine der Verbindungen **2d.1, 2d.4, 2d.5, 2d.7, 2d.8, 2d.9, 2d.10, 2d.11** oder **2d.12,** enthalten, wobei jene Kombinationen, die eine der Verbindungen **2d.1, 2d.4, 2d.5, 2d.7, 2d.8** oder **2d.9** enthalten, erfindungsgemäß besonders bedeutsam sind, wobei jenen Kombinationen, die eine der Verbindungen **2d.1, 2d.4** oder **2d.5** enthalten, herausragende Bedeutung zukommt.

Erfindungsgemäß ferner bevorzugte Arzneimittelkombinationen enthalten neben ein oder mehrerer, bevorzugt einer Verbindung der Formel **1** als weiteren Wirkstoff ein oder mehrere, bevorzugt einen EGFR-Hemmer **2e,** gegebenenfalls in Kombination mit pharmazeutisch verträglichen Hilfsstoffen.

In solchen Arzneimittelkombinationen ist der EGFR-Hemmer **2e** beispielsweise ausgewählt aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(3-Chlor-4-fluorphenyl)amino]-6-{[4-(N,N-diethylamino)-1-oxo-2-buten-1-yl]amino}-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopropylmethoxy-chinazolin, 4-[(*R*)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluor-phenyl)amino]-6-{[4-((*R*)-6-methyl-2-oxo-morpholin-4-yl)-1-oxo-2-buten-1-yl]amino}-7-cyclopropylmethoxy-chinazolin, 4-[(3-Chlor-4-fluorphenyl)amino]-6-{[4-((*R*)-6-methyl-2-oxo-morpholin-4-yl)-1-oxo-2-buten-1-yl]amino}-7-[(*S*)-(tetrahydrofuran-3-yl)oxy]-chinazolin, 4-[(3-Chlor-4-fluor-phenyl)amino]-6-{[4-((*R*)-2-methoxymethyl-6-oxo-morpholin-4-yl)-1-oxo-2-buten-1-yl]amino}-7-cyclopropylmethoxy-chinazolin, 4-[(3-Chlor-4-fluor-phenyl)amino]-6-[2-((*S*)-6-methyl-2-oxo-morpholin-4-yl)-ethoxy]-7-methoxy-chinazolin, 4-[(3-Chlor-4-fluorphenyl)amino]-6-({4-[N-(2-methoxyethyl)-N-methyl-amino]-1-oxo-2-buten-1-yl}amino)-7-cyclopropylmethoxy-chinazolin, 4-[(3-Chlor-4-fluorphenyl)amino]-6-([4-(N,N-dimethylamino)-1-oxo-2-buten-1-yl]amino}-7-cyclopentyloxy-chinazolin, 4-[(*R*)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin, 4-[(*R*)-(1-Phenylethyl)amino]-6-({4-[N-(2-methoxy-ethyl)-N-ethyl-amino]-1-oxo-2-buten-1-yl}amino)-7-cyclopropylmethoxy-chinazolin, 4-[(*R*)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin, 4-[(*R*)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-((*R*)-tetrahydrofuran-3-yloxy)-chinazolin, 4-[(3-Chlor-4-fluorphenyl)amino]-6-{[4-(N,N-dimethylamino)-1-oxo-2-buten-1-yl]amino}-7-((*S*)-tetrahydrofuran-3-yloxy)-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin, 4-[(3-Chlor-4-fluorphenyl)amino]-6-{[4-(N,N-dimethylamino)-1-oxo-2-buten-1-yl]amino}-7-[(*R*)-(tetrahydrofuran-2-yl)methoxy]-chinazolin, 4-[(3-Chlor-4-fluorphenyl)amino]-6-{[4-(N,N-dimethylamino)-1-oxo-2-buten-1-yl]amino}-7-[(*S*)-(tetrahydrofuran-2-yl)methoxy]-chinazolin, 4-[(3-Ethinyl-phenyl)amino]-6,7-bis-(2-methoxy-ethoxy)-chinazolin, 4-[(3-Chlor-4-fluorphenyl)amino]-7-[3-(morpholin-4-yl)-propyloxy]-6-[(vinylcarbonyl)amino)-chinazolin, 4-[(*R*)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-{[3-Chlor-4-(3-fluor-benzyloxy)-phenyl]amino}-6-(5-{[(2-methansulfonyl-ethyl)amino]methyl}-furan-2-yl)chinazolin, 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-[2-(2,2-dimethyl-6-oxo-morpholin-4-yl)-ethoxy]-7-methoxy-chinazolin, 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin, 4-[(3-Chlor-4-fluorphenyl)amino]-7-[2-(2,2-dimethyl-6-oxo-morpholin-4-yl)-ethoxy]-6-[(S)-(tetrahydrofuran-2-yl)methoxy]-chinazolin, 4-[(3-Chlor-4-fluor-phenyl)amino]-6-{2-[4-(2-oxo-morpholin-4-yl-) piperidin-1-yl]-ethoxy}-7-methoxy-chinazolin, 4-[(3-Chlor-4-fluor-phenyl)amino]-6-[1-(tert-butyloxycarbonyl)-piperidin-4-yloxy]-7-methoxy-chinazolin, 4-[(3-Chlor-4-fluorphenyl)amino]-6-(trans-4-amino-cyclohexan-1-yloxy)-7-methoxy-chinazolin, 4-[(3-Chlor,-4-fluor-phenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluor-phenyl)amino]-6-(1-methyl-piperidin-4-yloxy)-7-methoxy-chinazolin, 4-[(3-Chlor-4-fluor-phenyl)amino]-6-{1-[(morpholin-4-yl)carbonyl]-piperidin-4-yl-oxy}-7-methoxy-chinazolin, 4-[(3-Chlor-4-fluor-phenyl)amino]-6-{1-[(methoxymethyl)carbonyl]-piperidin-4-yloxy}-7-methoxy-chinazolin, 4-[(3-Chlor-4-fluorphenyl)amino]-6-(piperidin-3-yloxy)-7-methoxy-chinazolin, 4-[(3-Chlor-4-fluorphenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6-((S)-tetrahydrofuran-3-yloxy)-7-hydroxy-chinazolin, 4-[(3-Chlor-4-fluor-phenyl)amino]-6-(tetrahydropyran-4-yloxy)-7-(2-methoxy-ethoxy)-chinazolin, 4-[(3-Chlor-4-fluor-phenyl)amino]-6-{trans-4-[(dimethylamino)sulfonylamino]-cyclohexan-1-yloxy}-7-methoxy-chinazolin, 4-[(3-Chlor-4-fluor-phenyl)amino]-6-{trans-4-[(morpholin-4-yl)carbonylamino]-cyclohexan-1-yloxy}-7-methoxy-chinazolin, 4-[(3-Chlor-4-fluorphenyl)amino]-6-{trans-4-[(morpholin-4-yl)sulfonylamino]-cyclohexan-1-yloxy}-7-methoxy-chinazolin, 4-[(3-Chlor-4-fluor-phenyl)amino]-6-(tetrahydropyran-4-yloxy)-7-(2-acetylamino-ethoxy)-chinazolin, 4-[(3-Chlor-4-fluor-phenyl)amino]-6-(tetrahydropyran-4-yloxy)-7-(2-methansulfonylamino-ethoxy)-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluorphenyl)amino]-6-(1-aminocarbonylmethyl-piperidin-4-yloxy)-7-methoxy-chinazolin, 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin, 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-(cis-4-{N-[(morpholin-4-yl)sulfonyl]-N-methyl-amino}-cyclohexan-1-yloxy)-7-methoxy- chinazolin, 4-[(3-Chlor-4-fluor-phenyl)amino]-6-(trans-4-ethansulfonylamino-cyclohexan-1-yloxy)-7-methoxy-chinazolin, 4-[(3-Chlor-4-fluorphenyl)amino]-6-(1-methansulfonyl-piperidin-4-yloxy)-7-ethoxy-chinazolin, 4-[(3-Chlor-4-fluor-phenyl)amino]-6-(1-methansulfonyl-piperidin-4-yloxy)-7-(2-methoxy-ethoxy)-chinazolin, 4-[(3-Chlor-4-fluor-phenyl)amino]-6-[1-(2-methoxy-acetyl)-piperidin-4-yloxy]-7-(2-methoxy-ethoxy)-chinazolin, 4-[(3-Chlor-4-fluor-phenyl)amino]-6-(cis-4-acetylamino-cyclohexan-1-yloxy)-7-methoxy-chinazolin, 4-[(3-Ethinyl-phenyl)amino]-6-[1-(tert.-butyloxycarbonyl)-piperidin-4-yloxy]-7-methoxy-chinazolin, 4-[(3-Ethinyl-phenyl)amino]-6-(tetrahydropyran-4-yloxy]-7-methoxy-chinazolin, 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin, 4-[(3-Chlor-4-fluor-phenyl)amino]-6-{cis-4-[(morpholin-4-yl)carbonylamino]-cyclohexan-1-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Chlor-4-fluor-phenyl)amino]-6-{1-[(morpholin-4-yl)carbonyl]-piperidin-4-yloxy}-7-(2-methoxy-ethoxy)-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluor-phenyl)amino]-6-(1-methyl-piperidin-4-yloxy)-7(2-methoxy-ethoxy)-chinazolin, 4-[(3-Chlor-4-fluor-phenyl)amino]-6-(1-isopropyloxycarbonyl-piperidin-4-yloxy)-7-methoxy-chinazolin, 4-[(3-Chlor-4-fluor-phenyl)amino]-6-(cis-4-methylamino-cyclohexan-1-yloxy)-7-methoxy-chinazolin, 4-[(3-Chlor-4-fluor-phenyl)amino]-6-{cis-4-[N-(2-methoxy-acetyl)-N-methyl-amino]-cyclohexan-1-yloxy}-7-methoxy-chinazolin, 4-[(3-Ethinyl-phenyl)amino]-6-(piperidin-4-yloxy)-7-methoxy-chinazolin, 4-[(3-Ethinyl-phenyl)amino]-6-[1-(2-methoxyacetyl)-piperidin-4-yloxy]-7-methoxy-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluorphenyl)amino]-6-{1-[(cis-2,6-dimethyl-morpholin-4-yl)carbonyl]-piperidin-4-yloxy}-7-methoxy-chinazolin, 4-[(3-Chlor-4-fluor-phenyl)amino]-6-{1-[(2-methyl-morpholin-4-yl)carbonyl]-piperidin-4-yloxy}-7-methoxy-chinazolin, 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-4-fluorphenyl)amino]-6-(1-ethyl-piperidin-4-yloxy)-7-methoxy-chinazolin, 4-[(3-Chlor-4-fluorphenyl)amino]-6-{1-[(2-methoxyethyl)carbonyl]-piperidin-4-yloxy}-7-methoxy-chinazolin, 4-[(3-Chlor-4-fluor-phenyl)amino]-6-{1-[(3-methoxypropyl-amino)-carbonyl]-piperidin-4-yloxy}-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin , 4-[(3-Chlor-4-fluorphenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin , 4-[(3-Chlor-4-fluorphenyl)amino]-6-(trans-4-dimethylamino-cyclohexan-1-yloxy)-7-methoxy-chinazolin , 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluor-phenyl)amino]-6-(1-methansulfonyl-piperidin-4-yloxy)-7-methoxy-chinazolin, 4-[(3-Chlor-4-fluor-phenyl)amino]-6-(1-cyano-piperidin-4-yloxy)-7-methoxy-chinazolin, Cetuximab, Trastuzumab, ABX-EGF und Mab ICR-62, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

In solchen Arzneimittelkombinationen ist der EGFR-Hemmer **2e** bevorzugt ausgewählt aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(3-Chlor-4-fluorphenyl)amino]-6-{[4-(N,N-diethylamino)-1-oxo-2-buten-1-yl]amino}-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin, 4-[(3-Chlor-4-fluorphenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxyethyl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopentyloxy-chinazolin, 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin, 4-[(R)-(1-Phenylethyl)amino]-6-({4-[N-(2-methoxy-ethyl)-N-ethyl-amino]-1-oxo-2-buten-1-yl}amino)-7-cyclopropylmethoxy-chinazolin, 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin, 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin, 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin , 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin, 4-[(3-Ethinyl-phenyl)amino]-6,7-bis-(2-methoxy-ethoxy)-chinazolin, 4-[(3-Chlor-4-fluorphenyl)amino]-7-[3-(morpholin-4-yl)-propyloxy]-6-[(vinylcarbonyl)amino]-chinazolin, 4-[(R)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-{[3-Chlor-4-(3-fluor-benzyloxy)-phenyl]amino}-6-(5-{[(2-methansulfonyl-ethyl)amino]methyl}-furan-2-yl)chinazolin , 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-[2-(2,2-dimethyl-6-oxo-morpholin-4-yl)-ethoxy]-7-methoxy-chinazolin, 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin, 4-[(3-Chlor-4-fluorphenyl)amino]-7-[2-(2,2-dimethyl-6-oxo-morpholin-4-yl)-ethoxy]-6-[(S)-(tetrahydrofuran-2-yl)methoxy]-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluor-phenyl)amino]-6-[1-(tert.-butyloxycarbonyl)-piperidin-4-yloxy]-7-methoxy-chinazolin, 4-[(3-Chlor-4-fluorphenyl)amino]-6-(trans-4-amino-cyclohexan-1-yloxy)-7-methoxy-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluor-phenyl)amino]-6-(1-methyl-piperidin-4-yloxy)-7-methoxy-chinazolin, 4-[(3-Chlor-4-fluor-phenyl)amino]-6-{1-[(morpholin-4-yl)carbonyl]-piperidin-4-yl-oxy}-7-methoxy-chinazolin, 4-[(3-Chlor-4-fluor-phenyl)amino]-6-{1-[(methoxymethyl)carbonyl]-piperidin-4-yloxy}-7-methoxy-chinazolin, 4-[(3-Chlor-4-fluorphenyl)amino]-6-(piperidin-3-yloxy)-7-methoxy-chinazolin, 4-[(3-Chlor-4-fluorphenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6-((S)-tetrahydrofuran-3-yloxy)-7-hydroxy-chinazolin, 4-[(3-Chlor-4-fluor-phenyl)amino]-6-(tetrahydropyran-4-yloxy)-7-(2-methoxy-ethoxy)-chinazolin, 4-[(3-Chlor-4-fluor-phenyl)amino]-6-{trans-4-[(dimethylamino)sulfonylamino]-cyclohexan-1-yloxy}-7-methoxy-chinazolin, 4-[(3-Chlor-4-fluor-phenyl)amino]-6-{trans-4-[(morpholin-4-yl)carbonylamino]-cyclohexan-1-yloxy}-7-methoxy-chinazolin, 4-[(3-Chlor-4-fluorphenyl)amino]-6-{trans-4-[(morpholin-4-yl)sulfonylamino]-cyclohexan-1-yloxy}-7-methoxy-chinazolin, 4-[(3-Chlor-4-fluor-phenyl)amino]-6-(tetrahydropyran-4-yloxy)-7-(2-acetylamino-ethoxy)-chinazolin, 4-[(3-Chlor-4-fluor-phenyl)amino]-6-(tetrahydropyran-4-yloxy)-7-(2-methansulfonylamino-ethoxy)-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluorphenyl)amino]-6-(1-aminocarbonylmethyl-piperidin-4-yloxy)-7-methoxy-chinazolin, 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin, 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin, 4-[(3-Chlor-4-fluorphenyl)amino]-6-(trans-4-ethansulfonylamino-cyclohexan-1-yloxy)-7-methoxy-chinazolin, 4-[(3-Chlor-4-fluor-phenyl)amino]-6-(1-methansulfonyl-piperidin-4-yloxy)-7-ethoxy-chinazolin, 4-[(3-Chlor-4-fluor-phenyl)amino]-6-(1-methansulfonyl-piperidin-4-yloxy)-7-(2-methoxy-ethoxy)-chinazolin , 4-[(3-Chlor-4-fluor-phenyl)amino]-6-[1-(2-methoxy-acetyl)-piperidin-4-yloxy]-7-(2-methoxy-ethoxy)-chinazolin , 4-[(3-Chlor-4-fluor-phenyl)amino]-6-(cis-4-acetylamino-cyclohexan-1-yloxy)-7-methoxy-chinazolin, 4-[(3-Ethinyl-phenyl)amino]-6-[1-(tert.-butyloxycarbonyl)-piperidin-4-yloxy]-7-methoxy-chinazolin, 4-[(3-Ethinyl-phenyl)amino]-6-(tetrahydropyran-4-yloxy]-7-methoxy-chinazolin, 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluor-phenyl)amino]-6-(cis-4-{N-[(4-methylpiperazin-1-yl)carbonyl]-N-methyl-amino}-cyclohexan-1-yloxy)-7-methoxy-chinazolin, 4-[(3-Chlor-4-fluor-phenyl)amino]-6-{cis-4-[(morpholin-4-yl)carbonylamino]-cyclohexan-1-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Chlor-4-fluor-phenyl)amino]-6-{1-[(morpholin-4-yl)carbonyl]-piperidin-4-yloxy}-7-(2-methoxy-ethoxy)-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluor-phenyl)amino]-6-(1-methyl-piperidin-4-yloxy)-7(2-methoxy-ethoxy)-chinazolin, 4-[(3-Chlor-4-fluor-phenyl)amino]-6-(1-isopropyloxycarbonyl-piperidin-4-yloxy)-7-methoxy-chinazolin, 4-[(3-Chlor-4-fluor-phenyl)amino]-6-(cis-4-methylamino-cyclohexan-1-yloxy)-7-methoxy-chinazolin, 4-[(3-Chlor-4-fluor-phenyl)amino]-6-{cis-4-[N-(2-methoxy-acetyl)-N-methyl-amino]-cyclohexan-1-yloxy}-7-methoxy-chinazolin, 4-[(3-Ethinyl-phenyl)amino]-6-(piperidin-4-yloxy)-7-methoxy-chinazolin, 4-[(3-Ethinyl-phenyl)amino]-6-[1-(2-methoxyacetyl)-piperidin-4-yloxy]-7-methoxy-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluorphenyl)amino]-6-{1-[(cis-2,6-dimethyl-morpholin-4-yl)carbonyl]-piperidin-4-yloxy}-7-methoxy-chinazolin, 4-[(3-Chlor-4-fluor-phenyl)amino]-6-{1-[(2-methyl-morpholin-4-yl)carbonyl]-piperidin-4-yloxy}-7-methoxy-chinazolin, 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-4-fluorphenyl)amino]-6-(1-ethyl-piperidin-4-yloxy)-7-methoxy-chinazolin, 4-[(3-Chlor-4-fluorphenyl)amino]-6-{1-[(2-methoxyethyl)carbonyl]-piperidin-4-yloxy}-7-methoxy-chinazolin, 4-[(3-Chlor-4-fluor-phenyl)amino]-6-{1-[(3-methoxypropyl-amino)-carbonyl]-piperidin-4-yloxy}-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin, 4-[(3-Chlor-4-fluorphenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin, 4-[(3-Chlor-4-fluorphenyl)amino]-6-(trans-4-dimethylamino-cyclohexan-1-yloxy)-7-methoxy-chinazolin, 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluor-phenyl)amino]-6-(1-methansulfonyl-piperidin-4-yloxy)-7-methoxy-chinazolin, 4-[(3-Chlor-4-fluor-phenyl)amino]-6-(1-cyano-piperidin-4-yloxy)-7-methoxy-chinazolin, und Cetuximab, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Besonders bevorzugt gelangen im Rahmen der erfindungsgemäßen Arzneimittelkombinationen die EGFR-Hemmer **2e** zur Anwendung, die ausgewählt sind aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin, 4-[(3-Ethinyl-phenyl)amino]-6,7-bis-(2-methoxy-ethoxy)-chinazolin, 4-[(R)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluor-phenyl)amino]-6-(trans-4-amino-cyclohexan-1-yloxy)-7-methoxy-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluorphenyl)amino]-6-{1-[(morpholin-4-yl)carbonyl]-piperidin-4-yloxy}-7-methoxy-chinazolin, 4-[(3-Chlor-4-fluor-phenyl)amino]-6-(piperidin-3-yloxy)-7-methoxy-chinazolin, 4-[(3-Chlor-4-fluor-phenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6-{trans-4-[(morpholin-4-yl)carbonylamino]-cyclohexan-1-yloxy}-7-methoxy-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluor-phenyl)amino]-6-(cis-4-{N-[(morpholin-4-yl)carbonyl]-N-methyl-amino}cyclohexan-1-yloxy)-7-methoxy-chinazolin, 4-[(3-Chlor-4-fluor-phenyl)amino]-6-(trans-4-ethansulfonylamino-cyclohexan-1-yloxy)-7-methoxy-chinazolin, 4-[(3-Chlor-4-fluor-phenyl)amino]-6-(1-methansulfonyl-piperidin-4-yloxy)-7-(2-methoxy-ethoxy)-chinazolin, 4-[(3-Chlor-4-fluor-phenyl)amino]-6-[1-(2-methoxy-acetyl)-piperidin-4-yloxy]-7-(2-methoxy-ethoxy)-chinazolin, 4-[(3-Ethinyl-phenyl)amino]-6-(tetrahydropyran-4-yloxy]-7-methoxy-chinazolin, 4-[(3-Chlor-4-fluorphenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluor-phenyl)amino]-6-{cis-4-[(morpholin-4-yl)carbonylamino]-cyclohexan-1-yloxy}-7-methoxy-chinazolin, 4-[(3-Chlor-4-fluorphenyl)amino]-6-{1-[2-(2-oxopyrrolidin-1-yl)ethyl]-piperidin-4-yloxy}-7-methoxy-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluor-phenyl)amino]-6-(1-methyl-piperidin-4-yloxy)-7(2-methoxy-ethoxy)-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-4-fluorphenyl)amino]-6-(1-ethyl-piperidin-4-yloxy)-7-methoxy-chinazolin, 4-[(3-Chlor-4-fluorphenyl)amino]-6-[cis-4-(N-methansulfonyl-N-methyl-amino)-cyclohexan-1-yloxy]-7-methoxy-chinazolin , 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluorphenyl)amino]-6-[trans-4-(N-methansulfonyl-N-methyl-amino)-cyclohexan-1-yloxy]-7-methoxy-chinazolin , 4-[(3-Chlor-4-fluor-phenyl)amino]-6-(trans-4-dimethylamino-cyclohexan-1-yloxy)-7-methoxy-chinazolin, 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluor-phenyl)amino]-6-(1-methansulfonyl-piperidin-4-yloxy)-7-methoxy-chinazolin, 4-[(3-Chlor-4-fluor-phenyl)amino]-6-(1-cyano-piperidin-4-yloxy)-7-methoxy-chinazolin, und 4-[(3-Chlor-4-fluor-phenyl)amino]-6-{1-[(2-methoxyethyl)carbonyl]-piperidin-4-yloxy}-7-methoxy-chinazolin, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Erfindungsgemäß besonders bevorzugte Arzneimittelkombinationen enthalten als EGFR-Hemmer **2e** diejenigen Verbindungen, die ausgewählt sind aus der Gruppe bestehend aus
- 4-[(3-Chlor-4-fluorphenyl)amino]-6-{[4-(morpholin-4-yl)-1-oxo-2-buten-1-yl]amino}-7-cyclopropylmethoxy-chinazolin **(2e.1)**,
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin **(2e.2),**
- 4-[(3-Chlor-4-fluor-phenyl)amino]-6-[2-((S)-6-methyl-2-oxo-morpholin-4-yl)-ethoxy]-7-methoxy-chinazolin **(2e.3),**
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin **(2e.4),**
- 4-[(3-Ethinyl-phenyl)amino]-6,7-bis-(2-methoxy-ethoxy)-chinazolin **(2e.5),**
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin **(2e.6),**
- 4-[(3-Ethinyl-phenyl)amino]-6-{[4-(5,5-dimethyl-2-oxo-morpholin-4-yl)-1-oxo-2-buten-1-yl]amino}-chinazolin **(2e.7),**
- 4-[(3-Chlor-4-fluor-phenyl)amino]-6-(trans-4-methansulfonylamino-cyclohexan-1-yloxy)-7-methoxy-chinazolin **(2e.8),**
- 4-[(3-Chlor-4-fluor-phenyl)amino]-6-(tetrahydropyran-3-yloxy)-7-methoxy-chinazolin **(2e.9),**
- 4-[(3-Chlor-4-fluor-phenyl)amino]-6-{1-[(morpholin-4-yl)carbonyl]-piperidin-4-yloxy}-7-methoxy-chinazolin **(2e.10),**
- 4-[(3-Chlor-4-fluor-phenyl)amino]-6-{1-[2-(2-oxopyrrolidin-1-yl)ethyl]-piperidin-4-yloxy}-7-methoxy-chinazolin **(2e.11),**
- 4-[(3-Ethinyl-phenyl)amino]-6-(1-acetyl-piperidin-4-yloxy)-7-methoxy-chinazolin **(2e.12),**
- 4-[(3-Ethinyl-phenyl)amino]-6-(1-methyl-piperidin-4-yloxy)-7-methoxy-chinazolin **(2e.13),**
- 4-[(3-Ethinyl-phenyl)amino]-6-(1-methansulfonyl-piperidin-4-yloxy)-7-methoxy-chinazolin **(2e.14),**
- 4-[(3-Ethinyl-phenyl)amino]-6-{1-[(morpholin-4-yl)carbonyl]-piperidin-4-yloxy}-7-methoxy-chinazolin **(2e.15),**
- 4-[(3-Chlor-4-fluor-phenyl)amino]-6-{1-[(2-methoxyethyl)carbonyl]-piperidin-4-yloxy}-7-methoxy-chinazolin **(2e.16),**
- 4-[(3-Chlor-4-fluor-phenyl)amino]-6-[cis-4-(N-methansulfonyl-N-methyl-amino)-cyclohexan-1-yloxy]-7-methoxy-chinazolin **(2e.17),**
- 4-[(3-Chlor-4-fluor-phenyl)amino]-6-[cis-4-(N-acetyl-N-methyl-amino)-cyclohexan-1-yloxy]-7-methoxy-chinazolin **(2e.18),**
- 4-[(3-Chlor-4-fluor-phenyl)amino]-6-(trans-4-methylamino-cyclohexan-1-yloxy)-7-methoxy-chinazolin **(2e.19),**
- 4-[(3-Chlor-4-fluor-phenyl)amino]-6-[trans-4-(N-methansulfonyl-N-methyl-amino)-cyclohexan-1-yloxy]-7-methoxy-chinazolin **(2e.20),**
- 4-[(3-Chlor-4-fluor-phenyl)amino]-6-(trans-4-dimethylamino-cyclohexan-1-yloxy)-7-methoxy-chinazolin **(2e.21),**
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin **(2e.22),**
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin **(2e.23),**
- 4-[(3-Chlor-4-fluor-phenyl)amino]-6-(1-methansulfonyl-piperidin-4-yloxy)-7-methoxy-chinazolin **(2e.24)** und
- 4-[(3-Chlor-4-fluor-phenyl)amino]-6-(1-cyano-piperidin-4-yloxy)-7-methoxy-chinazolin **(2e.25),**
gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Unter Säureadditionssalzen mit pharmakologisch verträglichen Säuren zu deren Bildung die Verbindungen **2e** gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Beispiele für erfindungsgemäße bevorzugte Arzneimittelkombinationen bevorzugter Verbindungen der Formel 1 mit den vorstehend genannten EGFR-Hemmern **2e** sind Kombinationen enthaltend die Verbindungen **1.2** und **2e.1; 1.2** und **2e.2; 1.2** und **2e.3; 1.2** und **2e.4; 1.2** und **2e.5; 1.2** und **2e.6; 1.2** und **2e.7; 1.2** und **2e.8; 1.2** und **2e.9; 1.2** und **2e.10; 1.2** und **2e.11; 1.2** und **2e.12; 1.2** und **2e.13; 1.2** und **2e.14; 1.2** und **2e.15; 1.2** und **2e.16; 1.2** und **2e.17; 1.2** und **2e.18; 1.2** und **2e.19; 1.2** und **2e.20; 1.2** und **2e.21; 1.2** und **2e.22; 1.2** und **2e.23; 1.2** und **2e.24; 1.2** und **2e.25; 1.3** und **2e.1; 1.3** und **2e.2; 1.3** und **2e.3; 1.3** und **2e.4; 1.3** und **2e.5; 1.3** und **2e.6; 1.3** und **2e.7; 1.3** und **2e.8; 1.3** und **2e.9; 1.3** und **2e.10; 1.3** und **2e.11; 1.3** und **2e.12; 1.3** und **2e.13; 1.3** und **2e.14; 1.3** und **2e.15; 1.3** und **2e.16; 1.3** und **2e.17; 1.3** und **2e.18; 1.3** und **2e.19; 1.3** und **2e.20; 1.3** und **2e.21; 1.3** und **2e.22; 1.3** und **2e.23; 1.3** und **2e.24; 1.3** und **2e.25; 1.6** und **2e.1; 1.6** und **2e.2; 1.6** und **2e.3; 1.6** und **2e.4; 1.6** und **2e.5; 1.6** und **2e.6; 1.6** und **2e.7; 1.6** und **2e.8; 1.6** und **2e.9; 1.6** und **2e.10; 1.6** und **2e.11; 1.6** und **2e.12; 1.6** und **2e.13; 1.6** und **2e.14; 1.6** und **2e.15; 1.6** und **2e.16; 1.6** und **2e.17; 1.6** und **2e.18; 1.6** und **2e.19; 1.6** und **2e.20; 1.6** und **2e.21; 1.6** und **2e.22; 1.6** und **2e.23; 1.6** und **2e.24; 1.6** und **2e.25; 1.7** und **2e.1; 1.7** und **2e.2; 1.7** und **2e.3; 1.7** und **2e.4; 1.7** und **2e.5; 1.7** und **2e.6; 1.7** und **2e.7; 1.7** und **2e.8; 1.7** und 2e.9; **1.7** und **2e.10; 1.7** und **2e.11; 1.7** und **2e.12; 1.7** und **2e.13; 1.7** und **2e.14; 1.7** und **2e.15**; 1.7 und **2e.16; 1.7** und **2e.17; 1.7** und **2e.18; 1.7** und **2e.19; 1.7** und **2e.20; 1.7** und **2e.21; 1.7** und **2e.22; 1.7** und **2e.23; 1.7** und **2e.24; 1.7** und **2e.25; 1.9** und **2e.1; 1.9** und **2e.2; 1.9** und **2e.3; 1.9** und **2e.4; 1.9** und **2e.5; 1.9** und **2e.6;** 1.9 und **2e.7; 1.9** und **2e.8; 1.9** und **2e.9; 1.9** und **2e.10; 1.9** und **2e.11; 1.9** und **2e.12; 1.9** und **2e.13; 1.9** und **2e.14; 1.9** und **2e.15; 1.9** und **2e.16; 1.9** und **2e.17; 1.9** und **2e.18; 1.9** und **2e.19; 1.9** und **2e.20; 1.9** und **2e.21; 1.9** und **2e.22; 1.9** und **2e.23; 1.9** und **2e.24; 1.9** und **2e.25;** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate. Von den vorstehend genannten Kombinationen sind erfindungsgemäß ferner diejenigen bevorzugt, die als Verbindung **2e** eine der Verbindungen **2e.1, 2e.2, 2e.3, 2e.4, 2e.10, 2e.11, 2e.14, 2e.16, 2e.17, 2e.18, 2e.19, 2e.20, 2e.21, 2e.22, 2e.23, 2e.24** oder **2e.25** enthalten, wobei jene Kombinationen, die eine der Verbindungen **2e.2, 2e.3** oder **2e.4** enthalten, erfindungsgemäß besonders bedeutsam sind.

Die erfindungsgemäßen Arzneimittelkombinationen aus Verbindungen der Formel **1** mit wenigstens einem weiteren Wirkstoff **2** sind nicht beschränkt auf binäre Wirkstoffkombinationen. Die vorstehend teils exemplarisch genannten Kombinationen, die neben einer Verbindung der Formel 1 einen weiteren Wirkstoff **2** enthalten, können ferner einen dritten oder vierten, vorzugsweise einen dritten Wirkstoff enthalten, der ebenfalls aus der vorstehend genannten Gruppe von Anticholinergika **(2a),** PDEIV-Inhibitoren **(2b),** Steroide **(2c),** LTD4-Antagonisten **(2d)** und EGFR-Hemmern **(2e)** ausgewählt ist.

Besonders bevorzugte Kombinationen, die neben einer Verbindung der Formel 1 2 weitere Wirkstoffe enthalten sind ausgewählt aus den nachstehend aufgeführten Wirkstoffkombinationen. Es sind dies Arzneimittelkombinationen, die beispielsweise enthalten können:
- eine Verbindung der Formel **1,** ein Anticholinergikum **(2a),** ein PDEIV-Inhibitor **(2b);**
- eine Verbindung der Formel **1,** ein Anticholinergikum **(2a),** ein Steroid **(2c);**
- eine Verbindung der Formel **1,** ein Anticholinergikum **(2a),** ein LTD4-Antagonist **(2d);**
- eine Verbindung der Formel **1,** ein Anticholinergikum **(2a),** ein EGFR-Hemmer **(2e);**
- eine Verbindung der Formel **1,** ein PDEIV-Inhibitor **(2b),** ein Steroid **(2c);**
- eine Verbindung der Formel **1,** ein PDEIV-Inhibitor **(2b),** ein LTD4-Antagonist **(2d);**
- eine Verbindung der Formel **1,** ein PDEIV-Inhibitor **(2b),** ein EGFR-Hemmer **(2e);**
- eine Verbindung der Formel **1,** ein Steroid **(2c),** ein LTD4-Antagonist **(2d);**
- eine Verbindung der Formel 1, ein Steroid **(2c),** ein EGFR-Hemmer **(2e);**
- eine Verbindung der Formel 1, ein LTD4-Antagonist **(2d),** ein EGFR-Hemmer **(2e).**

Erfindungsgemäß von herausragender Bedeutung sind all diejenigen im Rahmen der vorliegenden Erfindung offenbarten Arzneimittelkombinationen, die die Verbindungen der Formel 1 in Form ihrer R-Enantiomere enthalten.

### VERWENDETE BEGRIFFE UND DEFINITIONEN

Im Rahmen der vorliegenden Erfindung wird unter Arzneimittelkombination der Komponenten **1** und **2** die gemeinsame Applikation beider Wirkstoffe in einer einzigen Darreichungsform bzw. Formulierung oder die getrennte Applikationen der beiden Wirkstoffe in getrennten Darreichungsformen verstanden. Werden die Wirkstoffe **1** und **2** in getrennten Darreichungsformen appliziert, kann diese getrennte Applikation gleichzeitig oder zeitlich abgestuft, das heißt nacheinander erfolgen. Ein Aspekt der vorliegenden Erfindung betrifft vorstehend genannte Arzneimittelkombinationen, welche neben therapeutisch wirksamen Mengen von **1** und **2** einen pharmazeutisch verträglichen Trägerstoff enthalten. Ein Aspekt der vorliegenden Erfindung betrifft vorstehend genannte Arzneimittel, welche neben therapeutisch wirksamen Mengen von 1 und **2** keinen pharmazeutisch verträglichen Trägerstoff enthalten.

Unter dem Begriff "C₁₋₆-Alkyl" (auch soweit sie Bestandteil anderer Reste sind) werden verzweigte und unverzweigte Alkylgruppen mit 1 bis 6 Kohlenstoffatomen verstanden und unter dem Begriff "C₁₋₄-Alkyl" werden verzweigte und unverzweigte Alkylgruppen mit 1 bis 4 Kohlenstoffatomen verstanden. Bevorzugt sind Alkylgruppen mit 1 bis 4 Kohlenstoffatomen. Beispielsweise werden hierfür genannt: Methyl, Ethyl, *n*-Propyl, *iso*-Propyl, *n*-Butyl, *iso*-Butyl, *sec*-Butyl, *tert*-Butyl, *n*-Pentyl, *iso*-Pentyl, *neo*-Pentyl oder Hexyl. Gegebenenfalls werden für vorstehend genannte Gruppen auch die Abkürzungen Me, Et, *n*-Pr, *i*-Pr, *n*-Bu, *i*-Bu, *t*-Bu, etc. verwendet. Sofern nicht anders beschrieben, umfassen die Definitionen Propyl, Butyl, Pentyl und Hexyl alle denkbaren isomeren Formen der jeweiligen Reste. So umfasst beispielsweise Propyl *n*-Propyl und *iso*-Propyl, Butyl umfasst *iso*-Butyl, *sec*-Butyl und *tert*-Butyl etc.

Unter dem Begriff "C₂₋₆-Alkenyl" (auch soweit sie Bestandteil anderer Reste sind) werden verzweigte und unverzweigte Alkenylgruppen mit 2 bis 6 Kohlenstoffatomen und unter dem Begriff "C₂₋₄-Alkenyl" verzweigte und unverzweigte Alkenylgruppen mit 2 bis 4 Kohlenstoffatomen verstanden, soweit sie mindestens eine Doppelbindung aufweisen. Bevorzugt sind Alkenylgruppen mit 2 bis 4 Kohlenstoffatomen. Beispielsweise werden hierfür genannt: Ethenyl oder Vinyl, Propenyl, Butenyl, Pentenyl, oder Hexenyl. Sofern nicht anders beschrieben, umfassen die Definitionen Propenyl, Butenyl, Pentenyl und Hexenyl alle denkbaren isomeren Formen der jeweiligen Reste. So umfasst beispielsweise Propenyl 1-Propenyl und 2-Propenyl, Butenyl umfasst 1-, 2- und 3-Butenyl, 1-Methyl-1-propenyl, 1-Methyl-2-propenyl etc.

Unter dem Begriff "C₂₋₆-Alkinyl" (auch soweit sie Bestandteil anderer Reste sind) werden verzweigte und unverzweigte Alkinylgruppen mit 2 bis 6 Kohlenstoffatomen und unter dem Begriff "C₂₋₄-Alkinyl" verzweigte und unverzweigte Alkinylgruppen mit 2 bis 4 Kohlenstoffatomen verstanden, soweit sie mindestens eine Dreifachbindung aufweisen. Bevorzugt sind Alkinylgruppen mit 2 bis 4 Kohlenstoffatomen. Beispielsweise werden hierfür genannt: Ethinyl, Propinyl, Butinyl, Pentinyl, oder Hexinyl. Sofern nicht anders beschrieben, umfassen die Definitionen Propinyl, Butinyl, Pentinyl und Hexinyl alle denkbaren isomeren Formen der jeweiligen Reste. So umfasst beispielsweise Propinyl 1-Propinyl und 2-Propinyl, Butinyl umfasst 1-, 2- und 3-Butinyl, 1-Methyl-1-propinyl, 1-Methyl-2-propinyl etc.

Unter dem Begriff "C₅₋₆-Cycloalkyl" (auch soweit sie Bestandteil anderer Reste sind) werden cyclische Alkylgruppen mit 5 oder 6 Kohlenstoffatomen verstanden. Beispielsweise werden hierfür genannt: Cyclopentyl oder Cyclohexyl. Soweit nicht anders beschrieben, können die cyclischen Alkylgruppen substituiert sein mit einem oder mehreren Resten ausgewählt aus der Gruppe bestehend aus Methyl, Ethyl, *iso*-Propyl, tert-Butyl, Hydroxy, Fluor, Chlor, Brom und Jod.

Unter dem Begriff "C₁₋₆-Haloalkyl" (auch soweit sie Bestandteil anderer Reste sind) werden verzweigte und unverzweigte Alkylgruppen mit 1 bis 6 Kohlenstoffatomen verstanden, die mit einem oder mehreren Halogenatomen substituiert sind. Unter dem Begriff "C₁₋₄-Alkyl" werden verzweigte und unverzweigte Alkylgruppen mit 1 bis 4 Kohlenstoffatomen verstanden, die mit einem oder mehreren Halogenatomen substituiert sind. Bevorzugt sind Alkylgruppen mit 1 bis 4 Kohlenstoffatomen. Bevorzugte Halogenatome sind Fluor, Chlor, besonders bevorzugt Fluor. Beispielsweise werden hierfür genannt: CF₃, CHF₂, CH₂F, CH₂CF₃.

Halogen steht im Rahmen der vorliegenden Erfindung für Fluor, Chlor, Brom oder Jod. Sofern nicht gegenteilig angegeben, gelten Fluor, Chlor und Brom als bevorzugte Halogene.

Verbindungen der allgemeinen Formel **1** können Säuregruppen besitzen, hauptsächlich Carboxylgruppen, und/oder basische Gruppen wie z.B. Aminofunktionen. Verbindungen der allgemeinen Formel **1** können deshalb als innere Salze, als Salze mit pharmazeutisch verwendbaren anorganischen Säuren wie Salzsäure, Schwefelsäure, Phosphorsäure, Sulfonsäure oder organischen Säuren (wie beispielsweise Maleinsäure, Fumarsäure, Zitronensäure, Weinsäure oder Essigsäure) oder als Salze mit pharmazeutisch verwendbaren Basen wie Alkali oder Erdalkalimetallhydroxiden oder Carbonaten, Zink oder Ammoniumhydroxiden oder organischen Aminen wie z.B. Diethylamin, Triethylamin, Triethanolamin u.a. vorliegen.

Unter Säureadditionssalzen mit pharmakologisch unbedenklichen Säuren der Verbindungen **1** werden dabei beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden. Von den vorstehend genannten Säureadditionssalzen sind die Salze der Chlorwasserstoffsäure, der Methansulfonsäure, der Benzoesäure und der Essigsäure erfindungsgemäß besonders bevorzugt.

### INDIKATIONEN

Die vorliegende Erfindung betrifft ferner die Verwendung therapeutisch wirksamer Mengen der Wirkstoffe **1** zur Herstellung eines einen oder mehrere, bevorzugt einen

Wirkstoff **2** enthaltenden Arzneimittels zur Behandlung von entzündlichen und obstruktiven Atemwegserkrankungen, zur Hemmung verfrüht einsetzender Wehen in der Geburtshilfe (Tokolyse), zur Wiederherstellung des Sinusrhythmus im Herzen bei atrioventrikulärem Block, zur Behebung bradykaler Herzrhythmusstörungen (Antiarrhythmikum), zur Therapie des Kreislaufschocks (Gefäßerweiterung und Steigerung des Herzzeitvolumens) sowie zur Behandlung von Juckreiz und Entzündungen der Haut.

Ein bevorzugter Aspekt der vorliegenden Erfindung betrifft die Verwendung therapeutisch wirksamer Mengen der Wirkstoffe **1** zur Herstellung eines einen oder mehrere, bevorzugt einen Wirkstoff **2** enthaltenden Arzneimittels zur Behandlung von Atemwegserkrankungen, die ausgewählt sind aus der Gruppe bestehend aus Obstruktive Lungenerkrankungen unterschiedlicher Genese, Lungenemphyseme unterschiedlicher Genese, Restriktive Lungenerkrankungen, Interstitielle Lungenerkrankungen, Zystische Fibrose, Bronchitiden unterschiedlicher Genese, Bronchiektasen, ARDS (adult respiratory distress syndrom) und alle Formen des Lungenödems.

Bevorzugt ist vorstehend genannte Verwendung der erfindungsgemäßen Arzneimittelkombinationen zur Herstellung eines Arzneimittels zur Behandlung von obstruktiven Lungenerkrankungen, die ausgewählt sind aus der Gruppe bestehend aus Asthma Bronchiale, pädiatrisches Asthma, schweres Asthma, akuter Asthma-Anfall, chronische Bronchitis und COPD (chronisch obstruktive pulmonale Erkrankung), wobei die Verwendung zur Herstellung eines Arzneimittels zur Behandlung von Asthma Bronchiale und COPD erfindungsgemäß besonders bevorzugt ist.

Bevorzugt ist ferner die vorstehend genannte Verwendung der erfindungsgemäßen Arzneimittelkombinationen zur Herstellung eines Arzneimittels zur Behandlung von Lungenemphysemen, die ihren Ursprung haben in COPD oder α1-Proteinase-Inhibitor-Mangel.

Bevorzugt ist ferner die vorstehend genannte Verwendung der erfindungsgemäßen Arzneimittelkombinationen zur Herstellung eines Arzneimittels zur Behandlung von Restriktiven Lungenerkrankungen, die ausgewählt sind aus der Gruppe bestehend aus Allergische Alveolitis, durch berufliche Noxen ausgelöste restriktive Lungenerkrankungen wie Asbestose oder Silikose und Restriktion aufgrund von Lungentumoren, wie beispielsweise Lymphangiosis carcinomatosa, bronchoalveoläres Karzinom und Lymphome.

Bevorzugt ist ferner die vorstehend genannte Verwendung der erfindungsgemäßen Arzneimittelkombinationen zur Herstellung eines Arzneimittels zur Behandlung von Interstitiellen Lungenerkrankungen, die ausgewählt sind aus der Gruppe bestehend aus infektiös bedingte Pneumonien, wie beispielsweise aufgrund einer Infektion mit Viren, Bakterien, Pilzen, Protozoen, Helminthen oder anderen Erregern, Pneumonitis aufgrund unterschiedlicher Genese, wie beispielsweise Aspiration und Linksherzinsuffizienz, Strahlen-induzierte Pneumonitis oder Fibrose, Kollagenosen, wie beispielsweise Lupus erythematodes, systemische Sklerodermie oder Sarkoidose, Granulomatosen, wie beispielsweise Morbus Boeck, idiopathische interstitielle Pneumonie oder idiopathische pulmonäre Fibrose (IPF).

Bevorzugt ist ferner die vorstehend genannte Verwendung der erfindungsgemäßen Arzneimittelkombinationen zur Herstellung eines Arzneimittels zur Behandlung von Zystischer Fibrose bzw. Mukoviszidose.

Bevorzugt ist ferner die vorstehend genannte Verwendung der erfindungsgemäßen Arzneimittelkombinationen zur Herstellung eines Arzneimittels zur Behandlung von Bronchitiden, wie beispielsweise Bronchitis aufgrund bakterieller oder viraler Infektion, Allergische Bronchitis und Toxische Bronchitis.

Bevorzugt ist ferner die vorstehend genannte Verwendung der erfindungsgemäßen Arzneimittelkombinationen zur Herstellung eines Arzneimittels zur Behandlung von Bronchiektasen.

Bevorzugt ist ferner die vorstehend genannte Verwendung der erfindungsgemäßen Arzneimittelkombinationen zur Herstellung eines Arzneimittels zur Behandlung von ARDS (adult respiratory distress syndrom).

Bevorzugt ist ferner die vorstehend genannte Verwendung der erfindungsgemäßen Arzneimittelkombinationen zur Herstellung eines Arzneimittels zur Behandlung von Lungenödemen, beispielsweise toxischer Lungenödeme nach Aspiration oder Inhalation von toxischen Substanzen und Fremdstoffen.

Besonders bevorzugt betrifft die vorstehend genannte Verwendung der erfindungsgemäßen Arzneimittelkombinationen zur Herstellung eines Arzneimittels zur Behandlung von Asthma oder COPD. Von besonderer Bedeutung ist ferner die vorstehend genannte Verwendung der erfindungsgemäßen Arzneimittelkombinationen zur Herstellung eines Arzneimittels zur einmal täglichen Behandlung von entzündlichen und obstruktiven Atemwegserkrankungen, besonders bevorzugt zur einmal täglichen Behandlung von Asthma oder COPD.

### FORMULIERUNG

Die vorliegende Erfindung betrifft ferner die Verwendung therapeutisch wirksamer Mengen eines Wirkstoffs der Formel **1** in Kombination mit therapeutisch wirksamen Mengen eines Wirkstoffs **2** zur Herstellung eines Arzneimittels zur Behandlung einer der vorstehend genannten Erkrankungen.

Die vorliegende Erfindung betrifft ferner ein Verfahren zur Behandlung einer der vorstehend genannten Erkrankungen, welches dadurch gekennzeichnet ist, dass therapeutisch wirksame Mengen eines Wirkstoffs der Formel 1 in Kombination mit therapeutisch wirksamen Mengen eines Wirkstoffs 2 appliziert werden.

Im Rahmen der erfindungsgemäßen Arzneimittelkombinationen können pro Einmalgabe beispielsweise 0,1 - 1000µg einer Verbindung der Formel **1** appliziert werden. Bevorzugt werden pro Einmalgabe 1 - 500 µg, besonders bevorzugt 3 - 100 µg der Verbindung der Formel **1** appliziert, wobei ein Dosierungsbereich von 5 - 75µg, bevorzugt von 7 - 50 µg erfindungsgemäß bevorzugt ist. Besonders bevorzugt werden die erfindungsgemäßen Arzneimittel in einer solchen Menge appliziert, dass pro Einmalgabe 9 - 40 µg, besonders bevorzugt 11 - 30µg, ferner bevorzugt 12 - 25 µg der Verbindung der Formel **1** verabreicht werden. Beispielsweise und ohne den Gegenstand der vorliegenden Erfindung darauf zu beschränken, können pro Einmalgabe 5µg, 7,5µg, 10µg, 12,5µg, 15µg, 17,5µg, 20µg, 22,5µg, 25µg, 27,5µg, 30µg, 32,5µg, 35µg, 37,5µg, 40µg, 42,5µg, 45µg, 47,5µg, 50µg, 52,5µg, 55µg, 57,5µg, 60µg, 62,5µg, 65µg, 67,5µg, 70µg, 72,5µg oder 75µg einer Verbindung der Formel **1** appliziert werden.

Die vorstehend genannten Dosierungen beziehen sich auf die Verbindungen der Formel **1** in Form ihrer freien Base. Werden die Verbindungen der Formel **1** in Form ihrer pharmazeutisch verträglichen Säureadditionssalze appliziert, sind aus vorstehend genannten Dosisbereichen die entsprechenden Dosisbereiche für die Säureadditionssalze unter Berücksichtigung des Molekulargewichts der verwendeten Säuren für den Fachmann leicht berechenbar. Besonders bevorzugt, werden die Verbindungen der Formel **1** bei o.g. Dosisbereichen in Form der enantiomerenreinen Verbindungen, besonders bevorzugt in Form ihrer R-Enantiomere appliziert.

Werden die Verbindungen der Formel **1** in Kombination mit einem Anticholinergikum **2a** appliziert, variiert die Menge des eingesetzten Anticholinergikums stark in Abhängigkeit von der Wahl des Wirkstoffs.

Ohne den Umfang der Erfindung darauf zu beschränken können im Falle des Tiotropiums **2a.1'** solche Mengen an Anticholinergikum **(2a.1')** appliziert werden, dass pro Einmalgabe 0,1 - 80µg, bevorzugt 0,5 - 60 µg, besonders bevorzugt etwa 1 - 50µg **2a.1'** enthalten sind. Beispielsweise und ohne den Gegenstand der vorliegenden Erfindung darauf zu beschränken, können pro Einmalgabe 2,5µg, 5µg, 10µg, 18µg, 20µg, 36µg oder 40µg **2a.1'** appliziert werden. Die jeweils entsprechende Menge des zum Einsatz gelangenden Salzes **2a.1** oder gegebenenfalls zum Einsatz gelangender Hydrate oder Solvate sind für den Fachmann je nach Wahl des Anions leicht berechenbar. Wird als erfindungsgemäß bevorzugtes Tiotropiumsalz **2a.1** beispielsweise Tiotropiumbromid verwendet, entsprechen die vorstehend beispielhaft genannten pro Einmalgabe applizierten Wirkstoffmengen von **2a.1' den** nachfolgenden pro Einmalgabe applizierten Mengen an **2a.1:** 3µg, 6µg, 12µg, 21,7µg, 24,1µg, 43,3µg und 48,1µg **2a.1.** Im Falle des Tiotropiums 2a.1' erfolgt die Applikation der vorstehend genannten Dosierungen vorzugsweise ein- oder zweimal täglich, wobei die einmal tägliche Applikation erfindungsgemäß besonders bevorzugt ist.

Ohne den Umfang der Erfindung darauf zu beschränken, können im Falle des Kations **2a.2'** solche Mengen an Anticholinergikum **(2a.2')** appliziert werden, dass pro Einmalgabe 1 - 500µg, bevorzugt 5 - 300 µg, besonders bevorzugt 15-200 µg **2a.2'** enthalten sind. Beispielsweise und ohne den Gegenstand der vorliegenden Erfindung darauf zu beschränken, können pro Einmalgabe 15µg, 20µg, 25µg, 30µg, 35µg, 40µg, 45µg, 50µg, 55µg, 60µg, 65µg, 70µg, 75µg, 80µg, 85g, 90µg, 95µg, 100µg, 105µg, 110µg, 115µg, 120µg, 125µg, 130µg, 135µg, 140µg, 145µg, 150µg, 155µg, 160µg, 165µg, 170µg, 175µg, 180µg, 185µg, 190µg, 195µg oder 200µg **2a.2'** appliziert werden. Die jeweils entsprechende Menge des zum Einsatz gelangenden Salzes **2a.2** oder gegebenenfalls zum Einsatz gelangender Hydrate oder Solvate sind für den Fachmann je nach Wahl des Anions leicht berechenbar. Im Falle des Oxitropiums **2a.2'** erfolgt die Applikation der vorstehend genannten Dosierungen vorzugsweise ein- bis viermal täglich, wobei die zweibis dreimal tägliche Applikation erfindungsgemäß besonders bevorzugt ist.

Ohne den Umfang der Erfindung darauf zu beschränken, können im Falle des Kations **2a.3'** solche Mengen an Anticholinergikum **(2a.3')** appliziert werden, dass pro Einmalgabe 1 - 500µg, bevorzugt 5 - 300 µg, besonders bevorzugt 15-200 µg **2a.3'** enthalten sind. Beispielsweise und ohne den Gegenstand der vorliegenden Erfindung darauf zu beschränken, können pro Einmalgabe 15µg, 20µg, 25µg, 30µg, 35µg, 40µg, 45µg, 50µg, 55µg, 60µg, 65µg, 70µg, 75µg, 80µg, 85µg, 90µg, 95µg, 100µg, 105µg, 110µg, 115µg, 120µg, 125µg, 130µg, 135µg, 140µg, 145µg, 150µg, 155µg, 160µg, 165µg, 170µg, 175µg, 180µg, 185µg, 190µg, 195µg oder 200µg **2a.3'** appliziert werden. Die jeweils entsprechende Menge des zum Einsatz gelangenden Salzes **2a.3** oder gegebenenfalls zum Einsatz gelangender Hydrate oder Solvate sind für den Fachmann je nach Wahl des Anions leicht berechenbar. Im Falle des Flutropiums **2a.3'** erfolgt die Applikation der vorstehend genannten Dosierungen vorzugsweise ein- bis viermal täglich, wobei die zweibis dreimal tägliche Applikation erfindungsgemäß besonders bevorzugt ist.

Ohne den Umfang der Erfindung darauf zu beschränken, können im Falle des Kations **2a.4'** solche Mengen an Anticholinergikum **(2a.4')** appliziert werden, dass pro Einmalgabe 1 - 500µg, bevorzugt 5 - 300 µg, besonders bevorzugt 20-200 µg **2a.4'** enthalten sind. Beispielsweise und ohne den Gegenstand der vorliegenden Erfindung darauf zu beschränken, können pro Einmalgabe 20µg, 25µg, 30µg, 35µg, 40µg, 45µg, 50µg, 55µg, 60µg, 65µg, 70µg, 75µg, 80µg, 85µg, 90µg, 95µg, 100µg, 105µg, 110µg, 115µg, 120µg, 125µg, 130µg, 135µg, 140µg, 145µg, 150µg, 155µg, 160µg, 165µg, 170µg, 175µg, 180µg, 185µg, 190µg, 195µg oder 200µg **2a.4'** appliziert werden. Die jeweils entsprechende Menge des zum Einsatz gelangenden Salzes **2a.4** oder gegebenenfalls zum Einsatz gelangender Hydrate oder Solvate sind für den Fachmann je nach Wahl des Anions leicht berechenbar. Im Falle des Ipratropiums **2a.4'** erfolgt die Applikation der vorstehend genannten Dosierungen vorzugsweise ein- bis viermal täglich, wobei die zweibis dreimal, besonders bevorzugt die dreimal tägliche Applikation erfindungsgemäß besonders bevorzugt ist.

Ohne den Umfang der Erfindung darauf zu beschränken, können im Falle des Kations **2a.5'** solche Mengen an Anticholinergikum **(2a.5')** appliziert werden, dass pro Einmalgabe 1 - 500µg, bevorzugt 5 - 300 µg, besonders bevorzugt 15-200 µg enthalten sind. Beispielsweise und ohne den Gegenstand der vorliegenden Erfindung darauf zu beschränken, können pro Einmalgabe 15µg, 20µg, 25µg, 30µg, 35µg, 40µg, 45µg, 50µg, 55µg, 60µg, 65µg, 70µg, 75µg, 80µg, 85µg, 90µg, 95µg, 100µg, 105µg, 110µg, 115µg, 120µg, 125µg, 130µg, 135µg, 140µg, 145µg, 150µg, 155µg, 160µg, 165µg, 170µg, 175µg, 180µg, 185µg, 190µg, 195µg oder 200µg **2a.5'** appliziert werden. Die jeweils entsprechende Menge des zum Einsatz gelangenden Salzes **2a.5** oder gegebenenfalls zum Einsatz gelangender Hydrate oder Solvate sind für den Fachmann je nach Wahl des Anions leicht berechenbar. Im Falle des Glycopyrroniums **2a.5'** erfolgt die Applikation der vorstehend genannten Dosierungen vorzugsweise ein- bis viermal täglich, wobei die zweibis dreimal tägliche Applikation erfindungsgemäß besonders bevorzugt ist.

Ohne den Umfang der Erfindung darauf zu beschränken, können im Falle des Kations **2a.6'** solche Mengen an Anticholinergikum **(2a.6')** appliziert werden, dass pro Einmalgabe 1000 - 6500µg, bevorzugt 2000 - 6000µg, besonders bevorzugt 3000 - 5500µg, besonders bevorzugt 4000 - 5000µg **2a.6'** enthalten sind. Beispielsweise und ohne den Gegenstand der vorliegenden Erfindung darauf zu beschränken, können pro Einmalgabe 3500µg, 3750µg, 4000µg, 4250µg, 4500µg, 4750µg, oder 5000µg **2a.6'** appliziert werden. Die jeweils entsprechende Menge des zum Einsatz gelangenden Salzes **2a.6** oder gegebenenfalls zum Einsatz gelangender Hydrate oder Solvate sind für den Fachmann je nach Wahl des Anions leicht berechenbar. Im Falle des Trospiums **2a.6'** erfolgt die Applikation der vorstehend genannten Dosierungen vorzugsweise ein- bis viermal täglich, wobei die zwei- bis dreimal tägliche Applikation erfindungsgemäß besonders bevorzugt ist.

Ohne den Umfang der Erfindung darauf zu beschränken, können im Falle des Kations **2a.7'** solche Mengen an Anticholinergikum **(2a.7')** appliziert werden, dass pro Einmalgabe 50 - 1000µg, bevorzugt 100 - 800µg, besonders bevorzugt 200 - 700µg, besonders bevorzugt 300 - 600µg **2a.7'** enthalten sind. Beispielsweise und ohne den Gegenstand der vorliegenden Erfindung darauf zu beschränken, können pro Einmalgabe 300µg, 350µg, 400µg, 450µg, 500µg, 550µg, oder 600µg **2a.7'** appliziert werden. Die jeweils entsprechende Menge des zum Einsatz gelangenden Salzes **2a.7** oder gegebenenfalls zum Einsatz gelangender Hydrate oder Solvate sind für den Fachmann je nach Wahl des Anions leicht berechenbar. Im Falle des Kations **2a.7'** erfolgt die Applikation der vorstehend genannten Dosierungen vorzugsweise ein- bis dreimal täglich, wobei die einbis zweimal, besonders bevorzugt die einmal tägliche Applikation erfindungsgemäß besonders bevorzugt ist.

Ohne den Umfang der Erfindung darauf zu beschränken, können im Falle der Kationen **2a.9'** und **2a.10'** solche Mengen an Anticholinergikum **(2a.9' oder 2a.10')** appliziert werden, dass pro Einmalgabe 1 - 500µg, bevorzugt 5 - 300 µg, besonders bevorzugt 15-200 µg **2a.9'** oder **2a.10'** enthalten sind. Beispielsweise und ohne den Gegenstand der vorliegenden Erfindung darauf zu beschränken, können pro Einmalgabe 15µg, 20µg, 25µg, 30µg, 35µg, 40µg, 45µg, 50µg, 55µg, 60µg, 65µg, 70µg, 75µg, 80µg, 85µg, 90µg, 95µg, 100µg, 105µg, 110µg, 115µg, 120µg, 125µg, 130µg, 135µg, 140µg, 145µg, 150µg, 155µg, 160µg, 165µg, 170µg, 175µg, 180µg, 185µg, 190µg, 195µg oder 200µg **2a.9' oder 2a.10'** appliziert werden. Die jeweils entsprechende Menge des zum Einsatz gelangenden Salzes **2a.9'** oder **2a.10'**oder gegebenenfalls zum Einsatz gelangender Hydrate oder Solvate sind für den Fachmann je nach Wahl des Anions leicht berechenbar. Im Falle der Kationen **2a.9'** oder **2a.10'**erfolgt die Applikation der vorstehend genannten Dosierungen vorzugsweise ein- bis dreimal täglich, wobei die ein- bis zweimal, besonders bevorzugt die einmal tägliche Applikation erfindungsgemäß besonders bevorzugt ist.

Ohne den Umfang der Erfindung darauf zu beschränken, können im Falle der Kationen **2a.11'** bis **2a.13'** solche Mengen an Anticholinergikum **(2a.11', 2a.12'** oder **2a.13')** appliziert werden, dass pro Einmalgabe 1 - 500µg, bevorzugt 5 - 300 µg, besonders bevorzugt 10-200 µg **2a.11', 2a.12'** oder **2a.13'** enthalten sind. Beispielsweise und ohne den Gegenstand der vorliegenden Erfindung darauf zu beschränken, können pro Einmalgabe 10µg, 15µg, 20µg, 25µg, 30µg, 35µg, 40µg, 45µg, 50µg, 55µg, 60µg, 65µg, 70µg, 75µg, 80µg, 85µg, 90µg, 95µg, 100µg, 105µg, 110µg, 115µg, 120µg, 125µg, 130µg, 135µg, 140µg, 145µg, 150µg, 155µg, 160µg, 165µg, 170µg, 175µg, 180µg, 185µg, 190µg, 195µg oder 200µg **2a.11', 2a.12'** oder **2a.13' appliziert** werden. Die jeweils entsprechende Menge des zum Einsatz gelangenden Salzes **2a.11, 2a.12** oder **2a.13** oder gegebenenfalls zum Einsatz gelangender Hydrate oder Solvate sind für den Fachmann je nach Wahl des Anions leicht berechenbar.

Im Falle der Kationen **2a.11, 2a.12** oder **2a.13** erfolgt die Applikation der vorstehend genannten Dosierungen vorzugsweise ein- bis dreimal täglich, wobei die ein- bis zweimal, besonders bevorzugt die einmal tägliche Applikation erfindungsgemäß besonders bevorzugt ist.

Werden die Verbindungen der Formel **1** in Kombination mit einem PDE IV-Inhibitor **2b** appliziert, werden vorzugsweise etwa 1 - 10000 µg **2b** pro Einmalgabe appliziert. Bevorzugt werden solche Mengen an **2b** appliziert, dass pro Einmalgabe 10 - 5000µg, bevorzugt 50 - 2500 µg, besonders bevorzugt 100-1000 µg **2b** enthalten sind. Beispielsweise und ohne den Gegenstand der vorliegenden Erfindung darauf zu beschränken, können pro Einmalgabe 100µg, 115µg, 120µg, 125µg, 130µg, 135µg, 140µg, 145µg, 150µg, 155µg, 160µg, 165µg, 170µg, 175µg, 180µg, 185µg, 190µg, 195µg, 200µg, 205µg, 210µg, 215µg, 220µg, 225µg, 230µg, 235µg, 240µg, 245µg, 250µg, 255µg, 260µg 265µg, 270µg, 275µg, 280µg, 285µg, 290µg, 295µg, 300µg, 305µg, 310µg, 315µg, 320µg, 325µg, 330µg, 335µg, 340µg, 345µg, 350µg, 355µg, 360µg, 365µg, 370µg, 375µg, 380µg, 385µg, 390µg, 395µg, 400µg, 405µg, 410µg, 415µg, 420µg, 425µg, 430µg, 435µg, 440µg, 445µg, 450µg, 455µg, 460µg, 465µg, 470µg, 475µg, 480µg, 485µg, 490µg, 495µg, 500µg, 505µg, 510µg, 515µg, 520µg, 525µg, 530µg, 535µg, 540µg, 545µg, 550µg, 555µg, 560µg, 565µg, 570µg, 575µg, 580µg, 585µg, 590µg, 595µg, 600µg, 605µg, 610µg, 615µg, 620µg, 625µg, 630µg, 635µg, 640µg, 645µg, 650µg, 655µg, 660µg, 665µg, 670µg, 675µg, 680µg, 685µg, 690µg, 695µg, 700µg, 705µg, 710µg, 715µg, 720µg, 725µg, 730µg, 735µg, 740µg, 745µg, 750µg, 755µg, 760µg, 765µg, 770µg, 775µg, 780µg, 785µg, 790µg, 795µg, 800µg, 805µg, 810µg, 815µg, 820µg, 825µg, 830µg, 835µg, 840µg, 845µg, 850µg, 855µg, 860µg, 865µg, 870µg, 875µg, 880µg, 885µg, 890µg, 895µg, 900µg, 905µg, 910µg, 915µg, 920µg, 925µg, 930µg, 935µg, 940µg, 945µg, 950µg, 955µg, 960µg, 965µg, 970µg, 975µg, 980µg, 985µg, 990µg, 995µg oder 1000µg 2b appliziert werden. Bei gegebenenfalls zum Einsatz gelangenden Säureadditionssalzen von 2b ist die entsprechende Menge des zum Einsatz gelangenden Salzes für den Fachmann je nach Wahl der Säure aus vorstehenden Werten leicht berechenbar.

Werden die Verbindungen der Formel **1** in Kombination mit einem Steroid **2c** appliziert, werden vorzugsweise etwa 1 - 10000 µg **2c** pro Einmalgabe appliziert. Bevorzugt werden solche Mengen an **2c** appliziert, dass pro Einmalgabe 5 - 5000µg, bevorzugt 5 - 2500 µg, besonders bevorzugt 10-1000 µg **2c** enthalten sind. Beispielsweise und ohne den Gegenstand der vorliegenden Erfindung darauf zu beschränken, können pro Einmalgabe 10µg, 15µg, 20µg, 25µg, 30µg, 35µg, 40µg, 45µg, 50µg, 55µg, 60µg, 65µg, 70µg, 75µg, 80µg, 85µg, 90µg, 95µg, 100µg, 115µg, 120µg, 125µg,130µg,135µg,140µg,145µg, 150µg, 155µg, 160µg, 165µg, 170µg, 175µg, 180µg, 185µg, 190µg, 195µg, 200µg, 205µg, 210µg, 215µg, 220µg, 225µg, 230µg, 235µg, 240µg, 245µg, 250µg, 255µg, 260µg, 265µg, 270µg, 275µg, 280µg, 285µg, 290µg, 295µg, 300µg, 305µg, 310µg, 315µg ,320µg, 325µg, 330µg, 335µg, 340µg, 345µg, 350µg, 355µg, 360µg, 365µg, 370µg, 375µg, 380µg, 385µg, 390µg, 395µg, 400µg, 405µg, 410µg, 415µg, 420µg, 425µg, 430µg, 435µg, 440µg, 445µg, 450µg, 455µg, 460µg, 465µg, 470µg, 475µg, 480µg, 485µg, 490µg, 495µg, 500µg, 505µg, 510µg, 515µg, 520µg, 525µg, 530µg, 535µg, 540µg, 545µg, 550µg, 555µg, 560µg, 565µg, 570µg, 575µg, 580µg, 585µg, 590µg, 595µg, 600µg, 605µg, 610µg, 615µg, 620µg, 625µg, 630µg, 635µg, 640µg, 645µg, 650µg, 655µg, 660µg, 665µg, 670µg, 675µg, 680µg, 685µg, 690µg, 695µg, 700µg, 705µg, 710µg, 715µg, 720µg, 725µg, 730µg, 735µg, 740µg, 745µg, 750µg, 755µg, 760µg, 765µg, 770µg, 775µg, 780µg, 785µg, 790µg, 795µg, 800µg, 805µg, 810µg, 815µg, 820µg, 825µg, 830µg, 835µg, 840µg, 845µg, 850µg, 855µg, 860µg, 865µg, 870µg, 875µg, 880µg, 885µg, 890µg, 895µg, 900µg, 905µg, 910µg, 915µg, 920µg, 925µg, 930µg, 935µg, 940µg, 945µg, 950µg, 955µg, 960µg, 965µg, 970µg, 975µg, 980µg, 985µg, 990µg, 995µg oder 1000µg **2c** appliziert werden. Bei gegebenenfalls zum Einsatz gelangenden Salzen oder Derivaten von **2c** ist die entsprechende Menge des zum Einsatz gelangenden Salzes/Derivates für den Fachmann je nach Wahl des Salzes/Derivates aus vorstehenden Werten leicht berechenbar.

Werden die Verbindungen der Formel **1** in Kombination mit einem LTD4-Antagonisten **2d** appliziert, werden vorzugsweise etwa 0,01 - 500 mg **2d** pro Einmalgabe appliziert. Bevorzugt werden solche Mengen an **2d** appliziert, dass pro Einmalgabe 0,1 - 250mg, bevorzugt 0,5 - 100 mg, besonders bevorzugt 1-50 mg **2d** enthalten sind. Beispielsweise und ohne den Gegenstand der vorliegenden Erfindung darauf zu beschränken, können pro Einmalgabe 1mg, 2,5mg, 5mg, 5,5mg, 7 mg, 7, 5mg, 10mg, 12,5mg, 15mg, 17,5mg, 20mg, 22,5mg, 25mg, 27,5mg, 30mg, 32,5mg, 35mg, 37,5mg, 40mg, 42,5mg, 45mg, 47,5mg oder 50mg **2d** appliziert werden. Bei gegebenenfalls zum Einsatz gelangenden Säureadditionssalzen, Salzen oder Derivaten von **2d** ist die entsprechende Menge des zum Einsatz gelangenden Salzes/Derivates für den Fachmann je nach Wahl des Salzes/Derivates aus vorstehenden Werten leicht berechenbar.

Werden die Verbindungen der Formel **1** in Kombination mit einem EGFR-Hemmer **2e** appliziert, werden vorzugsweise etwa 100 - 15000 µg **2e** pro Einmalgabe appliziert. Bevorzugt werden solche Mengen an **2e** appliziert, dass pro Einmalgabe 500 - 10000µg, bevorzugt 750 - 8000 µg, besonders bevorzugt 1000-7000 µg **2e** enthalten sind. Beispielsweise und ohne den Gegenstand der vorliegenden Erfindung darauf zu beschränken, können pro Einmalgabe 1000µg, 1150µg, 1200µg, 1250µg, 1300µg, 1350µg, 1400µg, 1450µg, 1500µg, 1550µg,1600µg,1650µg,1700µg,1750µg, 1800µg, 1850µg,1900µg,1950µg, 2000µg, 2050µg, 2100µg, 2150µg, 2200µg, 2250µg, 2300µg, 2350µg, 2400µg, 2450µg, 2500µg, 2550µg, 2600µg, 2650µg, 2700µg, 2750µg, 2800µg, 2850µg, 2900µg, 2950µg, 3000µg, 3050µg, 3100µg, 3150µg, 3200µg, 3250µg, 3300µg, 3350µg, 3400µg, 3450µg, 3500µg, 3550µg, 3600µg, 3650µg, 3700µg, 3750µg, 3800µg, 3850µg, 3900µg, 3950µg, 4000µg, 4050µg, 4100µg, 4150µg, 4200µg, 4250µg, 4300µg, 4350µg, 4400µg, 4450µg, 4500µg, 4550µg, 4600µg, 4650µg, 4700µg, 4750µg, 4800µg, 4850µg, 4900µg, 4950µg, 5000µg, 5050µg, 5100µg, 5150µg, 5200µg, 5250µg, 5300µg, 5350µg, 5400µg, 5450µg, 5500µg, 5550µg, 5600µg, 5650µg, 5700µg, 5750µg, 5800µg, 5850µg, 5900µg, 5950µg, 6000µg, 6050µg, 6100µg, 6150µg, 6200µg, 6250µg, 6300µg, 6350µg, 6400µg, 6450µg, 6500µg, 6550µg, 6600µg, 6650µg, 6700µg, 6750µg, 6800µg, 6850µg, 6900µg, 6950µg, oder 7000µg **2e** appliziert werden. Bei gegebenenfalls zum Einsatz gelangenden Säureadditionssalzen von **2e** ist die entsprechende Menge des zum Einsatz gelangenden Salzes für den Fachmann je nach Wahl der Säure aus vorstehenden Werten leicht berechenbar.

Die beiden Wirkstoffkomponenten **1** und **2** können - zusammen oder räumlich getrennt - jeweils in an sich bekannter Weise inhalativ, oral, parenteral oder auf anderem Wege in weitestgehend üblichen Formulierungen wie beispielsweise Tabletten, Dragees, Pillen, Granulate, Aerosole, Sirupe, Emulsionen, Suspensionen, Pulver und Lösungen, unter Verwendung inerter, nicht toxischer, pharmazeutisch geeigneter Trägerstoffe oder Lösungsmittel, appliziert werden.

Geeignete Anwendungsformen zur Applikation der Verbindungen der Formel **1** und **2** sind beispielsweise Tabletten, Kapseln, Zäpfchen, Lösungen, Pulver etc. Der Anteil der pharmazeutisch wirksamen Verbindung(en) sollte jeweils im Bereich von 0,05 bis 90 Gew.-%, bevorzugt 0,1 bis 50 Gew.% der Gesamtzusammensetzung liegen. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Lösungen werden in üblicher Weise, z.B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösungsmittel als Lösevermittler bzw. Hilfslösungsmittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösungsmittel, wie Paraffine (z.B. Erdölfraktionen), Öle pflanzlichen Ursprungs (z.B. Erdnuss- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide), synthetische Gesteinsmehle (z.B. hochdisperse Kieselsäure und Silikate), Zucker (z.B. Rohr Milch- und Traubenzucker), Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mit verwendet werden. Im Falle wässriger Suspensionen können die Wirkstoffe außer den oben genannten Hilfsstoffen mit verschiedenen Geschmacksaufbesserern oder Farbstoffen versetzt werden.

Vorzugsweise wird auch bei getrennter Applikation der beiden Komponenten **1** und **2** zumindest die Komponente **1** inhalativ appliziert. Wird die Komponente **1** inhalativ appliziert, kann die Komponente **2** bei getrennter Gabe der beiden Wirkstoffe auch beispielsweise oral oder auch parenteral auf Basis im Stand der Technik üblicher Formulierungen wie Tabletten, Dragees, Pillen, Granulate, Aerosole, Sirupe, Emulsionen, Suspensionen, Pulver und Lösungen, unter Verwendung inerter, nicht toxischer, pharmazeutisch geeigneter Trägerstoffe oder Lösungsmittel, appliziert werden.

Vorzugsweise werden die erfindungsgemäßen Arzneimittelkombinationen allerdings inhalativ mittels einer einzigen, beide Wirkstoffe **1** und **2** enthaltenden oder mittels getrennter, jeweils nur einen der Wirkstoffe **1** und **2** enthaltenden, für die inhalative Anwendung geeigneter Darreichungsformen appliziert.

Als inhalierbare Darreichungsformen kommen Inhalationspulver, treibgashaltige Dosieraerosole oder treibgasfreie Inhalationslösungen in Betracht. Erfindungsgemäße Inhalationspulver enthaltend die Wirkstoffkombination aus **1** und **2** können allein aus den genannten Wirkstoffen oder aus einem Gemisch der genannten Wirkstoffe mit physiologisch verträglichen Hilfsstoffen bestehen. Im Rahmen der vorliegenden Erfindung sind von dem Begriff treibgasfreie Inhalationslösungen auch Konzentrate oder sterile, gebrauchsfertige Inhalationslösungen umfasst. Die erfindungsgemäßen Darreichungsformen können die Wirkstoffkombination aus **1** und **2** entweder gemeinsam in einer oder in zwei getrennten Darreichungsformen enthalten. Diese im Rahmen der vorliegenden Erfindung einsetzbaren Darreichungsformen werden im nachfolgenden Teil der Beschreibung detailliert beschrieben.

**A) Inhalationspulver enthaltend die erfindungsgemäßen Wirkstoffkombinationen:** Die erfindungsgemäßen Inhalationspulver können **1** und **2** entweder allein oder im Gemisch mit geeigneten physiologisch unbedenkliche Hilfsstoffen enthalten. Sind die Wirkstoffe **1** und **2** im Gemisch mit physiologisch unbedenklichen Hilfsstoffen enthalten, können zur Darstellung dieser erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose, Trehalose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose, Trehalose oder Glucose, insbesondere, aber nicht ausschließlich in Form ihrer Hydrate, bevorzugt ist.

Die Hilfsstoffe weisen im Rahmen der erfindungsgemäßen Inhalationspulver eine maximale mittlere Teilchengröße von bis zu 250µm, bevorzugt zwischen 10 und 150µm, besonders bevorzugt zwischen 15 und 80µm auf. Gegebenenfalls kann es sinnvoll erscheinen, den vorstehend genannten Hilfsstoffen feinere Hilfsstofffraktionen mit einer mittleren Teilchengröße von 1 bis 9µm beizumischen. Letztgenannte feinere Hilfsstoffe sind ebenfalls ausgewählt aus der vorstehend genannten Gruppe an einsetzbaren Hilfsstoffen. Schließlich wird zur Herstellung der erfindungsgemäßen Inhalationspulver mikronisierter Wirkstoff **1** und **2,** vorzugsweise mit einer mittleren Teilchengröße von 0,5 bis 10µm, besonders bevorzugt von 1 bis 6µm, dem Hilfsstoff oder der Hilfsstoffmischung beigemischt. Verfahren zur Herstellung der erfindungsgemäßen Inhalationspulver durch Mahlen und Mikronisieren sowie durch abschließendes Mischen der Bestandteile sind aus dem Stand der Technik bekannt. Die erfindungsgemäßen Inhalationspulver können entweder in Form einer einzigen Pulvermischung, die sowohl **1** als auch **2** enthält oder in Form von separaten Inhalationspulvern, die lediglich **1** und **2** enthalten bereitgestellt und appliziert werden.

Die erfindungsgemäßen Inhalationspulver können mittels aus dem Stand der Technik bekannten Inhalatoren appliziert werden. Erfindungsgemäße Inhalationspulver, die neben **1** und **2** ferner einen physiologisch unbedenklichen Hilfsstoff enthalten, können beispielsweise mittels Inhalatoren appliziert werden, die eine einzelne Dosis aus einem Vorrat mittels einer Messkammer, wie er in der US 4570630A beschrieben wird, oder über andere apparative Vorrichtungen, wie sie in der DE 36 25 685 A beschrieben werden, dosieren. Die erfindungsgemäßen Inhalationspulver, die **1** und **2** gegebenenfalls in Verbindung mit einem physiologisch verträglichen Hilfsstoff enthalten, können beispielsweise mittels des unter dem Namen Turbohaler^{®} bekannten Inhalators beziehungsweise mit Inhalatoren wie sie beispielsweise in der EP 237507 A offenbart werden, appliziert werden. Vorzugsweise werden die erfindungsgemäßen Inhalationspulver, die neben **1** und **2** physiologisch unbedenkliche Hilfsstoffe enthalten, allerdings in Kapseln abgefüllt (zu so genannten Inhaletten), die in Inhalatoren wie beispielsweise in der WO 94/28958 beschrieben, zur Anwendung gelangen.

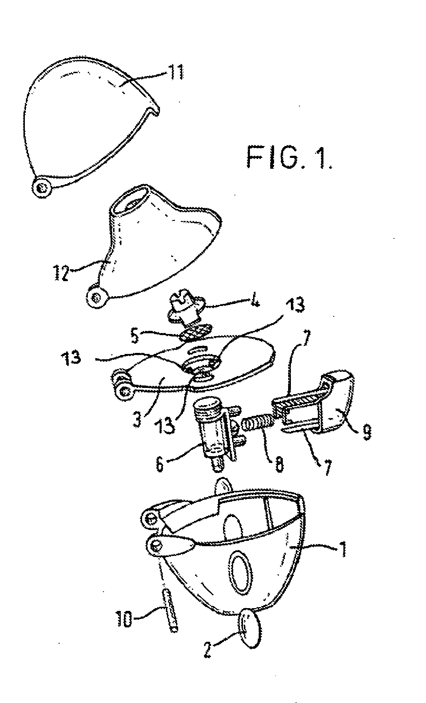

Ein zur Anwendung der erfindungsgemäßen Arzneimittelkombination in Inhaletten besonders bevorzugter Inhalator ist Figur 1 zu entnehmen. Dieser Inhalator (Handihaler^{®}) für die Inhalation pulverförmiger Arzneimittel aus Kapseln ist gekennzeichnet durch ein Gehäuse 1, enthaltend zwei Fenster 2, ein Deck 3, in dem sich Lufteinlassöffnungen befinden und welches mit einem über ein Siebgehäuse 4 befestigten Sieb 5 versehen ist, eine mit Deck 3 verbundene Inhalationskammer 6, an der ein mit zwei geschliffenen Nadeln 7 versehener, gegen eine Feder 8 beweglicher Drücker 9 vorgesehen ist, sowie ein über eine Achse 10 klappbar mit dem Gehäuse 1, dem Deck 3 und einer Kappe 11 verbundenes Mundstück 12, sowie Luftdurchlasslöcher 13 zur Einstellung des Strömungswiderstandes.

Sollen die erfindungsgemäßen Inhalationspulver im Sinne der vorstehend genannten bevorzugten Anwendung in Kapseln abgefüllt werden, bieten sich Füllmengen von 1 bis 30mg pro Kapsel an. Diese enthalten erfindungsgemäß entweder gemeinsam oder jeweils die bereits vorstehend für **1** und **2** genannten Dosierungen pro Einmalgabe.

**B) Treibgashaltige Inhalationsaerosole enthaltend die erfindungsgemäßen Wirkstoffkombinationen:** Erfindungsgemäße treibgashaltige Inhalationsaerosole können **1** und **2** im Treibgas gelöst oder in dispergierter Form enthalten. Hierbei können **1** und **2** in getrennten Darreichungsformen oder in einer gemeinsamen Darreichungsform enthalten sein, wobei **1** und **2** entweder beide gelöst, beide dispergiert oder jeweils nur eine Komponente gelöst und die andere dispergiert enthalten sein können.

Die zur Herstellung der erfindungsgemäßen Inhalationsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie bevorzugt chlorierten und fluorierten Derivaten des Methans, Ethans, Propans, Butans, Cyclopropans oder Cyclobutans. Die vorstehend genannten Treibgase können dabei allein oder in Mischungen derselben zur Verwendung kommen. Besonders bevorzugte Treibgase sind halogenierte Alkanderivate ausgewählt aus TG11, TG12, TG134a (1,1,1,2-Tetrafluorethan), TG227 (1,1,1,2,3,3,3-Heptafluorpropan) und Mischungen derselben, wobei die Treibgase TG134a, TG227 und Gemische derselben bevorzugt sind.

Die erfindungsgemäßem treibgashaltigen Inhalationsaerosole können ferner weitere Bestandteile wie Kosolventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

Die erfindungsgemäßen treibgashaltigen Inhalationsaerosole können bis zu 5 Gew-% an Wirkstoff **1** und/oder **2** enthalten. Erfindungsgemäße Aerosole enthalten beispielsweise 0,002 bis 5 Gew-%, 0,01 bis 3 Gew-%, 0,015 bis 2 Gew-%, 0,1 bis 2 Gew-%, 0,5 bis 2 Gew-% oder 0,5 bis 1 Gew-% an Wirkstoff **1** und/oder **2.**

Liegen die Wirkstoffe **1** und/oder **2** in dispergierter Form vor, weisen die Wirkstoffteilchen bevorzugt eine mittlere Teilchengröße von bis zu 10 µm, bevorzugt von 0,1 bis 6 µm, besonders bevorzugt von 1 bis 5 µm auf.

Die vorstehend genannten erfindungsgemäßen treibgashaltigen Inhalationsaerosole können mittels im Stand der Technik bekannten Inhalatoren (MDIs = metered dose inhalers) appliziert werden. Dementsprechend betrifft ein weiterer Aspekt der vorliegenden Erfindung Arzneimittel in Form von wie vorstehend beschriebenen treibgashaltigen Aerosolen in Verbindung mit einem oder mehreren zur Verabreichung dieser Aerosole geeigneten Inhalatoren. Ferner betrifft die vorliegende Erfindung Inhalatoren, dadurch gekennzeichnet, dass sie vorstehend beschriebene erfindungsgemäße treibgashaltige Aerosole enthalten.

Die vorliegende Erfindung betrifft ferner Kartuschen, die ausgestattet mit einem geeigneten Ventil in einem geeigneten Inhalator zur Anwendung gelangen können und die eine der vorstehend genannten erfindungsgemäßen treibgashaltigen Inhalationsaerosole enthalten. Geeignete Kartuschen und Verfahren zur Abfüllung dieser Kartuschen mit den erfindungsgemäßen treibgashaltigen Inhalationsaerosolen sind aus dem Stand der Technik bekannt.

**C) Treibgasfreie Inhalationslösungen oder Suspensionen enthaltend die erfindungsgemäßen Wirkstoffkombinationen:** Erfindungsgemäß treibgasfreie Inhalationslösungen enthalten beispielsweise wässrige oder alkoholische, bevorzugt ethanolische, gegebenenfalls ethanolische im Gemisch mit wässrigen Lösungsmitteln. Im Falle wässrig/ethanolischer Lösungsmittelgemische ist der relative Anteil an Ethanol gegenüber Wasser nicht begrenzt, bevorzugt liegt die maximale Grenze jedoch bei bis 70 Volumenprozent, insbesondere bei bis zu 60 Volumenprozent Ethanol. Die restlichen Volumenprozente werden von Wasser aufgefüllt. Die **1** und **2** getrennt oder gemeinsam enthaltenden Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.

Erfindungsgemäß kann in der vorliegenden Formulierung auf den Zusatz von Editinsäure (EDTA) oder einem der bekannten Salze davon, Natriumedetat, als Stabilisator oder Komplexbildner verzichtet werden. Andere Ausführungsformen beinhalten diese Verbindung(en). In einer solchen bevorzugten Ausführungsform liegt der Gehalt bezogen auf Natriumedetat unter 100 mg /100 ml, bevorzugt unter 50 mg/100 ml, besonders bevorzugt unter 20 mg/100 ml. Generell sind solche Inhalationslösungen bevorzugt, in denen der Gehalt an Natriumedetat bei 0 bis 10mg/100ml liegt.

Den erfindungsgemäßen treibgasfreien Inhalationslösungen können Co-Solventien und/oder weitere Hilfsstoffe zugesetzt werden.

Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester.

Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien.

Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.

Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration. Die vorstehend genannten Konservierungsstoffe sind vorzugsweise in Konzentrationen von bis zu 50mg/100ml, besonders bevorzugt zwischen 5 und 20 mg/100ml enthalten.

Bevorzugte Formulierungen enthalten außer dem Lösungsmittel Wasser und der Wirkstoffkombination aus **1** und **2** nur noch Benzalkoniumchlorid und Natriumedetat. In einer anderen bevorzugten Ausführungsform wird auf Natriumedetat verzichtet.

Zur Applikation der erfindungsgemäßen treibgasfreien Inhalationslösungen sind besonders solche Inhalatoren, die eine kleine Menge einer flüssigen Formulierung in der therapeutisch notwendigen Dosierung binnen weniger Sekunden in ein therapeutisch-inhalativ geeignetes Aerosol vernebeln können. Im Rahmen der vorliegenden Erfindung sind solche Vernebler bevorzugt, bei denen bereits eine Menge von weniger als 100 µL, bevorzugt weniger als 50 µL, besonders bevorzugt zwischen 10 und 30 µL Wirkstofflösung mit bevorzugt einem Hub zu einem Aerosol mit einer durchschnittlichen Teilchengröße von weniger als 20 µm, bevorzugt weniger als 10 µm, so vernebelt werden können, dass der inhalierbare Anteil des Aerosols bereits der therapeutisch wirksamen Menge entspricht.

Eine derartige Vorrichtung zur treibgasfreien Verabreichung einer dosierten Menge eines flüssigen Arzneimittels zur inhalativen Anwendung, wird beispielsweise in der internationalen Patentanmeldung WO 91/14468 als auch in der WO 97/12687 (dort insbesondere Figuren 6a und 6b) ausführlich beschrieben. Die dort beschriebenen Vernebler (Devices) sind auch unter der Bezeichnung Respimat^{®} bekannt.

Die vorstehend genannten Vertreter der Wirkstoffe **2** sind im Stand der Technik bekannt. Die Verbindungen der Formel **1** sind dagegen noch nicht im Stand der Technik bekannt. Die nachstehend beschriebenen Synthesebeispiele dienen der Illustration möglicher synthetischer Zugänge zu den neuen Verbindungen der Formel **1**. Sie sind allerdings nur als exemplarische Vorgehensweisen zur weitergehenden Erläuterung der Erfindung zu verstehen, ohne selbige auf den nachfolgend exemplarisch beschriebenen Gegenstand zu beschränken.

Die Herstellung der erfindungsgemäßen Verbindungen kann in Analogie zu im Stand der Technik bereits bekannten Vorgehensweisen erfolgen. Geeignete Herstellverfahren sind beispielsweise aus der US 4,460,581 und 4,154,829 bekannt, auf die an dieser Stelle vollinhaltlich Bezug genommen wird.

### BEISPIELE

### SYNTHESE DER ZWISCHENPRODUKTE

### Zwischenprodukt 1: 1,1-Dimethyl-3-(5-methyl-3-p-tolyl-[1,2,4]triazol-1-yl)-propylamin

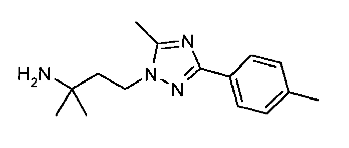

a) 4-Methyl-benzoesäure-(1-imino-ethyl)-hydrazid:1.65 g (72 mmol) Natrium werden in 80 mL Ethanol gelöst. 8.89 g (72 mmol) Ethylacetimidat hydrochlorid in 160 mL Ethanol werden bei Raumtemperatur zugegeben und das ausfallende Natriumchlorid wird abfiltriert. Das Filtrat wird mit 6.00 g (40 mmol) 4-Methyl-benzoesäure hydrazid versetzt und über Nacht gerührt. Die Reaktionsmischung wird eingeengt und gekühlt. Der ausfallende Feststoff wird abfiltriert und mit kaltem Ethanol und Diethylether gewaschen (5.7 g). Aus dem Filtrat werden nach dem Abdestillieren der Lösungsmittel und Umkristallisation aus Ethanol weitere 1.2 g Feststoff gewonnen. Ausbeute: 6.93 g (91%); Massenspektroskopie [M+H]⁺ = 192.
b) 5-Methyl-3-p-tolyl-[1,2,4]triazol: 7.58 g (40 mmol) 4-Methyl-benzoesäure-(1-imino-ethyl)-hydrazid werden unter Rühren für 30 Minuten auf 180°C erwärmt. Nach Abkühlung wird der Feststoff in Chloroform gelöst. Der bei Kühlung ausfallende Niederschlag wird abgesaugt und aus Chloroform umkristallisiert. Ausbeute: 4.82 g (70%); Massenspektroskopie [M+H]⁺ = 174.
c) [1,1-Dimethyl-3-(5-methyl-3-p-tolyl-[1,2,4]triazol-1-yl)-propyl]-carbaminsäure tert-butyl ester: Zu einer Lösung von 4.87 g (28 mmol) 5-Methyl-3-p-tolyl-[1,2,4]triazol in 40 mL DMPU werden bei 0°C 1.35 g (34 mmol, 60%ig) Natriumhydrid gegeben. Die Reaktionsmischung wird auf Raumtemperatur erwärmt und dann eine Stunde gerührt. 9.35 g (42 mmol) (3-Chlor-1,1-dimethyl-propyl)-carbaminsäure-tert-butylester und 1.87 g (5 mmol) Tetrabutylammoniumiodid werden zugegeben und es wird über Nacht bei Raumtemperatur und anschließend noch 2 Stunden bei 80°C gerührt. Man versetzt mit Wasser und Ethylacetat, trennt die wässrige Phase ab und extrahiert diese mit Ethylacetat. Die vereinigten organischen Phasen werden mit Wasser und NatriumchloridLösung gewaschen, mit Natriumsulfat getrocknet und eingeengt. Der Rückstand wird mittels Säulenchromatographie gereinigt (Kieselgel; Petrolether / Ethylacetat = 1:1). Öl. Ausbeute: 2.97 g (30%); Massenspektroskopie [M+H]⁺ = 359.
d) 1,1-Dimethyl-3-(5-methyl-3-p-tolyl-[1,2,4]triazol-1-yl)-propylamin: Zu einer Lösung von 2.97 g (8.3 mmol) [1,1-Dimethyl-3-(5-methyl-3-p-tolyl-[1,2,4]triazol-1-yl)-propyl]-carbaminsäure-tert-butyl ester in 80 mL Dichlormethan werden insgesamt 11 mL Trifluoressigsäure getropft und es wird über Nacht bei Raumtemperatur gerührt. Das Lösungsmittel wird abdestilliert und der Rückstand mit Diethylether versetzt und gerührt. Der ausfallende Feststoff wird abfiltriert und gewaschen. Ausbeute: 2.11 g (68%, Trifluoracetat); Massenspektroskopie [M+H]⁺ = 259.

### Zwischenprodukt 2: 3-[3-(4-Fluor-phenyl)-5-methyl-[1,2,4]triazol-1-yl]-1,1-dimethyl-propylamin

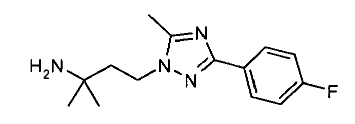

a) 4-Fluor-benzoesäure-(1-imino-ethyl)-hydrazid: Hergestellt aus 7.2 g (58 mmol) Ethylacetimidat hydrochlorid und 5.00 g (32 mmol) 4-Fluor-benzoesäure hydrazid in Analogie zu dem für Zwischenprodukt 1a) beschriebenen Verfahren. Ausbeute: 5.78 g (91%); Massenspektroskopie [M+H]⁺ = 196.
b) 3-(4-Fluor-phenyl)-5-methyl-[1,2,4]triazol: Die Darstellung erfolgt in Analogie zur Vorschrift für Zwischenprodukt 1 b) aus 5.77 g (30 mmol) 4-Fluor-benzoesäure-(1-imino-ethyl)-hydrazid. Ausbeute: 4.11 g (78%); Massenspektroskopie [M+H]⁺ = 178.
c) {3-[3-(4-Fluor-phenyl)-5-methyl-[1,2,4]triazol-1-yl]-1,1-dimethyl-propyl}-carbaminsäure tert-butyl ester: 5.88 g (33 mmol) 3-(4-Fluor-phenyl)-5-methyl-[1 ,2,4]triazol werden in 40 mL DMPU gelöst und in der für Zwischenprodukt 1 c) ausgeführten Weise mit 11.04 g (50 mmol) (3-Chlor-1,1-dimethyl-propyl)-carbaminsäure-tert-butylester, 1.59 g (40 mmol, 60%ig) Natriumhydrid und 2.21 g (6 mmol) Tetrabutylammoniumiodid umgesetzt. Ausbeute: 4.22 g (35%); Massenspektroskopie [M+H]⁺ = 363.
d) 3-[3-(4-Fluor-phenyl)-5-methyl-[1,2,4]triazol-1-yl]-1,1-dimethyl-propylamin: Erhalten aus der Umsetzung von 4.22 g (11.6 mmol) {3-[3-(4-Fluor-phenyl)-5-methyl-[1,2,4]triazol-1-yl]-1,1-dimethyl-propyl}-carbaminsäure-tert-butyl ester in 100 mL Dichlormethan und 15 mL Trifluoressigsäure. Ausbeute: 4.43 g (Trifluoracetat); Massenspektroskopie [M+H]⁺ = 263.

### Zwischenprodukt 3: 3-[3-(3,5-Difluor-phenyl)-5-methyl-[1,2,4]triazol-1-yl]-1,1-dimethyl-propylamin

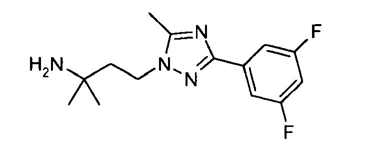

a) 3,5-Difluor-benzoesäure-(1-imino-ethyl)-hydrazid: Aus 4.91 g (40 mmol) Ethylacetimidat hydrochlorid und 3.80 g (22 mmol) 3,5 Difluor-benzoesäure hydrazid in Analogie zur Vorschrift für Zwischenprodukt 1a) erhalten. Ausbeute: 4.49 g (95%); Massenspektroskopie [M+H]⁺ = 214.
b) 3-(3,5-Difluor-phenyl)-5-methyl-[1,2,4]triazol: Hergestellt aus 4.61 g (22 mmol) 3,5-Difluor-benzoesäure-(1-imino-ethyl)-hydrazid. Ausbeute: 3.81 g (91%); Massenspektroskopie [M+H]⁺ = 196.
c) {3-[3-(3,5-Difluor-phenyl)-5-methyl-[1,2,4]triazol-1-yl]-1,1-dimethyl-propyl}-carbaminsäure tert-butyl ester: 3.74 g (19 mmol) 3-(3,5-Difluor-phenyl)-5-methyl-[1,2,4]triazol in 25 mL DMPU werden mit 0.92 g (23 mmol, 60%ig) Natriumhydrid, 6.37 g (29 mmol) (3-Chlor-1,1-dimethyl-propyl)-carbaminsäure-tert-butylester und 1.27 g (3.5 mmol) Tetrabutylammoniumiodid in Analogie zu Zwischenprodukt 1c) umgesetzt. Ausbeute: 2.62 g (36%); Massenspektroskopie [M+H]⁺ = 381.
d) 3-[3-(3,5-Difluor-phenyl)-5-methyl-[1,2,4]triazol-1-yl]-1,1-dimethyl-propylamin: 2.62 g (6.9 mmol) {3-[3-(3,5-Difluor-phenyl)-5-methyl-[1,2,4]triazol-1-yl]-1,1-dimethyl-propyl}-carbaminsäure-tert-butyl ester in 65 mL Dichlormethan werden mit 9 mL Trifluoressigsäure in der für Zwischenprodukt 1d) beschriebenen Weise umgesetzt. Ausbeute: 2.11 g (Trifluoracetat); Massenspektroskopie [M+H]⁺ = 281.

### Zwischenprodukt 6: 3-[1-(3-Amino-3-methyl-butyl)-5-methyl-1H-[1,2,4]triazol-3-yl]-benzoesäuremethylester

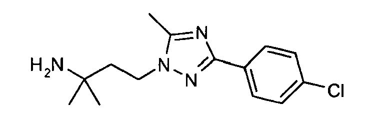

a) 4-Chlor-benzoesäure N'-(1-imino-ethyl)-hydrazid: Zu einer Lösung von 1.91 g (20 mmol) Acetamidin hydrochlorid in 30 mL Ethanol werden 1.09 g (20 mmol) Natriummethylat in 20 mL Ethanol gegeben. Es wird 30 Minuten bei Raumtemperatur gerührt und dann filtriert. Das Filtrat wird mit 2.3 g (13.5 mmol) 4-Chlorbenzoesäure hydrazid versetzt, über Nacht bei Raumtemperatur gerührt, mit einem Eisbad gekühlt und dann filtriert. Der Niederschlag wird mit kalten Ethanol gewaschen und getrocknet. Ausbeute: 1.45 g (51 %); Massenspektroskopie [M+H]⁺ = 212/214.
b) 3-(4-Chlor-phenyl)-5-methyl-1 H-[1,24]triazol: 6.10 g (28.8 mmol) 4-Chlor-benzoesäure N'-(1-imino-ethyl)-hydrazid werden für 30 Minuten auf 180°C erwärmt. Nach Abkühlung werden aus dem Rückstand durch Umkristallisation in Chloroform 2.3 g Produkt erhalten. Einengen der Mutterlauge und anschließende Reinigung des Rückstands mittels Chromatographie (Kieselgel, Petrolether/Ethylacetat = 1:6) liefern zusätzliche 1.22 g Produkt. Ausbeute: 3.51 g (63%); Massenspektroskopie [M+H]⁺ = 194/196.
c) {3-[3-(4-Chlor-phenyl)-5-methyl-[1,2,4]triazol-1-yl]-1,1-dimethyl-propyl}-carbaminsäure tert-butyl ester: 3.48 g (18.0 mmol) 3-(4-Chlor-phenyl)-5-methyl-1 H-[1,24]triazol und 5.98 g (27.0 mmol) (3-Chlor-1,1-dimethyl-propyl)-carbaminsäure-tert-butylester werden in der für Zwischenprodukt 1c) beschriebenen Weise umgesetzt und aufgearbeitet. Ausbeute: 3.89 g (57%); Massenspektroskopie [M+H]⁺ = 379/381.
d) 3-[1-(3-Amino-3-methyl-butyl)-5-methyl-1H-[1,2,4]triazol-3-yl]-benzoesäuremethylester: Erhalten aus {3-[3-(4-Chlor-phenyl)-5-methyl-[1,2,4]triazol-1-yl]-1,1-dimethyl-propyl}-carbaminsäure-tert-butyl ester nach dem für Zwischenprodukt 1d) beschriebenen Verfahren. Ausbeute: 3.65 g (Trifluoracetat); Massenspektroskopie [M+H]⁺ = 279/281.

### Zwischenprodukt 7: 1,1-Dimethyl-3-[5-methyl-3-(4-trifluormethyl-phenyl)-[1,2,4]triazol-1-yl]-propylamin

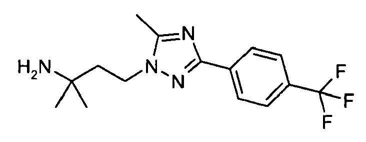

a) 4-Trifluormethyl-benzoesäure N'-(1-imino-ethyl)-hydrazid: 4.78 g (23.4 mmol) 4-(Trifluormethyl)benzoesäure hydrazid und 5.21 g (42.1 mmol) Ethylacetimidat hydrochlorid werden in der für Zwischenprodukt 1a) beschriebenen Weise umgesetzt. Ausbeute: 6.02 g; Massenspektroskopie [M+H]⁺ = 246.
b) 5-Methyl-3-(4-trifluormethyl-phenyl)-1 H-[1,2,4]triazol: Hergestellt aus 6.02 g (24.6 mmol) 4-Trifluormethyl-benzoesäure N'-(1-imino-ethyl)-hydrazid in Analogie zu dem für Zwischenprodukt 1b) beschriebenen Verfahren. Ausbeute: 4.76 g (85%); Massenspektroskopie [M+H]⁺ = 228.
c) {1,1-Dimethyl-3-[5-methyl-3-(4-trifluormethyl-phenyl)-[1,2,4]triazol-1-yl]-propyl}-carbaminsäure-tert-butylester : Die Zielverbindung wird in Analogie zu dem für Zwischenprodukt 1c) beschriebenen Verfahren aus 4.90 g (21.6 mmol) 5-Methyl-3-(4-trifluormethyl-phenyl)-1H-[1,2,4]triazol und 7.17 g (32.4 mmol) (3-Chlor-1,1-dimethylpropyl)-carbaminsäure-tert-butylester erhalten. Ausbeute: 5.06 g (57%); Massenspektroskopie [M+H]⁺ = 413.
d) 1,1-Dimethyl-3-[5-methyl-3-(4-trifluormethyl-phenyl)-[1,2,4]triazol-1-yl]-propylamin: Herstellung nach dem für Zwischenprodukt 1d) beschriebenen Verfahren aus {1,1-Dimethyl-3-[5-methyl-3-(4-trifluormethyl-phenyl)-[1,2,4]triazol-1-yl]-propyl}-carbaminsäure-tert-butylester. Ausbeute: 4.72 g (Trifluoracetat); Massenspektroskopie [M+H]⁺ = 313.

### SYNTHESE DER BEISPIELE

**Allgemeine Arbeitsvorschrift 1:** 1 mmol 6-Benzyloxy-8-(2-ethoxy-2-hydroxy-acetyl)-4H-benzo[1,4]oxazin-3-on und 1 mmol Amin werden 15 Minuten in 5 mL Tetrahydrofuran bei 60°C gerührt. Man kühlt auf 0°C ab und tropft unter Argonatmosphäre 1.5 mL einer 2 molaren Lösung von Lithiumborhydrid in Tetrahydrofuran zu. Es wird 15 min bei 0°C gerührt, mit 10 mL Dichlormethan und 3 mL Wasser versetzt, eine weitere Stunde bei Raumtemperatur gerührt und dann über Kieselgur filtriert, wobei man mit Dichlormethan eluiert. Das Eluat wird vom Lösungsmittel befreit und der Rückstand, falls notwendig, chromatographisch gereinigt. Der so erhaltene Benzylether wird in Methanol gelöst und mit Palladium auf Kohle (10%ig) als Katalysator bei 2.5 bar und Raumtemperatur hydriert. Anschließend wird der Katalysator abgetrennt und das Rohprodukt mittels Chromatographie (Reverse Phase, Acetonitril/Wasser-Gradient mit 0.1% Trifluoressigsäure) gereinigt.

**Allgemeine Arbeitsvorschrift 2:** 1 mmol 6-Benzyloxy-8-(2-ethoxy-2-hydroxy-acetyl)-4H-benzo[1,4]oxazin-3-on und 1 mmol Amin werden in 5 mL Ethanol suspendiert und auf 70°C erwärmt. Die entstandene Lösung wird eine Stunde bei 70°C gerührt und dann auf Raumtemperatur abgekühlt. Nach Zugabe von 113 mg (3 mmol) Natriumborhydrid wird 3 Stunden bei Raumtemperatur gerührt, mit 0.7 mL gesättigter Kaliumcarbonatlösung versetzt und weitere 30 Minuten gerührt. Es wird über Aluminiumoxid (basisch) filtriert, wiederholt mit Dichlormethan/Methanol = 15:1 nachgewaschen, eingeengt und chromatographiert (Kieselgel; Dichlormethan mit 0-10% Methanol:Ammoniak = 9:1). Der so erhaltene Benzylether wird in 10 mL Methanol gelöst und mit Palladium auf Kohle als Katalysator bei 1 bar Wasserstoffdruck hydriert. Anschließend wird der Katalysator abfiltriert und das Filtrat eingeengt.

### Beispiel 1: 8-(2-{3-[3-(4-Fluor-phenyl)-5-methyl-[1,2,4]triazol-1-yl]-1,1-dimethyl-propylamino}-1-hydroxy-ethyl)-6-hydroxy-4H-benzo[1,4]oxazin-3-on

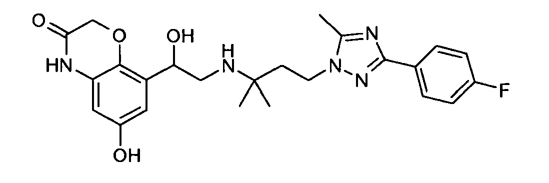

Erhalten aus der Umsetzung von 6-Benzyloxy-8-(2-ethoxy-2-hydroxy-acetyl)-4H-benzo[1,4]oxazin-3-on und 3-[3-(4-Fluor-phenyl)-5-methyl-[1,2,4]triazol-1-yl]-1,1-dimethyl-propylamin nach der allgemeinen Arbeitsvorschrift 1. Die abschließende Reinigung erfolgt mittels Chromatographie (Reverse Phase, Acetonitril/Wasser-Gradient mit 0.1% Trifluoressigsäure). Ausbeute: 134 mg (29%, Trifluoracetat); Massenspektroskopie [M+H]⁺ = 470.

### Beispiel 2: 8-{2-[1,1-Dimethyl-3-(5-methyl-3-p-tolyl-[1,2,4]triazol-1yl)-propylamino]-1-hydroxy-ethyl}-6-hydroxy-4H-benzo[1,4]oxazin-3-on

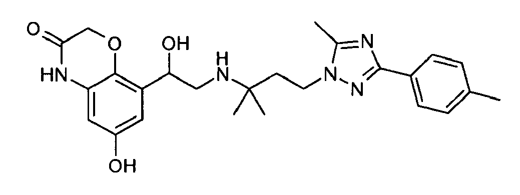

Hergestellt aus 6-Benzyloxy-8-(2-ethoxy-2-hydroxy-acetyl)-4H-benzo[1,4]oxazin-3-on und 1,1-Dimethyl-3-(5-methyl-3-p-tolyl-[1,2,4]triazol-1-yl]-propylamin nach der allgemeinen Arbeitsvorschrift 1. Ausbeute: 283 mg (49%, Trifluoracetat); Massenspektroskopie [M+H]⁺ = 466.

### Beispiel 3: 8-(2-{1,1-Dimethyl-3-[5-methyl-3-(4-trifluormethyl-phenyl)-[1,2,4]triazol-1-yl]-propylamino}-1-hydroxy-ethyl)-6-hydroxy-4H-benzo[1,4]oxazin-3-on

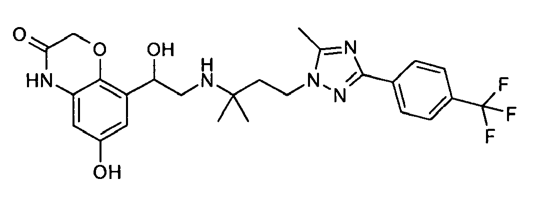

Hergestellt aus 6-Benzyloxy-8-(2-ethoxy-2-hydroxy-acetyl)-4H-benzo[1,4]oxazin-3-on und 1,1-Dimethyl-3-[5-methyl-3-(4-trifluormethyl-phenyl)-[1,2,4]triazol-1-yl]-propylamin in Analogie zur allgemeinen Arbeitsvorschrift 1. Ausbeute: 234 mg (37%, Trifluoracetat); Massenspektroskopie [M+H]⁺ = 520.

### Beispiel 4: 8-(2-{3-[3-(3,5-Difluor-phenyl)-5-methyl-[1,2,4]triazol-1-yl]-1,1-dimethyl-propylamino}-1-hydroxy-ethyl)-6-hydroxy-4H-benzo[1,4]oxazin-3-on

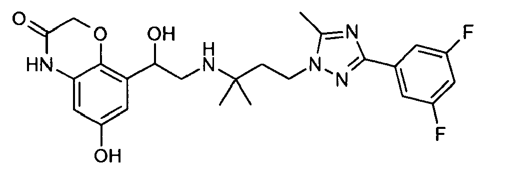

Hergestellt aus 6-Benzyloxy-8-(2-ethoxy-2-hydroxy-acetyl)-4H-benzo[1,4]oxazin-3-on und 3-[3-(3,5-Difluor-phenyl)-5-methyl-[1,2,4]triazol-1-yl]-1,1-dimethyl-propylamin nach der allgemeinen Arbeitsvorschrift 1. Ausbeute: 208 mg (35%, Trifluoracetat); Massenspektroskopie [M+H]⁺ = 488.

### Beispiel 6: 8-(2-{3-[3-(4-Chlor-phenyl)-5-methyl-[1,2,4]triazol-1-yl]-1,1-dimethyl-propylamino}-1-hydroxy-ethyl)-6-hydroxy-4H-benzo[1,4]oxazin-3-on

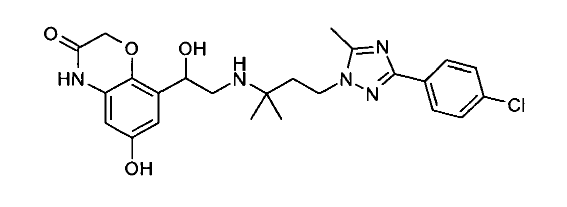

a) 6-Benzyloxy-8-(2-{3-[3-(4-chlor-phenyl)-5-methyl-[1,2,4]triazol-1-yl]-1,1-dimethyl-propylamino}-1-hydroxy-ethyl)-4H-benzo[1,4]oxazin-3-on: Hergestellt aus 6-Benzyloxy-8-(2-ethoxy-2-hydroxy-acetyl)-4H-benzo[1,4]oxazin-3-on und 3-[3-(4-Chlor-phenyl)-5-methyl-[1,2,4]triazol-1-yl]-1,1-dimethyl-propylamin in Analogie zur allgemeinen Arbeitsvorschrift 1. Das Rohprodukt wird mittels Chromatographie (Reverse Phase, Acetonitril/Wasser-Gradient mit 0.1% Trifluoressigsäure) gereinigt. Ausbeute: 550 mg (80%, Trifluoracetat); Massenspektroskopie [M+H]⁺ = 576.

8-(2-{3-[3-(4-Chlor-phenyl)-5-methyl-[1,2,4]triazol-1-yl]-1,1-dimethyl-propylamino}-1-hydroxy-ethyl)-6-hydroxy-4H-benzo[1,4]oxazin-3-on: 550 mg (0.80 mmol) 6-Benzyloxy-8-(2-{3-[3-(4-chlor-phenyl)-5-methyl-[1,2,4]triazol-1-yl]-1,1-dimethyl-propylamino}-1-hydroxyethyl)-4H-benzo[1,4]oxazin-3-on werden in 3 mL Dichlormethan gelöst und auf -78°C abgekühlt. 2 mL einer 1 molaren Lösung von Bortribromid in Dichlormethan werden zugetropft und es wird auf Raumtemperatur erwärmt. Man lässt 10 Minuten bei dieser Temperatur Rühren und versetzt dann mit 10 mL Dichlormethan und 3 mL Wasser und rührt 30 Minuten. Es wird über Kieselgur filtriert, wobei man mit Dichlormethan und Methanol eluiert. Das Eluat wird eingeengt und der Rückstand mittels Chromatographie (Reverse Phase, Acetonitril/Wasser-Gradient mit 0.1% Trifluoressigsäure) gereinigt. Ausbeute: 29 mg (6%, Trifluoracetat); Massenspektroskopie [M+H]⁺ = 486/8.

## Patentansprüche

1. Arzneimittelkombinationen, die neben einer oder mehrerer Verbindungen der allgemeinen Formel **1** 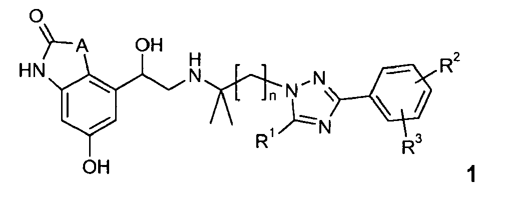 worin die Verbindung der Formel 1 ausgewählt ist aus der Gruppe bestehend aus:
**1.1:** 8-(2-{3-[3-(4-Fluor-phenyl)-5-methyl-[1,2,4]triazol-1-yl]-1,1-dimethyl-propylamino}-1-hydroxy-ethyl)-6-hydroxy-4H-benzo[1,4]oxazin-3-on;
**1.2:** 8-{2-[1,1-Dimethyl-3-(5-methyl-3-p-tolyl-[1,2,4]triazol-1yl)-propylamino]-1-hydroxyethyl}-6-hydroxy-4H-benzo[1,4]oxazin-3-on;
**1.3:** 8-(2-{1,1-Dimethyl-3-[5-methyl-3-(4-trifluormethyl-phenyl)-[1,2,4]triazol-1-yl]-propylamino}-1-hydroxy-ethyl)-6-hydroxy-4H-benzo[1,4]oxazin-3-on;
**1.4:** 8-(2-{3-[3-(3,5-Difluor-phenyl)-5-methyl-[1,2,4]triazol-1-yl]-1,1-dimethyl-propylamino}-1-hydroxy-ethyl)-6-hydroxy-4H-benzo[1,4]oxazin-3-on;
**1.6:** 8-(2-{3-[3-(4-Chlor-phenyl)-5-methyl-[1,2,4]triazol-1-yl]-1,1-dimethyl-propylamino}-1-hydroxy-ethyl)-6-hydroxy-4H-benzo[1,4]oxazin-3-on;
gegebenenfalls in Form der einzelnen optischen Isomeren, Mischungen der einzelnen Enantiomeren oder Racemate oder in Form der Säureadditionssalze mit pharmakologisch unbedenklichen Säuren sowie gegebenenfalls in Form der Solvate und/oder Hydrate enthalten;
wenigstens einen weiteren Wirkstoff **2** enthalten.

2. Arzneimittelkombinationen nach Anspruch 1, die neben einer oder mehrerer Verbindungen der Formel **1** als weiteren Wirkstoff **2** einen oder mehrere Verbindungen enthalten, die ausgewählt sind aus den Klassen der Anticholinergika **(2a),** PDE-IV-Inhibitoren **(2b),** Steroide **(2c),** LTD4-Antagonisten **(2d)** und EGFR-Hemmer **(2e).**

3. Arzneimittelkombinationen nach einem der Ansprüche 1 oder 2, die neben einer oder mehrerer Verbindungen der allgemeinen Formel **1** als weiteren Wirkstoff **2** ein Anticholinergikum **(2a)** enthalten.

4. Arzneimittelkombinationen nach einem der Ansprüche 1 oder 2, die neben einer oder mehrerer Verbindungen der allgemeinen Formel 1 als weiteren Wirkstoff **2** ein Steroid **(2c)** enthalten.

5. Arzneimittelkombinationen nach einem der Ansprüche 1 oder 2, **dadurch gekennzeichnet, dass** sie neben therapeutisch wirksamen Mengen von **1** ferner therapeutisch wirksame Mengen eines Anticholinergikums **(2a)** sowie therapeutische Mengen eines PDEIV- Inhibitors **(2b)** enthalten.

6. Arzneimittelkombinationen nach einem der Ansprüche 1 oder 2, **dadurch gekennzeichnet, dass** sie neben therapeutisch wirksamen Mengen von **1** ferner therapeutisch wirksame Mengen eines Anticholinergikums **(2a),** sowie therapeutische Mengen eines Steroids **(2c)** enthalten.

7. Arzneimittelkombinationen nach einem der Ansprüche 1 oder 2, **dadurch gekennzeichnet, dass** sie neben therapeutisch wirksamen Mengen von **1** ferner therapeutisch wirksame Mengen eines PDEIV- Inhibitors **(2b),** sowie therapeutische Mengen eines Steroids **(2c)** enthalten.

8. Arzneimittelkombination nach einem der Ansprüche 1 bis 7, **dadurch gekennzeichnet, dass** sie in Form einer für die Inhalation geeigneten Darreichungsform vorliegt.

9. Arzneimittelkombination nach Anspruch 8, **dadurch gekennzeichnet, dass** es sich bei der Darreichungsform um eine treibgasfreie Inhalationslösung oder -suspension handelt, die als Lösemittel Wasser, Ethanol oder ein Gemisch aus Wasser und Ethanol enthält.

10. Verwendung einer Arzneimittelkombination nach einem der Ansprüche 1 bis 9 zur Herstellung eines Arzneimittels zur Behandlung von entzündlichen und obstruktiven Atemwegserkrankungen, zur Hemmung verfrüht einsetzender Wehen in der Geburtshilfe (Tokolyse), zur Wiederherstellung des Sinusrhythmus im Herzen bei atrioventrikulärem Block, zur Behebung bradykaler Herzrhythmusstörungen (Antiarrhythmikum), zur Therapie des Kreislaufschocks (Gefäßerweiterung und Steigerung des Herzzeitvolumens) sowie zur Behandlung von Juckreiz und Entzündungen der Haut.

11. Verwendung einer Verbindung der Formel 1 nach einem der Ansprüche 1 bis 9 zur Herstellung eines Arzneimittels zur Behandlung von entzündlichen und obstruktiven Atemwegserkrankungen, zur Hemmung verfrüht einsetzender Wehen in der Geburtshilfe (Tokolyse), zur Wiederherstellung des Sinusrhythmus im Herzen bei atrioventrikulärem Block, zur Behebung bradykaler Herzrhythmusstörungen (Antiarrhythmikum), zur Therapie des Kreislaufschocks (Gefäßerweiterung und Steigerung des Herzzeitvolumens) sowie zur Behandlung von Juckreiz und Entzündungen der Haut, in Kombination mit wenigstens einem weiteren Wirkstoff **2.**

12. Verwendung nach Anspruch 9 oder 10, zur Herstellung eines Arzneimittels zur Behandlung von Atemwegserkrankungen, die ausgewählt sind aus der Gruppe bestehend aus Obstruktive Lungenerkrankungen unterschiedlicher Genese, Lungenemphyseme unterschiedlicher Genese, Restriktive Lungenerkrankungen, Interstitielle Lungenerkrankungen, Zystische Fibrose, Bronchitiden unterschiedlicher Genese, Bronchiektasen, ARDS (adult respiratory distress syndrom) und alle Formen des Lungenödems.

## Claims

1. Pharmaceutical combinations which contain in addition to one or more compounds of general formula **1** 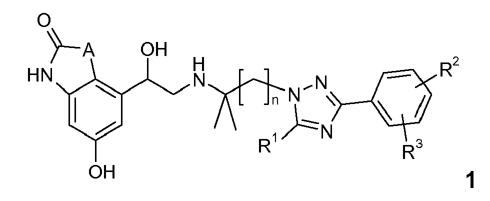 wherein the compound of formula 1 is selected from among:
**1.1:** 8-(2-{-[3-(4-fluoro-phenyl)-5-methyl-[1,2,4]triazol-1-yl]-1.1-dimethyl-propylamino}-1-hydroxy-ethyl)-6-hydroxy-4H-benzo[1,4]oxazin-3-one;
**1.2:** 8-{2-[1,1-dimethyl-3-(5-methyl-3-p-tolyl-[1,2,4]triazol-1yl)-propylamino]-1-hydroxy-ethyl}-6-hydroxy-4H-benzo[1,4]oxazin-3-one;
**1.3:** 8-(2-{1,1-dimethyl-3-[5-methyl-3-(4-trifluoromethyl-phenyl)-[1,2,4]triazol-1-yl]-propylamino}-1-hydroxy-ethyl)-6-hydroxy-4H-benzo[1,4]oxazin-3-one;
**1.4:** 8-(2-{3-[3-(3,5-difluoro-phenyl)-5-methyl-[1,2,4]triazol-1-yl]-1,1-dimethyl-propylamino}-1-hydroxy-ethyl)-6-hydroxy-4H-benzo[1,4]oxazin-3-one;
**1.6:** 8-(2-{3-[3-(4-chloro-phenyl)-5-methyl-[1,2,4]triazol-1-yl]-1,1-dimethyl-propylamino}-1-hydroxy-ethyl)-6-hydroxy-4H-benzo[1,4]oxazin-3-one;
optionally in the form of the individual optical isomers, mixtures of the individual enantiomers or racemates or in the form of the acid addition salts with pharmacologically acceptable acids, and optionally in the form of the solvates and/or hydrates thereof;
at least one further active substance **2.**

2. Pharmaceutical combinations according to claim 1, which contain in addition to one or more compounds of general formula **1** as a further active substance **2** one or more compounds which are selected from among the categories of the anticholinergics **(2a),** PDE-IV-inhibitors **(2b),** steroids **(2c),** LTD4-antagonists **(2d)** and EGFR-inhibitors **(2e).**

3. Pharmaceutical combinations according to claim 1 or 2, which contain an anticholinergic **(2a)** as a further active substance **2** in addition to one or more compounds of general formula **1.**

4. Pharmaceutical combinations according to one of claims 1 or 2, which contain a steroid **(2c)** as a further active substance **2** in addition to one or more compounds of general formula **1.**

5. Pharmaceutical combinations according to one of claims 1 or 2, **characterised in that** they also contain, in addition to therapeutically effective amounts of **1,** therapeutically effective amounts of an anticholinergic **(2a)** as well as therapeutic amounts of a PDEIV inhibitor **(2b).**

6. Pharmaceutical combinations according to one of claims 1 or 2, **characterised in that** they also contain, in addition to therapeutically effective amounts of **1,** therapeutically effective amounts of an anticholinergic **(2a),** as well as therapeutic amounts of a steroid **(2c).**

7. Pharmaceutical combinations according to one of claims 1 or 2, **characterised in that** they also contain, in addition to therapeutically effective amounts of **1,** therapeutically effective amounts of a PDEIV inhibitor **(2b),** as well as therapeutic amounts of a steroid **(2c).**

8. Pharmaceutical combination according to one of claims 1 to 7, **characterised in that** it is in the form of a formulation suitable for inhalation.

9. Pharmaceutical combination according to claim 8, **characterised in that** the preparation is a propellant-free inhalable solution or suspension which contains as solvent water, ethanol or a mixture of water and ethanol.

10. Use of a pharmaceutical combination according to one of claims 1 to 9 for preparing a pharmaceutical composition for the treatment of inflammatory and obstructive respiratory complaints, for inhibiting premature labour in midwifery (tocolysis), for restoring sinus rhythm in the heart in atrioventricular block, for correcting bradycardic heart rhythm disorders (antiarrhythmic), for treating circulatory shock (vasodilatation and increasing the heart volume) as well as for the treatment of skin irritations and inflammation.

11. Use of a compound of formula 1 according to one of claims 1 to 9 for preparing a pharmaceutical composition for the treatment of inflammatory and obstructive respiratory complaints, for inhibiting premature labour in midwifery (tocolysis), for restoring sinus rhythm in the heart in atrioventricular block, for correcting bradycardic heart rhythm disorders (antiarrhythmic), for treating circulatory shock (vasodilatation and increasing the heart volume) as well as for the treatment of skin irritations and inflammation, in combination with at least one additional active substance **2.**

12. Use according to claim 9 or 10, for preparing a pharmaceutical composition for the treatment of respiratory complaints selected from the group comprising obstructive pulmonary diseases of various origins, pulmonary emphysema of various origins, restrictive pulmonary diseases, interstitial pulmonary diseases, cystic fibrosis, bronchitis of various origins, bronchiectasis, ARDS (adult respiratory distress syndrome) and all forms of pulmonary oedema.

## Revendications

1. Combinaisons de médicaments qui contiennent, parallèlement à un ou plusieurs composés de formule générale 1 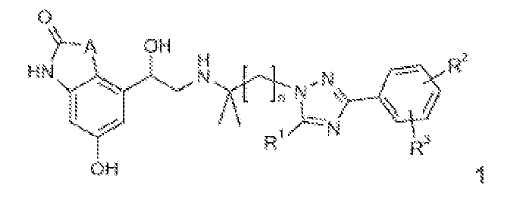 le composé de formule 1 étant choisi dans le groupe comprenant les :
1.1 : 8-(2-{3-[3-(4-fluorophényl)-5-méthyl-[1,2,4]-triazol-1-yl]-1,1-diméthylpropylamino}-1-hydroxy-éthyl)-6-hydroxy-4H-benzo[1,4]oxazin-3-one ;
1.2 : 8-{2-[1,1-diméthyl-3-(5-méthyl-3-p-tolyl-[1, 2,4]triazol-1-yl)-propylamino]-1-hydroxy-éthyl}-6-hydroxy-4H-benzo[1,4]oxazin-3-one ;
1.3 : 8-(2-{1,1-diméthyl-3-[5-méthyl-3-(4-trifluorométhyl-phényl)-[1,2,4]triazol-1-yl]-propylamino}-1-hydroxyéthyl)-6-hydroxy-4H-benzo [1,4]oxazin-3-one ;
1.4 : 8-(2-{3-[3-(3,5-difluorophényl)-5-méthyl-[1, 2, 4] - triazol-1-yl]-1,1-diméthylpropylamino)-1-hydroxy-éthyl)-6-hydroxy-4H-benzo[1,4]oxazin-3-one ;
1.6 : 8- (2-{3- [3- (4-chlorophényl)-5-méthyl-[1, 2, 4] - triazol-1-yl]-1,1-diméthylpropylamino}-1-hydroxy-éthyl)-6-hydroxy-4H-benzo[1,4]oxazin-3-one ;
éventuellement sous la forme des isomères optiques individuels, de mélanges d'énantiomères individuels ou de racémates ou sous la forme de sels d'addition acide avec des acides pharmacologiquement acceptables et éventuellement sous la forme de solvates et/ou hydrates ;
au moins une autre substance active 2.

2. Combinaisons de médicaments selon la revendication 1, qui contiennent comme autre substance active 2, parallèlement à un ou plusieurs composés de formule 1, un ou plusieurs composés qui sont choisis dans la classe des anticholinergiques (2a), des inhibiteurs de PDE-IV (2b), des stéroïdes (2c), des antagonistes de LTD4 (2d) et des inhibiteurs de EGFR (2e).

3. Combinaisons de médicaments selon l'une des revendications 1 ou 2 qui contiennent, comme autre substance active 2, parallèlement à un ou plusieurs composés de formule générale 1, un anticholinergique (2a).

4. Combinaisons de médicaments selon l'une des revendications 1 ou 2 qui contiennent, comme autre substance active 2, parallèlement à un ou plusieurs composés de formule générale 1, un stéroïde (2c).

5. Combinaisons de médicaments selon l'une des revendications 1 ou 2, **caractérisées en ce qu'**elles contiennent, parallèlement à une quantité thérapeutiquement efficace de composé 1, en outre des quantités thérapeutiquement efficaces d'un anticholinergique (2a) et des quantités thérapeutiques d'un inhibiteur de PDEV (2b).

6. Combinaisons de médicaments selon l'une des revendications 1 ou 2, **caractérisées en ce qu'**elles contiennent, parallèlement à des quantités thérapeutiquement efficaces du composé 1, en outre des quantités thérapeutiquement efficaces d'un anticholinergique (2a) et des quantités thérapeutiques d'un stéroïde (2c).

7. Combinaisons de médicaments selon l'une des revendications 1 ou 2, **caractérisées en ce qu'**elles contiennent, parallèlement à une quantité thérapeutiquement efficace de composé 1, en outre des quantités thérapeutiquement efficaces d'un inhibiteur de PDEIV (2b) et des quantités thérapeutiques d'un stéroïde (2c).

8. Combinaison médicamenteuse selon l'une des revendications 1 à 7, **caractérisé en ce qu'**il se présente sous une forme galénique appropriée pour l'inhalation.

9. Combinaison de médicaments selon la revendication 8, **caractérisée en ce que** la forme galénique est une solution ou suspension pour inhalation sans gaz propulseur qui contient comme solvant, de l'eau, de l'éthanol ou un mélange d'eau et d'éthanol.

10. Utilisation d'une combinaison de médicaments selon l'une des revendications 1 à 9, pour la production d'un médicament pour le traitement des maladies des voies respiratoires inflammatoires et obstructives, pour l'inhibition des contractions précoces dans l'accouchement (tocolyse) ou pour la restauration du rythme sinusal dans le coeur dans le bloc auriculo-ventriculaire, pour l'élimination des troubles du rythme cardiaque bradycardique (anti-arythmique), pour le traitement du choc circulatoire (élargissement vasculaire et augmentation du débit sanguin) et pour le traitement du prurit et des inflammations cutanées.

11. Utilisation d'un composé de formule 1 selon l'une des revendications 1 à 9, pour la production d'un médicament pour le traitement des maladies des voies respiratoires inflammatoires et obstructives, pour l'inhibition des contractions précoces dans l'accouchement (tocolyse), pour la restauration du rythme sinusal dans le coeur dans le bloc auriculo-ventriculaire, pour l'élimination des troubles du rythme cardiaque bradycardique (anti-arythmique), pour le traitement du choc circulatoire (élargissement vasculaire et augmentation du débit sanguin) et pour le traitement du prurit et des inflammations cutanées, en combinaison avec au moins une autre substance active 2.

12. Utilisation selon la revendication 9 ou 10, pour la production d'un médicament pour le traitement des maladies des voies respiratoires qui sont choisies dans le groupe comprenant les maladies pulmonaires obstructives d'étiologies diverses, l'emphysème pulmonaire d'étiologies diverses, les maladies pulmonaires restrictives, les maladies pulmonaires interstitielles, la fibrose kystique, les bronchitides d'étiologies diverses, les bronchiectasies, l'ARDS (syndrome de détresse respiratoire adulte) et toutes les formes d'oedème pulmonaire.
